(19) Europäisches Patentamt
European Patent Office
Office européen des brevets

(11) EP 2 695 950 A1

# (12) EUROPEAN PATENT APPLICATION

(43) Date of publication:
**12.02.2014 Bulletin 2014/07**

(21) Application number: **12180179.9**

(22) Date of filing: **10.08.2012**

(51) Int Cl.:
***C12Q 1/68** (2006.01)* ***G01N 33/574** (2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Blackfield AG**
**51105 Köln (DE)**

(72) Inventors:
- **Thomas, Roman**
**Bornheim (DE)**
- **Malchers, Florian**
**50679 Köln (DE)**

(74) Representative: **Vossius & Partner**
**Siebertstrasse 4**
**81675 München (DE)**

## (54) Markers for responsiveness to an inhibitor of the fibroblast growth factor receptor

(57) The present invention relates to a method for predicting the responsiveness of a mammalian tumor cell or cancer cell to an inhibitor of a fibroblast growth factor receptor (FGFR). Also a method for predicting the responsiveness of an individual to an inhibitor of a fibroblast growth factor receptor (FGFR) is provided. These methods involve the evaluation of the status of FGFR and the status of c-myc, whereby said status of FGFR and of c-myc is indicative for the responsiveness to the inhibitor. The status of FGFR may be its amplifcation status or its expression level and the status of c-myc may be its expression level, like an intermediate or high expression level of c-myc. Furthermore, a kit useful for carrying out the methods described herein as well as an oligo- or polynucleotide and/or antibodies capable of detecting the expression level or gene amplification status of FGFR or the expression level of c-myc for predicting the responsiveness to the inhibitor are provided.

EP 2 695 950 A1

SEQUENCE LISTING

<110> Thomas, Roman

<120> A method for identifying and predicting a responder to a FGFR1 inhibitor

<130> U1843 EP S3

<160> 20

<170> BiSSAP 1.0

<210> 1
<211> 2202
<212> DNA
<213> Homo sapiens

<220>
<221> source
<222> 1..2202
<223> /mol_type="DNA"
/organism="Homo sapiens"

<400> 1
atgtggagct ggaagtgcct cctcttctgg gctgtgctgg tcacagccac actctgcacc 60

gctaggccgt ccccgacctt gcctgaacaa gatgctctcc cctcctcgga ggatgatgat 120

gatgatgatg actcctcttc agaggagaaa gaaacagata acaccaaacc aaaccgtatg 180

cccgtagctc catattggac atccccagaa aagatggaaa agaaattgca tgcagtgccg 240

gctgccaaga cagtgaagtt caaatgccct tccagtggga ccccaaaccc cacactgcgc 300

tggttgaaaa atggcaaaga attcaaacct gaccacagaa ttggaggcta caaggtccgt 360

tatgccacct ggagcatcat aatggactct gtggtgccct ctgacaaggg caactacacc 420

tgcattgtgg agaatgagta cggcagcatc aaccacacat accagctgga tgtcgtggag 480

cggtcccctc accggcccat cctgcaagca gggttgcccg ccaacaaaac agtggccctg 540

ggtagcaacg tggagttcat gtgtaaggtg tacagtgacc cgcagccgca catccagtgg 600

ctaaagcaca tcgaggtgaa tgggagcaag attggcccag acaacctgcc ttatgtccag 660

atcttgaaga ctgctggagt taataccacc gacaaagaga tggaggtgct tcacttaaga 720

aatgtctcct ttgaggacgc aggggagtat acgtgcttgg cgggtaactc tatcggactc 780

tcccatcact ctgcatggtt gaccgttctg gaagccctgg aagagaggcc ggcagtgatg 840

acctcgcccc tgtacctgga gatcatcatc tattgcacag ggccttcct catctcctgc 900

atggtggggt cggtcatcgt ctacaagatg aagagtggta ccaagaagag tgacttccac 960

agccagatgg ctgtgcacaa gctggccaag agcatccctc tgcgcagaca ggtaacagtg 1020

tctgctgact ccagtgcatc catgaactct ggggttcttc tggttcggcc atcacggctc 1080

tcctccagtg ggactcccat gctagcaggg gtctctgagt atgagcttcc cgaagaccct 1140

cgctgggagc tgcctcggga cagactggtc ttaggcaaac ccctgggaga gggctgcttt 1200

```
gggcaggtgg tgttggcaga ggctatcggg ctggacaagg acaaacccaa ccgtgtgacc    1260
aaagtggctg tgaagatgtt gaagtcggac gcaacagaga aagacttgtc agacctgatc    1320
tcagaaatgg agatgatgaa gatgatcggg aagcataaga atatcatcaa cctgctgggg    1380
gcctgcacgc aggatggtcc cttgtatgtc atcgtggagt atgcctccaa gggcaacctg    1440
cgggagtacc tgcaggcccg gaggcccca gggctggaat actgctacaa ccccagccac     1500
aacccagagg agcagctctc ctccaaggac ctggtgtcct gcgcctacca ggtggcccga    1560
ggcatggagt atctggcctc caagaagtgc atacaccgag acctggcagc caggaatgtc    1620
ctggtgacag aggacaatgt gatgaagata gcagactttg gcctcgcacg ggacattcac    1680
cacatcgact actataaaaa gacaaccaac ggccgactgc ctgtgaagtg gatggcaccc    1740
gaggcattat ttgaccggat ctacacccac cagagtgatg tgtggtcttt cggggtgctc    1800
ctgtgggaga tcttcactct gggcggctcc ccataccccg gtgtgcctgt ggaggaactt    1860
ttcaagctgc tgaaggaggg tcaccgcatg gacaagccca gtaactgcac caacgagctg    1920
tacatgatga tgcgggactg ctggcatgca gtgccctcac agagacccac cttcaagcag    1980
ctggtggaag acctggaccg catcgtggcc ttgacctcca accaggagta cctggacctg    2040
tccatgcccc tggaccagta ctcccccagc tttcccgaca cccggagctc tacgtgctcc    2100
tcaggggagg attccgtctt ctctcatgag ccgctgcccg aggagccctg cctgccccga    2160
cacccagccc agcttgccaa tggcggactc aaacgccgct ga                       2202
```

```
<210> 2
<211> 733
<212> PRT
<213> Homo sapiens

<220>
<221> SOURCE
<222> 1..733
<223> /mol_type="protein"
      /organism="Homo sapiens"

<400> 2
Met Trp Ser Trp Lys Cys Leu Leu Phe Trp Ala Val Leu Val Thr Ala
1               5                   10                  15
Thr Leu Cys Thr Ala Arg Pro Ser Pro Thr Leu Pro Glu Gln Asp Ala
            20                  25                  30
Leu Pro Ser Ser Glu Asp Asp Asp Asp Asp Asp Asp Ser Ser Ser Glu
        35                  40                  45
Glu Lys Glu Thr Asp Asn Thr Lys Pro Asn Arg Met Pro Val Ala Pro
    50                  55                  60
Tyr Trp Thr Ser Pro Glu Lys Met Glu Lys Lys Leu His Ala Val Pro
65                  70                  75                  80
Ala Ala Lys Thr Val Lys Phe Lys Cys Pro Ser Ser Gly Thr Pro Asn
                85                  90                  95
Pro Thr Leu Arg Trp Leu Lys Asn Gly Lys Glu Phe Lys Pro Asp His
            100                 105                 110
Arg Ile Gly Gly Tyr Lys Val Arg Tyr Ala Thr Trp Ser Ile Ile Met
        115                 120                 125
Asp Ser Val Val Pro Ser Asp Lys Gly Asn Tyr Thr Cys Ile Val Glu
    130                 135                 140
```

```
Asn Glu Tyr Gly Ser Ile Asn His Thr Tyr Gln Leu Asp Val Val Glu
145                 150                 155                 160
Arg Ser Pro His Arg Pro Ile Leu Gln Ala Gly Leu Pro Ala Asn Lys
                165                 170                 175
Thr Val Ala Leu Gly Ser Asn Val Glu Phe Met Cys Lys Val Tyr Ser
            180                 185                 190
Asp Pro Gln Pro His Ile Gln Trp Leu Lys His Ile Glu Val Asn Gly
        195                 200                 205
Ser Lys Ile Gly Pro Asp Asn Leu Pro Tyr Val Gln Ile Leu Lys Thr
    210                 215                 220
Ala Gly Val Asn Thr Thr Asp Lys Glu Met Glu Val Leu His Leu Arg
225                 230                 235                 240
Asn Val Ser Phe Glu Asp Ala Gly Glu Tyr Thr Cys Leu Ala Gly Asn
                245                 250                 255
Ser Ile Gly Leu Ser His His Ser Ala Trp Leu Thr Val Leu Glu Ala
            260                 265                 270
Leu Glu Glu Arg Pro Ala Val Met Thr Ser Pro Leu Tyr Leu Glu Ile
        275                 280                 285
Ile Ile Tyr Cys Thr Gly Ala Phe Leu Ile Ser Cys Met Val Gly Ser
    290                 295                 300
Val Ile Val Tyr Lys Met Lys Ser Gly Thr Lys Lys Ser Asp Phe His
305                 310                 315                 320
Ser Gln Met Ala Val His Lys Leu Ala Lys Ser Ile Pro Leu Arg Arg
                325                 330                 335
Gln Val Thr Val Ser Ala Asp Ser Ser Ala Ser Met Asn Ser Gly Val
            340                 345                 350
Leu Leu Val Arg Pro Ser Arg Leu Ser Ser Ser Gly Thr Pro Met Leu
        355                 360                 365
Ala Gly Val Ser Glu Tyr Glu Leu Pro Glu Asp Pro Arg Trp Glu Leu
    370                 375                 380
Pro Arg Asp Arg Leu Val Leu Gly Lys Pro Leu Gly Glu Gly Cys Phe
385                 390                 395                 400
Gly Gln Val Val Leu Ala Glu Ala Ile Gly Leu Asp Lys Asp Lys Pro
                405                 410                 415
Asn Arg Val Thr Lys Val Ala Val Lys Met Leu Lys Ser Asp Ala Thr
            420                 425                 430
Glu Lys Asp Leu Ser Asp Leu Ile Ser Glu Met Glu Met Met Lys Met
        435                 440                 445
Ile Gly Lys His Lys Asn Ile Ile Asn Leu Leu Gly Ala Cys Thr Gln
    450                 455                 460
Asp Gly Pro Leu Tyr Val Ile Val Glu Tyr Ala Ser Lys Gly Asn Leu
465                 470                 475                 480
Arg Glu Tyr Leu Gln Ala Arg Arg Pro Pro Gly Leu Glu Tyr Cys Tyr
                485                 490                 495
Asn Pro Ser His Asn Pro Glu Glu Gln Leu Ser Ser Lys Asp Leu Val
            500                 505                 510
Ser Cys Ala Tyr Gln Val Ala Arg Gly Met Glu Tyr Leu Ala Ser Lys
        515                 520                 525
Lys Cys Ile His Arg Asp Leu Ala Ala Arg Asn Val Leu Val Thr Glu
    530                 535                 540
Asp Asn Val Met Lys Ile Ala Asp Phe Gly Leu Ala Arg Asp Ile His
545                 550                 555                 560
His Ile Asp Tyr Tyr Lys Lys Thr Thr Asn Gly Arg Leu Pro Val Lys
                565                 570                 575
Trp Met Ala Pro Glu Ala Leu Phe Asp Arg Ile Tyr Thr His Gln Ser
            580                 585                 590
Asp Val Trp Ser Phe Gly Val Leu Leu Trp Glu Ile Phe Thr Leu Gly
        595                 600                 605
Gly Ser Pro Tyr Pro Gly Val Pro Val Glu Glu Leu Phe Lys Leu Leu
    610                 615                 620
Lys Glu Gly His Arg Met Asp Lys Pro Ser Asn Cys Thr Asn Glu Leu
625                 630                 635                 640
Tyr Met Met Met Arg Asp Cys Trp His Ala Val Pro Ser Gln Arg Pro
                645                 650                 655
Thr Phe Lys Gln Leu Val Glu Asp Leu Asp Arg Ile Val Ala Leu Thr
```

```
            660                 665                 670
Ser Asn Gln Glu Tyr Leu Asp Leu Ser Met Pro Leu Asp Gln Tyr Ser
        675                 680                 685
Pro Ser Phe Pro Asp Thr Arg Ser Ser Thr Cys Ser Ser Gly Glu Asp
    690                 695                 700
Ser Val Phe Ser His Glu Pro Leu Pro Glu Glu Pro Cys Leu Pro Arg
705                 710                 715                 720
His Pro Ala Gln Leu Ala Asn Gly Gly Leu Lys Arg Arg
                725                 730
```

<210> 3
<211> 2463
<212> DNA
<213> Homo sapiens

<220>
<221> source
<222> 1..2463
<223> /mol_type="DNA"
/organism="Homo sapiens"

<400> 3

```
atgtggagct ggaagtgcct cctcttctgg gctgtgctgg tcacagccac actctgcacc      60
gctaggccgt ccccgacctt gcctgaacaa gcccagccct ggggagcccc tgtggaagtg     120
gagtccttcc tggtccaccc cggtgacctg ctgcagcttc gctgtcggct gcgggacgat     180
gtgcagagca tcaactggct gcgggacggg gtgcagctgg cggaaagcaa ccgcacccgc     240
atcacagggg aggaggtgga ggtgcaggac tccgtgcccg cagactccgg cctctatgct     300
tgcgtaacca gcagcccctc gggcagtgac accacctact tctccgtcaa tgtttcagat     360
gctctcccct cctcggagga tgatgatgat gatgatgact cctcttcaga ggagaaagaa     420
acagataaca ccaaaccaaa ccccgtagct ccatattgga catccccaga aaagatggaa     480
aagaaattgc atgcagtgcc ggctgccaag acagtgaagt tcaaatgccc ttccagtggg     540
accccaaacc ccacactgcg ctggttgaaa aatggcaaag aattcaaacc tgaccacaga     600
attggaggct acaaggtccg ttatgccacc tggagcatca taatggactc tgtggtgccc     660
tctgacaagg gcaactacac ctgcattgtg gagaatgagt acggcagcat caaccacaca     720
taccagctgg atgtcgtgga gcggtcccct caccggccca tcctgcaagc agggttgccc     780
gccaacaaaa cagtggccct gggtagcaac gtggagttca tgtgtaaggt gtacagtgac     840
ccgcagccgc acatccagtg gctaaagcac atcgaggtga atgggagcaa gattggccca     900
gacaacctgc cttatgtcca gatcttgaag actgctggag ttaataccac cgacaaagag     960
atggaggtgc ttcacttaag aaatgtctcc tttgaggacg caggggagta tacgtgcttg    1020
gcgggtaact ctatcggact ctcccatcac tctgcatggt tgaccgttct ggaagccctg    1080
gaagagaggc cggcagtgat gacctcgccc ctgtacctgg agatcatcat ctattgcaca    1140
ggggccttcc tcatctcctg catggtgggg tcggtcatcg tctacaagat gaagagtggt    1200
accaagaaga gtgacttcca cagccagatg gctgtgcaca agctggccaa gagcatccct    1260
ctgcgcagac aggtaacagt gtctgctgac tccagtgcat ccatgaactc tggggttctt    1320
```

ctggttcggc catcacggct ctcctccagt gggactccca tgctagcagg ggtctctgag 1380

tatgagcttc ccgaagaccc tcgctgggag ctgcctcggg acagactggt cttaggcaaa 1440

cccctgggag agggctgctt tgggcaggtg gtgttggcag aggctatcgg gctggacaag 1500

gacaaaccca accgtgtgac caaagtggct gtgaagatgt tgaagtcgga cgcaacagag 1560

aaagacttgt cagacctgat ctcagaaatg gagatgatga agatgatcgg gaagcataag 1620

aatatcatca acctgctggg ggcctgcacg caggatggtc ccttgtatgt catcgtggag 1680

tatgcctcca agggcaacct gcgggagtac ctgcaggccc ggaggccccc agggctggaa 1740

tactgctaca accccagcca caacccagag gagcagctct cctccaagga cctggtgtcc 1800

tgcgcctacc aggtggcccg aggcatggag tatctggcct ccaagaagtg catacaccga 1860

gacctggcag ccaggaatgt cctggtgaca gaggacaatg tgatgaagat agcagacttt 1920

ggcctcgcac gggacattca ccacatcgac tactataaaa agacaaccaa cggccgactg 1980

cctgtgaagt ggatggcacc cgaggcatta tttgaccgga tctacaccca ccagagtgat 2040

gtgtggtctt tcggggtgct cctgtgggag atcttcactc tgggcggctc cccatacccc 2100

ggtgtgcctg tggaggaact tttcaagctg ctgaaggagg gtcaccgcat ggacaagccc 2160

agtaactgca ccaacgagct gtacatgatg atgcgggact gctggcatgc agtgccctca 2220

cagagaccca ccttcaagca gctggtggaa gacctggacc gcatcgtggc cttgacctcc 2280

aaccaggagt acctggacct gtccatgccc ctggaccagt actcccccag ctttcccgac 2340

acccggagct ctacgtgctc ctcaggggag gattccgtct tctctcatga gccgctgccc 2400

gaggagccct gcctgccccg acacccagcc cagcttgcca atggcggact caaacgccgc 2460

tga 2463

<210> 4
<211> 820
<212> PRT
<213> Homo sapiens

<220>
<221> SOURCE
<222> 1..820
<223> /mol_type="protein"
/organism="Homo sapiens"

<400> 4
```
Met Trp Ser Trp Lys Cys Leu Leu Phe Trp Ala Val Leu Val Thr Ala
1               5                   10                  15
Thr Leu Cys Thr Ala Arg Pro Ser Pro Thr Leu Pro Glu Gln Ala Gln
            20                  25                  30
Pro Trp Gly Ala Pro Val Glu Val Glu Ser Phe Leu Val His Pro Gly
        35                  40                  45
Asp Leu Leu Gln Leu Arg Cys Arg Leu Arg Asp Asp Val Gln Ser Ile
    50                  55                  60
Asn Trp Leu Arg Asp Gly Val Gln Leu Ala Glu Ser Asn Arg Thr Arg
65                  70                  75                  80
Ile Thr Gly Glu Glu Val Glu Val Gln Asp Ser Val Pro Ala Asp Ser
```

```
                85                  90                  95
Gly Leu Tyr Ala Cys Val Thr Ser Ser Pro Ser Gly Ser Asp Thr Thr
            100                 105                 110
Tyr Phe Ser Val Asn Val Ser Asp Ala Leu Pro Ser Ser Glu Asp Asp
        115                 120                 125
Asp Asp Asp Asp Asp Ser Ser Ser Glu Glu Lys Glu Thr Asp Asn Thr
    130                 135                 140
Lys Pro Asn Pro Val Ala Pro Tyr Trp Thr Ser Pro Glu Lys Met Glu
145                 150                 155                 160
Lys Lys Leu His Ala Val Pro Ala Ala Lys Thr Val Lys Phe Lys Cys
                165                 170                 175
Pro Ser Ser Gly Thr Pro Asn Pro Thr Leu Arg Trp Leu Lys Asn Gly
            180                 185                 190
Lys Glu Phe Lys Pro Asp His Arg Ile Gly Gly Tyr Lys Val Arg Tyr
        195                 200                 205
Ala Thr Trp Ser Ile Ile Met Asp Ser Val Val Pro Ser Asp Lys Gly
    210                 215                 220
Asn Tyr Thr Cys Ile Val Glu Asn Glu Tyr Gly Ser Ile Asn His Thr
225                 230                 235                 240
Tyr Gln Leu Asp Val Val Glu Arg Ser Pro His Arg Pro Ile Leu Gln
                245                 250                 255
Ala Gly Leu Pro Ala Asn Lys Thr Val Ala Leu Gly Ser Asn Val Glu
            260                 265                 270
Phe Met Cys Lys Val Tyr Ser Asp Pro Gln Pro His Ile Gln Trp Leu
        275                 280                 285
Lys His Ile Glu Val Asn Gly Ser Lys Ile Gly Pro Asp Asn Leu Pro
    290                 295                 300
Tyr Val Gln Ile Leu Lys Thr Ala Gly Val Asn Thr Thr Asp Lys Glu
305                 310                 315                 320
Met Glu Val Leu His Leu Arg Asn Val Ser Phe Glu Asp Ala Gly Glu
                325                 330                 335
Tyr Thr Cys Leu Ala Gly Asn Ser Ile Gly Leu Ser His His Ser Ala
            340                 345                 350
Trp Leu Thr Val Leu Glu Ala Leu Glu Glu Arg Pro Ala Val Met Thr
        355                 360                 365
Ser Pro Leu Tyr Leu Glu Ile Ile Ile Tyr Cys Thr Gly Ala Phe Leu
    370                 375                 380
Ile Ser Cys Met Val Gly Ser Val Ile Val Tyr Lys Met Lys Ser Gly
385                 390                 395                 400
Thr Lys Lys Ser Asp Phe His Ser Gln Met Ala Val His Lys Leu Ala
                405                 410                 415
Lys Ser Ile Pro Leu Arg Arg Gln Val Thr Val Ser Ala Asp Ser Ser
            420                 425                 430
Ala Ser Met Asn Ser Gly Val Leu Leu Val Arg Pro Ser Arg Leu Ser
        435                 440                 445
Ser Ser Gly Thr Pro Met Leu Ala Gly Val Ser Glu Tyr Glu Leu Pro
    450                 455                 460
Glu Asp Pro Arg Trp Glu Leu Pro Arg Asp Arg Leu Val Leu Gly Lys
465                 470                 475                 480
Pro Leu Gly Glu Gly Cys Phe Gly Gln Val Val Leu Ala Glu Ala Ile
                485                 490                 495
Gly Leu Asp Lys Asp Lys Pro Asn Arg Val Thr Lys Val Ala Val Lys
            500                 505                 510
Met Leu Lys Ser Asp Ala Thr Glu Lys Asp Leu Ser Asp Leu Ile Ser
        515                 520                 525
Glu Met Glu Met Met Lys Met Ile Gly Lys His Lys Asn Ile Ile Asn
    530                 535                 540
Leu Leu Gly Ala Cys Thr Gln Asp Gly Pro Leu Tyr Val Ile Val Glu
545                 550                 555                 560
Tyr Ala Ser Lys Gly Asn Leu Arg Glu Tyr Leu Gln Ala Arg Arg Pro
                565                 570                 575
Pro Gly Leu Glu Tyr Cys Tyr Asn Pro Ser His Asn Pro Glu Glu Gln
            580                 585                 590
Leu Ser Ser Lys Asp Leu Val Ser Cys Ala Tyr Gln Val Ala Arg Gly
        595                 600                 605
```

```
Met Glu Tyr Leu Ala Ser Lys Lys Cys Ile His Arg Asp Leu Ala Ala
    610                 615                 620
Arg Asn Val Leu Val Thr Glu Asp Asn Val Met Lys Ile Ala Asp Phe
625                 630                 635                 640
Gly Leu Ala Arg Asp Ile His His Ile Asp Tyr Tyr Lys Lys Thr Thr
                645                 650                 655
Asn Gly Arg Leu Pro Val Lys Trp Met Ala Pro Glu Ala Leu Phe Asp
            660                 665                 670
Arg Ile Tyr Thr His Gln Ser Asp Val Trp Ser Phe Gly Val Leu Leu
        675                 680                 685
Trp Glu Ile Phe Thr Leu Gly Gly Ser Pro Tyr Pro Gly Val Pro Val
    690                 695                 700
Glu Glu Leu Phe Lys Leu Leu Lys Glu Gly His Arg Met Asp Lys Pro
705                 710                 715                 720
Ser Asn Cys Thr Asn Glu Leu Tyr Met Met Met Arg Asp Cys Trp His
                725                 730                 735
Ala Val Pro Ser Gln Arg Pro Thr Phe Lys Gln Leu Val Glu Asp Leu
            740                 745                 750
Asp Arg Ile Val Ala Leu Thr Ser Asn Gln Glu Tyr Leu Asp Leu Ser
        755                 760                 765
Met Pro Leu Asp Gln Tyr Ser Pro Ser Phe Pro Asp Thr Arg Ser Ser
    770                 775                 780
Thr Cys Ser Ser Gly Glu Asp Ser Val Phe Ser His Glu Pro Leu Pro
785                 790                 795                 800
Glu Glu Pro Cys Leu Pro Arg His Pro Ala Gln Leu Ala Asn Gly Gly
                805                 810                 815
Leu Lys Arg Arg
            820

<210> 5
<211> 1365
<212> DNA
<213> Homo sapiens

<220>
<221> source
<222> 1..1365
<223> /mol_type="DNA"
      /organism="Homo sapiens"

<400> 5
ctggattttt ttcgggtagt ggaaaaccag cagcctcccg cgacgatgcc cctcaacgtt      60

agcttcacca acaggaacta tgacctcgac tacgactcgg tgcagccgta tttctactgc     120

gacgaggagg agaacttcta ccagcagcag cagcagagcg agctgcagcc cccggcgccc     180

agcgaggata tctggaagaa attcgagctg ctgcccaccc cgcccctgtc ccctagccgc     240

cgctccgggc tctgctcgcc ctcctacgtt gcggtcacac ccttctccct tcggggagac     300

aacgacggcg gtggcgggag cttctccacg gccgaccagc tggagatggt gaccgagctg     360

ctgggaggag acatggtgaa ccagagtttc atctgcgacc cggacgacga gaccttcatc     420

aaaaacatca tcatccagga ctgtatgtgg agcggcttct cggccgccgc caagctcgtc     480

tcagagaagc tggcctccta ccaggctgcg cgcaaagaca gcggcagccc gaacccccgcc     540

cgcggccaca gcgtctgctc cacctccagc ttgtacctgc aggatctgag cgccgccgcc     600

tcagagtgca tcgacccctc ggtggtcttc ccctaccctc tcaacgacag cagctcgccc     660

aagtcctgcg cctcgcaaga ctccagcgcc ttctctccgt cctcggattc tctgctctcc     720
```

tcgacggagt cctccccgca gggcagcccc gagcccctgg tgctccatga ggagacaccg 780

cccaccacca gcagcgactc tgaggaggaa caagaagatg aggaagaaat cgatgttgtt 840

tctgtggaaa agaggcaggc tcctggcaaa aggtcagagt ctggatcacc ttctgctgga 900

ggccacagca aacctcctca cagcccactg gtcctcaaga ggtgccacgt ctccacacat 960

cagcacaact acgcagcgcc tccctccact cggaaggact atcctgctgc caagagggtc 1020

aagttggaca gtgtcagagt cctgagacag atcagcaaca accgaaaatg caccagcccc 1080

aggtcctcgg acaccgagga gaatgtcaag aggcgaacac acaacgtctt ggagcgccag 1140

aggaggaacg agctaaaacg gagctttttt gccctgcgtg accagatccc ggagttggaa 1200

aacaatgaaa aggcccccaa ggtagttatc cttaaaaaag ccacagcata catcctgtcc 1260

gtccaagcag aggagcaaaa gctcatttct gaagaggact tgttgcggaa acgacgagaa 1320

cagttgaaac acaaacttga acagctacgg aactcttgtg cgtaa 1365

<210> 6
<211> 454
<212> PRT
<213> Homo sapiens

<220>
<221> SOURCE
<222> 1..454
<223> /mol_type="protein"
/organism="Homo sapiens"

<400> 6
Leu Asp Phe Phe Arg Val Val Glu Asn Gln Gln Pro Pro Ala Thr Met
1 5 10 15
Pro Leu Asn Val Ser Phe Thr Asn Arg Asn Tyr Asp Leu Asp Tyr Asp
20 25 30
Ser Val Gln Pro Tyr Phe Tyr Cys Asp Glu Glu Glu Asn Phe Tyr Gln
35 40 45
Gln Gln Gln Gln Ser Glu Leu Gln Pro Pro Ala Pro Ser Glu Asp Ile
50 55 60
Trp Lys Lys Phe Glu Leu Leu Pro Thr Pro Pro Leu Ser Pro Ser Arg
65 70 75 80
Arg Ser Gly Leu Cys Ser Pro Ser Tyr Val Ala Val Thr Pro Phe Ser
85 90 95
Leu Arg Gly Asp Asn Asp Gly Gly Gly Gly Ser Phe Ser Thr Ala Asp
100 105 110
Gln Leu Glu Met Val Thr Glu Leu Leu Gly Gly Asp Met Val Asn Gln
115 120 125
Ser Phe Ile Cys Asp Pro Asp Asp Glu Thr Phe Ile Lys Asn Ile Ile
130 135 140
Ile Gln Asp Cys Met Trp Ser Gly Phe Ser Ala Ala Ala Lys Leu Val
145 150 155 160
Ser Glu Lys Leu Ala Ser Tyr Gln Ala Ala Arg Lys Asp Ser Gly Ser
165 170 175
Pro Asn Pro Ala Arg Gly His Ser Val Cys Ser Thr Ser Ser Leu Tyr
180 185 190
Leu Gln Asp Leu Ser Ala Ala Ala Ser Glu Cys Ile Asp Pro Ser Val
195 200 205
Val Phe Pro Tyr Pro Leu Asn Asp Ser Ser Ser Pro Lys Ser Cys Ala
210 215 220
Ser Gln Asp Ser Ser Ala Phe Ser Pro Ser Ser Asp Ser Leu Leu Ser
225 230 235 240

```
Ser Thr Glu Ser Ser Pro Gln Gly Ser Pro Glu Pro Leu Val Leu His
                245                 250                 255
Glu Glu Thr Pro Pro Thr Thr Ser Ser Asp Ser Glu Glu Glu Gln Glu
            260                 265                 270
Asp Glu Glu Glu Ile Asp Val Val Ser Val Glu Lys Arg Gln Ala Pro
        275                 280                 285
Gly Lys Arg Ser Glu Ser Gly Ser Pro Ser Ala Gly Gly His Ser Lys
    290                 295                 300
Pro Pro His Ser Pro Leu Val Leu Lys Arg Cys His Val Ser Thr His
305                 310                 315                 320
Gln His Asn Tyr Ala Ala Pro Pro Ser Thr Arg Lys Asp Tyr Pro Ala
                325                 330                 335
Ala Lys Arg Val Lys Leu Asp Ser Val Arg Val Leu Arg Gln Ile Ser
            340                 345                 350
Asn Asn Arg Lys Cys Thr Ser Pro Arg Ser Ser Asp Thr Glu Glu Asn
        355                 360                 365
Val Lys Arg Arg Thr His Asn Val Leu Glu Arg Gln Arg Arg Asn Glu
    370                 375                 380
Leu Lys Arg Ser Phe Phe Ala Leu Arg Asp Gln Ile Pro Glu Leu Glu
385                 390                 395                 400
Asn Asn Glu Lys Ala Pro Lys Val Val Ile Leu Lys Lys Ala Thr Ala
                405                 410                 415
Tyr Ile Leu Ser Val Gln Ala Glu Glu Gln Lys Leu Ile Ser Glu Glu
            420                 425                 430
Asp Leu Leu Arg Lys Arg Arg Glu Gln Leu Lys His Lys Leu Glu Gln
        435                 440                 445
Leu Arg Asn Ser Cys Ala
    450

<210> 7
<211> 2466
<212> DNA
<213> Homo sapiens

<220>
<221> source
<222> 1..2466
<223> /mol_type="DNA"
      /organism="Homo sapiens"

<400> 7
atggtcagct ggggtcgttt catctgcctg gtcgtggtca ccatggcaac cttgtccctg      60

gcccggccct ccttcagttt agttgaggat accacattag agccagaaga gccaccaacc     120

aaataccaaa tctctcaacc agaagtgtac gtggctgcgc caggggagtc gctagaggtg     180

cgctgcctgt tgaaagatgc cgccgtgatc agttggacta aggatggggt gcacttgggg     240

cccaacaata ggacagtgct tattggggag tacttgcaga taaagggcgc cacgcctaga     300

gactccggcc tctatgcttg tactgccagt aggactgtag acagtgaaac ttggtacttc     360

atggtgaatg tcacagatgc catctcatcc ggagatgatg aggatgacac cgatggtgcg     420

gaagattttg tcagtgagaa cagtaacaac aagagagcac catactggac caacacagaa     480

aagatggaaa agcggctcca tgctgtgcct gcggccaaca ctgtcaagtt tcgctgccca     540

gccgggggga acccaatgcc aaccatgcgg tggctgaaaa acgggaagga gtttaagcag     600

gagcatcgca ttggaggcta caaggtacga aaccagcact ggagcctcat tatggaaagt     660

gtggtcccat ctgacaaggg aaattatacc tgtgtagtgg agaatgaata cgggtccatc     720
```

```
aatcacacgt accacctgga tgttgtggag cgatcgcctc accggcccat cctccaagcc     780
ggactgccgg caaatgcctc cacagtggtc ggaggagacg tagagtttgt ctgcaaggtt     840
tacagtgatg cccagcccca catccagtgg atcaagcacg tggaaaagaa cggcagtaaa     900
tacgggcccg acgggctgcc ctacctcaag gttctcaagg ccgccggtgt taacaccacg     960
gacaaagaga ttgaggttct ctatattcgg aatgtaactt ttgaggacgc tggggaatat    1020
acgtgcttgg cgggtaattc tattgggata tcctttcact ctgcatggtt gacagttctg    1080
ccagcgcctg gaagagaaaa ggagattaca gcttccccag actacctgga gatagccatt    1140
tactgcatag gggtcttctt aatcgcctgt atggtggtaa cagtcatcct gtgccgaatg    1200
aagaacacga ccaagaagcc agacttcagc agccagccgg ctgtgcacaa gctgaccaaa    1260
cgtatccccc tgcggagaca ggtaacagtt tcggctgagt ccagctcctc catgaactcc    1320
aacaccccgc tggtgaggat aacaacacgc ctctcttcaa cggcagacac cccatgctg     1380
gcaggggtct ccgagtatga acttccagag gacccaaaat gggagtttcc aagagataag    1440
ctgacactgg gcaagcccct gggagaaggt tgctttgggc aagtggtcat ggcggaagca    1500
gtgggaattg acaaagacaa gcccaaggag gcggtcaccg tggccgtgaa gatgttgaaa    1560
gatgatgcca cagagaaaga cctttctgat ctggtgtcag agatggagat gatgaagatg    1620
attgggaaac acaagaatat cataaatctt cttggagcct gcacacagga tgggcctctc    1680
tatgtcatag ttgagtatgc ctctaaaggc aacctccgag aatacctccg agcccggagg    1740
ccacccggga tggagtactc ctatgacatt aaccgtgttc ctgaggagca gatgaccttc    1800
aaggacttgg tgtcatgcac ctaccagctg gccagaggca tggagtactt ggcttcccaa    1860
aaatgtattc atcgagattt agcagccaga aatgttttgg taacagaaaa caatgtgatg    1920
aaaatagcag actttggact cgccagagat atcaacaata tagactatta caaaaagacc    1980
accaatgggc ggcttccagt caagtggatg gctccagaag ccctgtttga tagagtatac    2040
actcatcaga gtgatgtctg gtccttcggg gtgttaatgt gggagatctt cactttaggg    2100
ggctcgccct acccagggat tcccgtggag gaacttttta agctgctgaa ggaaggacac    2160
agaatggata agccagccaa ctgcaccaac gaactgtaca tgatgatgag ggactgttgg    2220
catgcagtgc cctcccagag accaacgttc aagcagttgg tagaagactt ggatcgaatt    2280
ctcactctca caaccaatga ggaatacttg gacctcagcc aacctctcga acagtattca    2340
cctagttacc ctgacacaag aagttcttgt tcttcaggag atgattctgt tttttctcca    2400
gaccccatgc cttacgaacc atgccttcct cagtatccac acataaacgg cagtgttaaa    2460
acatga                                                               2466
```

<210> 8
<211> 821
<212> PRT
<213> Homo sapiens

<220>
<221> SOURCE
<222> 1..821
<223> /mol_type="protein"
/organism="Homo sapiens"

<400> 8
Met Val Ser Trp Gly Arg Phe Ile Cys Leu Val Val Val Thr Met Ala
1 5 10 15
Thr Leu Ser Leu Ala Arg Pro Ser Phe Ser Leu Val Glu Asp Thr Thr
20 25 30
Leu Glu Pro Glu Glu Pro Pro Thr Lys Tyr Gln Ile Ser Gln Pro Glu
35 40 45
Val Tyr Val Ala Ala Pro Gly Glu Ser Leu Glu Val Arg Cys Leu Leu
50 55 60
Lys Asp Ala Ala Val Ile Ser Trp Thr Lys Asp Gly Val His Leu Gly
65 70 75 80
Pro Asn Asn Arg Thr Val Leu Ile Gly Glu Tyr Leu Gln Ile Lys Gly
85 90 95
Ala Thr Pro Arg Asp Ser Gly Leu Tyr Ala Cys Thr Ala Ser Arg Thr
100 105 110
Val Asp Ser Glu Thr Trp Tyr Phe Met Val Asn Val Thr Asp Ala Ile
115 120 125
Ser Ser Gly Asp Asp Glu Asp Asp Thr Asp Gly Ala Glu Asp Phe Val
130 135 140
Ser Glu Asn Ser Asn Asn Lys Arg Ala Pro Tyr Trp Thr Asn Thr Glu
145 150 155 160
Lys Met Glu Lys Arg Leu His Ala Val Pro Ala Ala Asn Thr Val Lys
165 170 175
Phe Arg Cys Pro Ala Gly Gly Asn Pro Met Pro Thr Met Arg Trp Leu
180 185 190
Lys Asn Gly Lys Glu Phe Lys Gln Glu His Arg Ile Gly Gly Tyr Lys
195 200 205
Val Arg Asn Gln His Trp Ser Leu Ile Met Glu Ser Val Val Pro Ser
210 215 220
Asp Lys Gly Asn Tyr Thr Cys Val Val Glu Asn Glu Tyr Gly Ser Ile
225 230 235 240
Asn His Thr Tyr His Leu Asp Val Val Glu Arg Ser Pro His Arg Pro
245 250 255
Ile Leu Gln Ala Gly Leu Pro Ala Asn Ala Ser Thr Val Val Gly Gly
260 265 270
Asp Val Glu Phe Val Cys Lys Val Tyr Ser Asp Ala Gln Pro His Ile
275 280 285
Gln Trp Ile Lys His Val Glu Lys Asn Gly Ser Lys Tyr Gly Pro Asp
290 295 300
Gly Leu Pro Tyr Leu Lys Val Leu Lys Ala Ala Gly Val Asn Thr Thr
305 310 315 320
Asp Lys Glu Ile Glu Val Leu Tyr Ile Arg Asn Val Thr Phe Glu Asp
325 330 335
Ala Gly Glu Tyr Thr Cys Leu Ala Gly Asn Ser Ile Gly Ile Ser Phe
340 345 350
His Ser Ala Trp Leu Thr Val Leu Pro Ala Pro Gly Arg Glu Lys Glu
355 360 365
Ile Thr Ala Ser Pro Asp Tyr Leu Glu Ile Ala Ile Tyr Cys Ile Gly
370 375 380
Val Phe Leu Ile Ala Cys Met Val Val Thr Val Ile Leu Cys Arg Met
385 390 395 400
Lys Asn Thr Thr Lys Lys Pro Asp Phe Ser Ser Gln Pro Ala Val His
405 410 415
Lys Leu Thr Lys Arg Ile Pro Leu Arg Arg Gln Val Thr Val Ser Ala
420 425 430
Glu Ser Ser Ser Ser Met Asn Ser Asn Thr Pro Leu Val Arg Ile Thr
435 440 445
Thr Arg Leu Ser Ser Thr Ala Asp Thr Pro Met Leu Ala Gly Val Ser

450 455 460
Glu Tyr Glu Leu Pro Glu Asp Pro Lys Trp Glu Phe Pro Arg Asp Lys
465 470 475 480
Leu Thr Leu Gly Lys Pro Leu Gly Glu Gly Cys Phe Gly Gln Val Val
485 490 495
Met Ala Glu Ala Val Gly Ile Asp Lys Asp Lys Pro Lys Glu Ala Val
500 505 510
Thr Val Ala Val Lys Met Leu Lys Asp Asp Ala Thr Glu Lys Asp Leu
515 520 525
Ser Asp Leu Val Ser Glu Met Glu Met Met Lys Met Ile Gly Lys His
530 535 540
Lys Asn Ile Ile Asn Leu Leu Gly Ala Cys Thr Gln Asp Gly Pro Leu
545 550 555 560
Tyr Val Ile Val Glu Tyr Ala Ser Lys Gly Asn Leu Arg Glu Tyr Leu
565 570 575
Arg Ala Arg Arg Pro Pro Gly Met Glu Tyr Ser Tyr Asp Ile Asn Arg
580 585 590
Val Pro Glu Glu Gln Met Thr Phe Lys Asp Leu Val Ser Cys Thr Tyr
595 600 605
Gln Leu Ala Arg Gly Met Glu Tyr Leu Ala Ser Gln Lys Cys Ile His
610 615 620
Arg Asp Leu Ala Ala Arg Asn Val Leu Val Thr Glu Asn Asn Val Met
625 630 635 640
Lys Ile Ala Asp Phe Gly Leu Ala Arg Asp Ile Asn Asn Ile Asp Tyr
645 650 655
Tyr Lys Lys Thr Thr Asn Gly Arg Leu Pro Val Lys Trp Met Ala Pro
660 665 670
Glu Ala Leu Phe Asp Arg Val Tyr Thr His Gln Ser Asp Val Trp Ser
675 680 685
Phe Gly Val Leu Met Trp Glu Ile Phe Thr Leu Gly Gly Ser Pro Tyr
690 695 700
Pro Gly Ile Pro Val Glu Glu Leu Phe Lys Leu Leu Lys Glu Gly His
705 710 715 720
Arg Met Asp Lys Pro Ala Asn Cys Thr Asn Glu Leu Tyr Met Met Met
725 730 735
Arg Asp Cys Trp His Ala Val Pro Ser Gln Arg Pro Thr Phe Lys Gln
740 745 750
Leu Val Glu Asp Leu Asp Arg Ile Leu Thr Leu Thr Thr Asn Glu Glu
755 760 765
Tyr Leu Asp Leu Ser Gln Pro Leu Glu Gln Tyr Ser Pro Ser Tyr Pro
770 775 780
Asp Thr Arg Ser Ser Cys Ser Ser Gly Asp Asp Ser Val Phe Ser Pro
785 790 795 800
Asp Pro Met Pro Tyr Glu Pro Cys Leu Pro Gln Tyr Pro His Ile Asn
805 810 815
Gly Ser Val Lys Thr
820

<210> 9
<211> 2421
<212> DNA
<213> Homo sapiens

<220>
<221> source
<222> 1..2421
<223> /mol_type="DNA"
/organism="Homo sapiens"

<400> 9
atgggcgccc ctgcctgcgc cctcgcgctc tgcgtggccg tggccatcgt ggccggcgcc 60

tcctcggagt ccttggggac ggagcagcgc gtcgtggggc gagcggcaga agtcccgggc 120

ccagagcccg gccagcagga gcagttggtc ttcggcagcg gggatgctgt ggagctgagc 180

tgtcccccgc ccgggggtgg tcccatgggg cccactgtct gggtcaagga tggcacaggg 240

ctggtgccct cggagcgtgt cctggtgggg ccccagcggc tgcaggtgct gaatgcctcc 300

cacgaggact ccggggccta cagctgccgg cagcggctca cgcagcgcgt actgtgccac 360

ttcagtgtgc gggtgacaga cgctccatcc tcgggagatg acgaagacgg ggaggacgag 420

gctgaggaca caggtgtgga cacaggggcc ccttactgga cacggcccga gcggatggac 480

aagaagctgc tggccgtgcc ggccgccaac accgtccgct tccgctgccc agccgctggc 540

aaccccactc cctccatctc ctggctgaag aacggcaggg agttccgcgg cgagcaccgc 600

attggaggca tcaagctgcg gcatcagcag tggagcctgg tcatggaaag cgtggtgccc 660

tcggaccgcg gcaactacac ctgcgtcgtg gagaacaagt ttggcagcat ccggcagacg 720

tacacgctgg acgtgctgga gcgctccccg caccggccca tcctgcaggc ggggctgccg 780

gccaaccaga cggcggtgct gggcagcgac gtggagttcc actgcaaggt gtacagtgac 840

gcacagcccc acatccagtg gctcaagcac gtggaggtga atggcagcaa ggtgggcccg 900

gacggcacac cctacgttac cgtgctcaag acggcgggcg ctaacaccac cgacaaggag 960

ctagaggttc tctccttgca caacgtcacc tttgaggacg ccggggagta cacctgcctg 1020

gcgggcaatt ctattgggtt ttctcatcac tctgcgtggc tggtggtgct gccagccgag 1080

gaggagctgg tggaggctga cgaggcgggc agtgtgtatg caggcatcct cagctacggg 1140

gtgggcttct tcctgttcat cctggtggtg gcggctgtga cgctctgccg cctgcgcagc 1200

ccccccaaga aaggcctggg ctcccccacc gtgcacaaga tctcccgctt cccgctcaag 1260

cgacaggtgt ccctggagtc caacgcgtcc atgagctcca acacaccact ggtgcgcatc 1320

gcaaggctgt cctcagggga gggccccacg ctggccaatg tctccgagct cgagctgcct 1380

gccgacccca aatgggagct gtctcgggcc cggctgaccc tgggcaagcc ccttggggag 1440

ggctgcttcg gccaggtggt catggcggag gccatcggca ttgacaagga ccgggccgcc 1500

aagcctgtca ccgtagccgt gaagatgctg aaagacgatg ccactgacaa ggacctgtcg 1560

gacctggtgt ctgagatgga gatgatgaag atgatcggga aacacaaaaa catcatcaac 1620

ctgctgggcg cctgcacgca gggcgggccc ctgtacgtgc tggtggagta cgcggccaag 1680

ggtaacctgc gggagtttct gcgggcgcgg cggcccccgg gcctggacta ctccttcgac 1740

acctgcaagc cgcccgagga gcagctcacc ttcaaggacc tggtgtcctg tgcctaccag 1800

gtggcccggg gcatggagta cttggcctcc cagaagtgca tccacaggga cctggctgcc 1860

cgcaatgtgc tggtgaccga ggacaacgtg atgaagatcg cagacttcgg gctggcccgg 1920

gacgtgcaca acctcgacta ctacaagaag acgaccaacg gccggctgcc cgtgaagtgg 1980

atggcgcctg aggccttgtt tgaccgagtc tacactcacc agagtgacgt ctggtccttt 2040

ggggtcctgc tctgggagat cttcacgctg gggggctccc cgtaccccgg catccctgtg 2100

```
gaggagctct tcaagctgct gaaggagggc caccgcatgg acaagcccgc caactgcaca    2160

cacgacctgt acatgatcat gcgggagtgc tggcatgccg cgccctccca gaggcccacc    2220

ttcaagcagc tggtggagga cctggaccgt gtccttaccg tgacgtccac cgacgagtac    2280

ctggacctgt cggcgccttt cgagcagtac tccccgggtg gccaggacac ccccagctcc    2340

agctcctcag gggacgactc cgtgtttgcc cacgacctgc tgcccccggc cccacccagc    2400

agtgggggct cgcggacgtg a                                              2421


<210> 10
<211> 806
<212> PRT
<213> Homo sapiens

<220>
<221> SOURCE
<222> 1..806
<223> /mol_type="protein"
      /organism="Homo sapiens"

<400> 10
Met Gly Ala Pro Ala Cys Ala Leu Ala Leu Cys Val Ala Val Ala Ile
1               5                   10                  15
Val Ala Gly Ala Ser Ser Glu Ser Leu Gly Thr Glu Gln Arg Val Val
            20                  25                  30
Gly Arg Ala Ala Glu Val Pro Gly Pro Glu Pro Gly Gln Gln Glu Gln
        35                  40                  45
Leu Val Phe Gly Ser Gly Asp Ala Val Glu Leu Ser Cys Pro Pro Pro
    50                  55                  60
Gly Gly Gly Pro Met Gly Pro Thr Val Trp Val Lys Asp Gly Thr Gly
65                  70                  75                  80
Leu Val Pro Ser Glu Arg Val Leu Val Gly Pro Gln Arg Leu Gln Val
                85                  90                  95
Leu Asn Ala Ser His Glu Asp Ser Gly Ala Tyr Ser Cys Arg Gln Arg
            100                 105                 110
Leu Thr Gln Arg Val Leu Cys His Phe Ser Val Arg Val Thr Asp Ala
        115                 120                 125
Pro Ser Ser Gly Asp Asp Glu Asp Gly Glu Asp Glu Ala Glu Asp Thr
    130                 135                 140
Gly Val Asp Thr Gly Ala Pro Tyr Trp Thr Arg Pro Glu Arg Met Asp
145                 150                 155                 160
Lys Lys Leu Leu Ala Val Pro Ala Ala Asn Thr Val Arg Phe Arg Cys
                165                 170                 175
Pro Ala Ala Gly Asn Pro Thr Pro Ser Ile Ser Trp Leu Lys Asn Gly
            180                 185                 190
Arg Glu Phe Arg Gly Glu His Arg Ile Gly Gly Ile Lys Leu Arg His
        195                 200                 205
Gln Gln Trp Ser Leu Val Met Glu Ser Val Val Pro Ser Asp Arg Gly
    210                 215                 220
Asn Tyr Thr Cys Val Val Glu Asn Lys Phe Gly Ser Ile Arg Gln Thr
225                 230                 235                 240
Tyr Thr Leu Asp Val Leu Glu Arg Ser Pro His Arg Pro Ile Leu Gln
                245                 250                 255
Ala Gly Leu Pro Ala Asn Gln Thr Ala Val Leu Gly Ser Asp Val Glu
            260                 265                 270
Phe His Cys Lys Val Tyr Ser Asp Ala Gln Pro His Ile Gln Trp Leu
        275                 280                 285
Lys His Val Glu Val Asn Gly Ser Lys Val Gly Pro Asp Gly Thr Pro
    290                 295                 300
Tyr Val Thr Val Leu Lys Thr Ala Gly Ala Asn Thr Thr Asp Lys Glu
305                 310                 315                 320
```

Leu Glu Val Leu Ser Leu His Asn Val Thr Phe Glu Asp Ala Gly Glu
325 330 335
Tyr Thr Cys Leu Ala Gly Asn Ser Ile Gly Phe Ser His His Ser Ala
340 345 350
Trp Leu Val Val Leu Pro Ala Glu Glu Glu Leu Val Glu Ala Asp Glu
355 360 365
Ala Gly Ser Val Tyr Ala Gly Ile Leu Ser Tyr Gly Val Gly Phe Phe
370 375 380
Leu Phe Ile Leu Val Val Ala Ala Val Thr Leu Cys Arg Leu Arg Ser
385 390 395 400
Pro Pro Lys Lys Gly Leu Gly Ser Pro Thr Val His Lys Ile Ser Arg
405 410 415
Phe Pro Leu Lys Arg Gln Val Ser Leu Glu Ser Asn Ala Ser Met Ser
420 425 430
Ser Asn Thr Pro Leu Val Arg Ile Ala Arg Leu Ser Ser Gly Glu Gly
435 440 445
Pro Thr Leu Ala Asn Val Ser Glu Leu Glu Leu Pro Ala Asp Pro Lys
450 455 460
Trp Glu Leu Ser Arg Ala Arg Leu Thr Leu Gly Lys Pro Leu Gly Glu
465 470 475 480
Gly Cys Phe Gly Gln Val Val Met Ala Glu Ala Ile Gly Ile Asp Lys
485 490 495
Asp Arg Ala Ala Lys Pro Val Thr Val Ala Val Lys Met Leu Lys Asp
500 505 510
Asp Ala Thr Asp Lys Asp Leu Ser Asp Leu Val Ser Glu Met Glu Met
515 520 525
Met Lys Met Ile Gly Lys His Lys Asn Ile Ile Asn Leu Leu Gly Ala
530 535 540
Cys Thr Gln Gly Gly Pro Leu Tyr Val Leu Val Glu Tyr Ala Ala Lys
545 550 555 560
Gly Asn Leu Arg Glu Phe Leu Arg Ala Arg Arg Pro Pro Gly Leu Asp
565 570 575
Tyr Ser Phe Asp Thr Cys Lys Pro Pro Glu Glu Gln Leu Thr Phe Lys
580 585 590
Asp Leu Val Ser Cys Ala Tyr Gln Val Ala Arg Gly Met Glu Tyr Leu
595 600 605
Ala Ser Gln Lys Cys Ile His Arg Asp Leu Ala Ala Arg Asn Val Leu
610 615 620
Val Thr Glu Asp Asn Val Met Lys Ile Ala Asp Phe Gly Leu Ala Arg
625 630 635 640
Asp Val His Asn Leu Asp Tyr Tyr Lys Lys Thr Thr Asn Gly Arg Leu
645 650 655
Pro Val Lys Trp Met Ala Pro Glu Ala Leu Phe Asp Arg Val Tyr Thr
660 665 670
His Gln Ser Asp Val Trp Ser Phe Gly Val Leu Leu Trp Glu Ile Phe
675 680 685
Thr Leu Gly Gly Ser Pro Tyr Pro Gly Ile Pro Val Glu Glu Leu Phe
690 695 700
Lys Leu Leu Lys Glu Gly His Arg Met Asp Lys Pro Ala Asn Cys Thr
705 710 715 720
His Asp Leu Tyr Met Ile Met Arg Glu Cys Trp His Ala Ala Pro Ser
725 730 735
Gln Arg Pro Thr Phe Lys Gln Leu Val Glu Asp Leu Asp Arg Val Leu
740 745 750
Thr Val Thr Ser Thr Asp Glu Tyr Leu Asp Leu Ser Ala Pro Phe Glu
755 760 765
Gln Tyr Ser Pro Gly Gly Gln Asp Thr Pro Ser Ser Ser Ser Ser Gly
770 775 780
Asp Asp Ser Val Phe Ala His Asp Leu Leu Pro Pro Ala Pro Pro Ser
785 790 795 800
Ser Gly Gly Ser Arg Thr
805

<210> 11
<211> 2409

<212> DNA
<213> Homo sapiens

<220>
<221> source
<222> 1..2409
<223> /mol_type="DNA"
/organism="Homo sapiens"

<400> 11
atgcggctgc tgctggccct gttgggggtc ctgctgagtg tgcctgggcc tccagtcttg 60

tccctggagg cctctgagga agtggagctt gagccctgcc tggctcccag cctggagcag 120

caagagcagg agctgacagt agcccttggg cagcctgtgc gtctgtgctg tgggcgggct 180

gagcgtggtg gccactggta caaggagggc agtcgcctgg cacctgctgg ccgtgtacgg 240

ggctggaggg gccgcctaga gattgccagc ttcctacctg aggatgctgg ccgctacctc 300

tgcctggcac gaggctccat gatcgtcctg cagaatctca ccttgattac aggtgactcc 360

ttgacctcca gcaacgatga tgaggacccc aagtcccata gggacccctc gaataggcac 420

agttaccccc agcaagcacc ctactggaca cacccccagc gcatggagaa gaaactgcat 480

gcagtacctg cggggaacac cgtcaagttc cgctgtccag ctgcaggcaa ccccacgccc 540

accatccgct ggcttaagga tggacaggcc tttcatgggg agaaccgcat tggaggcatt 600

cggctgcgcc atcagcactg gagtctcgtg atggagagcg tggtgccctc ggaccgcggc 660

acatacacct gcctggtaga gaacgctgtg ggcagcatcc gctataacta cctgctagat 720

gtgctggagc ggtccccgca ccggcccatc ctgcaggccg ggctcccggc caacaccaca 780

gccgtggtgg gcagcgacgt ggagctgctg tgcaaggtgt acagcgatgc ccagccccac 840

atccagtggc tgaagcacat cgtcatcaac ggcagcagct tcggagccga cggtttcccc 900

tatgtgcaag tcctaaagac tgcagacatc aatagctcag aggtggaggt cctgtacctg 960

cggaacgtgt cagccgagga cgcaggcgag tacacctgcc tcgcaggcaa ttccatcggc 1020

ctctcctacc agtctgcctg gctcacggtg ctgccagagg aggaccccac atggaccgca 1080

gcagcgcccg aggccaggta tacggacatc atcctgtacg cgtcgggctc cctggccttg 1140

gctgtgctcc tgctgctggc cgggctgtat cgagggcagg cgctccacgg ccggcacccc 1200

cgcccgcccg ccactgtgca gaagctctcc cgcttccctc tggcccgaca gttctccctg 1260

gagtcaggct cttccggcaa gtcaagctca tccctggtac gaggcgtgcg tctctcctcc 1320

agcggccccg ccttgctcgc cggcctcgtg agtctagatc tacctctcga cccactatgg 1380

gagttccccc gggacaggct ggtgcttggg aagcccctag gcgagggctg ctttggccag 1440

gtagtacgtg cagaggcctt tggcatggac cctgcccggc ctgaccaagc cagcactgtg 1500

gccgtcaaga tgctcaaaga caacgcctct gacaaggacc tggccgacct ggtctcggag 1560

atggaggtga tgaagctgat cggccgacac aagaacatca tcaacctgct tggtgtctgc 1620

acccaggaag ggcccctgta cgtgatcgtg gagtgcgccg ccaagggaaa cctgcgggag 1680

ttcctgcggg cccggcgccc cccaggcccc gacctcagcc ccgacggtcc tcggagcagt 1740

gaggggccgc tctccttccc agtcctggtc tcctgcgcct accaggtggc ccgaggcatg 1800

cagtatctgg agtcccggaa gtgtatccac cgggacctgg ctgcccgcaa tgtgctggtg 1860

actgaggaca atgtgatgaa gattgctgac tttgggctgg cccgcggcgt ccaccacatt 1920

gactactata agaaaaccag caacggccgc ctgcctgtga agtggatggc gcccgaggcc 1980

ttgtttgacc gggtgtacac acaccagagt gacgtgtggt cttttgggat cctgctatgg 2040

gagatcttca ccctcggggg ctccccgtat cctggcatcc cggtggagga gctgttctcg 2100

ctgctgcggg agggacatcg gatggaccga cccccacact gcccccccaga gctgtacggg 2160

ctgatgcgtg agtgctggca cgcagcgccc tcccagaggc ctaccttcaa gcagctggtg 2220

gaggcgctgg acaaggtcct gctggccgtc tctgaggagt acctcgacct ccgcctgacc 2280

ttcggaccct attcccccctc tggtggggac gccagcagca cctgctcctc cagcgattct 2340

gtcttcagcc acgacccccct gccattggga tccagctcct tcccccttcgg gtctggggtg 2400

cagacatga 2409

<210> 12
<211> 802
<212> PRT
<213> Homo sapiens

<220>
<221> SOURCE
<222> 1..802
<223> /mol_type="protein"
/organism="Homo sapiens"

<400> 12

```
Met Arg Leu Leu Leu Ala Leu Leu Gly Val Leu Leu Ser Val Pro Gly
1               5                   10                  15
Pro Pro Val Leu Ser Leu Glu Ala Ser Glu Glu Val Glu Leu Glu Pro
            20                  25                  30
Cys Leu Ala Pro Ser Leu Glu Gln Gln Glu Gln Glu Leu Thr Val Ala
        35                  40                  45
Leu Gly Gln Pro Val Arg Leu Cys Cys Gly Arg Ala Glu Arg Gly Gly
    50                  55                  60
His Trp Tyr Lys Glu Gly Ser Arg Leu Ala Pro Ala Gly Arg Val Arg
65                  70                  75                  80
Gly Trp Arg Gly Arg Leu Glu Ile Ala Ser Phe Leu Pro Glu Asp Ala
                85                  90                  95
Gly Arg Tyr Leu Cys Leu Ala Arg Gly Ser Met Ile Val Leu Gln Asn
            100                 105                 110
Leu Thr Leu Ile Thr Gly Asp Ser Leu Thr Ser Ser Asn Asp Asp Glu
        115                 120                 125
Asp Pro Lys Ser His Arg Asp Pro Ser Asn Arg His Ser Tyr Pro Gln
    130                 135                 140
Gln Ala Pro Tyr Trp Thr His Pro Gln Arg Met Glu Lys Lys Leu His
145                 150                 155                 160
Ala Val Pro Ala Gly Asn Thr Val Lys Phe Arg Cys Pro Ala Ala Gly
                165                 170                 175
Asn Pro Thr Pro Thr Ile Arg Trp Leu Lys Asp Gly Gln Ala Phe His
            180                 185                 190
Gly Glu Asn Arg Ile Gly Gly Ile Arg Leu Arg His Gln His Trp Ser
```

```
        195                 200                 205
Leu Val Met Glu Ser Val Val Pro Ser Asp Arg Gly Thr Tyr Thr Cys
    210                 215                 220
Leu Val Glu Asn Ala Val Gly Ser Ile Arg Tyr Asn Tyr Leu Leu Asp
225                 230                 235                 240
Val Leu Glu Arg Ser Pro His Arg Pro Ile Leu Gln Ala Gly Leu Pro
                245                 250                 255
Ala Asn Thr Thr Ala Val Val Gly Ser Asp Val Glu Leu Leu Cys Lys
            260                 265                 270
Val Tyr Ser Asp Ala Gln Pro His Ile Gln Trp Leu Lys His Ile Val
        275                 280                 285
Ile Asn Gly Ser Ser Phe Gly Ala Asp Gly Phe Pro Tyr Val Gln Val
    290                 295                 300
Leu Lys Thr Ala Asp Ile Asn Ser Ser Glu Val Glu Val Leu Tyr Leu
305                 310                 315                 320
Arg Asn Val Ser Ala Glu Asp Ala Gly Glu Tyr Thr Cys Leu Ala Gly
                325                 330                 335
Asn Ser Ile Gly Leu Ser Tyr Gln Ser Ala Trp Leu Thr Val Leu Pro
            340                 345                 350
Glu Glu Asp Pro Thr Trp Thr Ala Ala Ala Pro Glu Ala Arg Tyr Thr
        355                 360                 365
Asp Ile Ile Leu Tyr Ala Ser Gly Ser Leu Ala Leu Ala Val Leu Leu
    370                 375                 380
Leu Leu Ala Gly Leu Tyr Arg Gly Gln Ala Leu His Gly Arg His Pro
385                 390                 395                 400
Arg Pro Pro Ala Thr Val Gln Lys Leu Ser Arg Phe Pro Leu Ala Arg
                405                 410                 415
Gln Phe Ser Leu Glu Ser Gly Ser Ser Gly Lys Ser Ser Ser Ser Leu
            420                 425                 430
Val Arg Gly Val Arg Leu Ser Ser Ser Gly Pro Ala Leu Leu Ala Gly
        435                 440                 445
Leu Val Ser Leu Asp Leu Pro Leu Asp Pro Leu Trp Glu Phe Pro Arg
    450                 455                 460
Asp Arg Leu Val Leu Gly Lys Pro Leu Gly Glu Gly Cys Phe Gly Gln
465                 470                 475                 480
Val Val Arg Ala Glu Ala Phe Gly Met Asp Pro Ala Arg Pro Asp Gln
                485                 490                 495
Ala Ser Thr Val Ala Val Lys Met Leu Lys Asp Asn Ala Ser Asp Lys
            500                 505                 510
Asp Leu Ala Asp Leu Val Ser Glu Met Glu Val Met Lys Leu Ile Gly
        515                 520                 525
Arg His Lys Asn Ile Ile Asn Leu Leu Gly Val Cys Thr Gln Glu Gly
    530                 535                 540
Pro Leu Tyr Val Ile Val Glu Cys Ala Ala Lys Gly Asn Leu Arg Glu
545                 550                 555                 560
Phe Leu Arg Ala Arg Arg Pro Pro Gly Pro Asp Leu Ser Pro Asp Gly
                565                 570                 575
Pro Arg Ser Ser Glu Gly Pro Leu Ser Phe Pro Val Leu Val Ser Cys
            580                 585                 590
Ala Tyr Gln Val Ala Arg Gly Met Gln Tyr Leu Glu Ser Arg Lys Cys
        595                 600                 605
Ile His Arg Asp Leu Ala Ala Arg Asn Val Leu Val Thr Glu Asp Asn
    610                 615                 620
Val Met Lys Ile Ala Asp Phe Gly Leu Ala Arg Gly Val His His Ile
625                 630                 635                 640
Asp Tyr Tyr Lys Lys Thr Ser Asn Gly Arg Leu Pro Val Lys Trp Met
                645                 650                 655
Ala Pro Glu Ala Leu Phe Asp Arg Val Tyr Thr His Gln Ser Asp Val
            660                 665                 670
Trp Ser Phe Gly Ile Leu Leu Trp Glu Ile Phe Thr Leu Gly Gly Ser
        675                 680                 685
Pro Tyr Pro Gly Ile Pro Val Glu Glu Leu Phe Ser Leu Leu Arg Glu
    690                 695                 700
Gly His Arg Met Asp Arg Pro Pro His Cys Pro Pro Glu Leu Tyr Gly
705                 710                 715                 720
```

Leu Met Arg Glu Cys Trp His Ala Ala Pro Ser Gln Arg Pro Thr Phe
725 730 735
Lys Gln Leu Val Glu Ala Leu Asp Lys Val Leu Leu Ala Val Ser Glu
740 745 750
Glu Tyr Leu Asp Leu Arg Leu Thr Phe Gly Pro Tyr Ser Pro Ser Gly
755 760 765
Gly Asp Ala Ser Ser Thr Cys Ser Ser Ser Asp Ser Val Phe Ser His
770 775 780
Asp Pro Leu Pro Leu Gly Ser Ser Ser Phe Pro Phe Gly Ser Gly Val
785 790 795 800
Gln Thr

<210> 13
<211> 1182
<212> DNA
<213> Homo sapiens

<220>
<221> source
<222> 1..1182
<223> /mol_type="DNA"
/organism="Homo sapiens"

<400> 13
atggaggagc cgcagtcaga tcctagcgtc gagccccctc tgagtcagga aacattttca 60

gacctatgga aactacttcc tgaaaacaac gttctgtccc ccttgccgtc ccaagcaatg 120

gatgatttga tgctgtcccc ggacgatatt gaacaatggt tcactgaaga cccaggtcca 180

gatgaagctc ccagaatgcc agaggctgct ccccccgtgg cccctgcacc agcagctcct 240

acaccggcgg cccctgcacc agccccctcc tggcccctgt catcttctgt cccttcccag 300

aaaacctacc agggcagcta cggtttccgt ctgggcttct tgcattctgg gacagccaag 360

tctgtgactt gcacgtactc ccctgccctc aacaagatgt tttgccaact ggccaagacc 420

tgccctgtgc agctgtgggt tgattccaca cccccgcccg gcacccgcgt ccgcgccatg 480

gccatctaca agcagtcaca gcacatgacg gaggttgtga ggcgctgccc ccaccatgag 540

cgctgctcag atagcgatgg tctggcccct cctcagcatc ttatccgagt ggaaggaaat 600

ttgcgtgtgg agtatttgga tgacagaaac acttttcgac atagtgtggt ggtgccctat 660

gagccgcctg aggttggctc tgactgtacc accatccact acaactacat gtgtaacagt 720

tcctgcatgg gcggcatgaa ccggaggccc atcctcacca tcatcacact ggaagactcc 780

agtggtaatc tactgggacg gaacagcttt gaggtgcgtg tttgtgcctg tcctgggaga 840

gaccggcgca cagaggaaga gaatctccgc aagaaagggg agcctcacca cgagctgccc 900

ccagggagca ctaagcgagc actgcccaac aacaccagct cctctcccca gccaaagaag 960

aaaccactgg atggagaata tttcaccctt cagatccgtg ggcgtgagcg cttcgagatg 1020

ttccgagagc tgaatgaggc cttggaactc aaggatgccc aggctgggaa ggagccaggg 1080

gggagcaggg ctcactccag ccacctgaag tccaaaaagg gtcagtctac ctcccgccat 1140

aaaaaactca tgttcaagac agaagggcct gactcagact ga 1182

<210> 14
<211> 1182
<212> DNA
<213> Homo sapiens

<220>
<221> source
<222> 1..1182
<223> /mol_type="DNA"
/organism="Homo sapiens"

<400> 14
atggaggagc cgcagtcaga tcctagcgtc gagccccctc tgagtcagga aacattttca 60

gacctatgga aactacttcc tgaaaacaac gttctgtccc ccttgccgtc ccaagcaatg 120

gatgatttga tgctgtcccc ggacgatatt gaacaatggt tcactgaaga cccaggtcca 180

gatgaagctc ccagaatgcc agaggctgct ccccccgtgg cccctgcacc agcagctcct 240

acaccggcgg cccctgcacc agccccctcc tggcccctgt catcttctgt cccttcccag 300

aaaacctacc agggcagcta cggtttccgt ctgggcttct tgcattctgg gacagccaag 360

tctgtgactt gcacgtactc ccctgccctc aacaagatgt tttgccaact ggccaagacc 420

tgccctgtgc agctgtgggt tgattccaca cccccgcccg gcacccgcgt ccgcgccatg 480

gccatctaca agcagtcaca gcacatgacg gaggttgtga ggcgctgccc ccaccatgag 540

cgctgctcag atagcgatgg tctggcccct cctcagcatc ttatccgagt ggaaggaaat 600

ttgcgtgtgg agtatttgga tgacagaaac acttttcgac atagtgtggt ggtgccctat 660

gagccgcctg aggttggctc tgactgtacc accatccact acaactacat gtgtaacagt 720

tcctgcatgg gcgtcatgaa ccggaggccc atcctcacca tcatcacact ggaagactcc 780

agtggtaatc tactgggacg gaacagcttt gaggtgcgtg tttgtgcctg tcctgggaga 840

gaccggcgca cagaggaaga gaatctccgc aagaaagggg agcctcacca cgagctgccc 900

ccagggagca ctaagcgagc actgcccaac aacaccagct cctctcccca gccaaagaag 960

aaaccactgg atggagaata tttcaccctt cagatccgtg ggcgtgagcg cttcgagatg 1020

ttccgagagc tgaatgaggc cttggaactc aaggatgccc aggctgggaa ggagccaggg 1080

gggagcaggg ctcactccag ccacctgaag tccaaaaagg gtcagtctac ctcccgccat 1140

aaaaaactca tgttcaagac agaagggcct gactcagact ga 1182

<210> 15
<211> 18
<212> DNA
<213> Artificial Sequence

<220>
<221> source
<222> 1..18
<223> /mol_type="DNA"
/note="Nucleotide sequence of FGFR1 specific primer (forward)"
/organism="Artificial Sequence"

<400> 15
atgtggagct ggaagtgc 18

<210> 16
<211> 17
<212> DNA
<213> Artificial Sequence

<220>
<221> source
<222> 1..17
<223> /mol_type="DNA"
/note="Nucleotide sequence of FGFR1 specific primer (reverse)"
/organism="Artificial Sequence"

<400> 16
tcagcggcgt ttgagtc 17

<210> 17
<211> 35
<212> DNA
<213> Artificial Sequence

<220>
<221> source
<222> 1..35
<223> /mol_type="DNA"
/note="Nucleotide sequence of FGFR1 specific primers with attB ov
erhangs(forward)"
/organism="Artificial Sequence"

<400> 17
aaaaagcagg cttcaccatg tggagctgga agtgc 35

<210> 18
<211> 30
<212> DNA
<213> Artificial Sequence

<220>
<221> source
<222> 1..30
<223> /mol_type="DNA"
/note="Nucleotide sequence of FGFR1 specific primers with attB ov
erhangs(reverse)"
/organism="Artificial Sequence"

<400> 18
agaaagctgg gtctcagcgg cgtttgagtc 30

<210> 19
<211> 29
<212> DNA
<213> Artificial Sequence

<220>
<221> source
<222> 1..29
<223> /mol_type="DNA"
/note="Nucleotide sequence of attB Primers (forward)"

```
      /organism="Artificial Sequence"

<400> 19
ggggacaagt ttgtacaaaa aagcaggct                                    29


<210> 20
<211> 29
<212> DNA
<213> Artificial Sequence

<220>
<221> source
<222> 1..29
<223> /mol_type="DNA"
      /note="Nucleotide sequence of attB Primers (reverse)"
      /organism="Artificial Sequence"

<400> 20
ggggaccact ttgtacaaga aagctgggt                                    29
```

## Description

The present invention relates to a method for predicting the responsiveness of a mammalian tumor cell or cancer cell to an inhibitor of a fibroblast growth factor receptor (FGFR). Also a method for predicting the responsiveness of an individual to an inhibitor of a fibroblast growth factor receptor (FGFR) is provided. These methods involve the evaluation of the status of FGFR and the status of c-myc, whereby said status of FGFR and of c-myc is indicative for the responsiveness to the inhibitor. The status of FGFR may be its amplifcation status or its expression level and the status of c-myc may be its expression level, like an intermediate or high expression level of c-myc Furthermore, a kit useful for carrying out the methods described herein as well as an oligo- or polynucleotide and/or antibodies capable of detecting the expression level or gene amplification status of FGFR or the expression level of c-myc for predicting the responsiveness to the inhibitor are provided.

Cancer is a disease of the genome, initiated by inactivation of tumor suppressor genes by deletion, mutation or epigenetic mechanisms. Furthermore, cancer is initiated by the activation or deregulation of proto-oncogenes as a consequence of point mutation, amplification, over expression or cytogenetic abnormalities. Some of these well-known events can be successfully targeted using small molecule kinase inhibitors. This leads to better overall survival than treatment with chemo-therapy. Small molecule kinase inhibitors shut down a specific signaling pathway on which the tumor is dependent, and leads to a collapse of the tumor. A good example is treatment of EGFR mutated tumors with erlotinib (Tarceva) or gefitinib (Iressa). Another example is a fusion gene BCR-ABL1 in chronic myelogenous leukemia (CML) which is the target of the tyrosine kinase inhibitor imatinib (Gleevec). An ALK small molecule inhibitor, crizotinib (PF-02341066) has been tested in non-small-cell lung cancer patients having activating ALK rearrangments; yet certain ALK mutations may confer resistance to crizotinib (Zhu (2012) Cancer Cell 21, 362-373. One major problem of small molecule kinase inhibitor therapy is to determine whom to treat with it. For example a patient with an EGFR L858R mutation will perfectly respond to erlotinib or gefitinib, while a patient with an EGFR T790M mutation will not. Due to this fact a major roll of small molecule treatment is to determine when a patient will respond to or benefit from treatment and when not.

Fibroblast Growth Factor Receptor 1 (FGFR1) was found to be amplified in breast, bladder and lung tumors; see inter alia Dutt (2011) PloS one 6, e20351, Courjal, F. et al. Cancer Res. 57, 4360-4367 (1997) and Simon, R. et al. Cancer Res. 61, 4514-4519 (2001). Furthermore, Fibroblast Growth Factor Receptor 1 (FGFR1) is found to be mutated in glioblastoma, melanoma and lung cancer; see Greulich and Pollock (2011) Trends Mol Med 17, 283-292. It is also known that these tumors are, in principle, dependent on FGFR1 and patients with FGFR1 gene amplification may benefit from an FGFR1 small molecule kinase inhibitor therapy; see Weiss (2010) Sci Transl Med 2, 62ra93. Several FGFR inhibitors are in clinical development and clinical trials.

The FGFR family of receptor tyrosine kinases and its role in cancer development is summarized in Schildhaus (2012) Modem Pathology, 1-8. FGFR1 belongs to the type 4 family of receptor tyrosine kinases (FGFR1-4). The ligands of FGFRs, Fibroblast Growth Factors (FGFs comprise 22 family members), initiate signal transduction by binding to FGFR1-4. FGFs and FGFRs promote oncogenesis through gene amplifications, somatic mutations, and increased expression of FGFs and/or FGFRs in various human cancer types. Further, FGFR1, FGFR2 and FGFR4 are known to be overexpressed in breast cancer and an inhibitor of FGFR activity was used to inhibit growth of breast cancer cell lines. In view of the above, FGFR inhibitors can be a viable therapeutic option in the treatment of cancer patients with gene amplification or increased expression of members of the type 4 family of receptor tyrosine kinases (FGFR1-4; short FGFRs). Yet, up to now it is unknown whether such an amplification or expression of FGFRs (like FGFR1) alone is enough to predict a response to a small molecule kinase therapy with an inhibitor of FGFR.

Thus, the technical problem underlying the present invention is the provision of means and methods for the evaluation of cells, in particular tumor cells, and/or individuals for their responsiveness to anti-cancer treatment with inhibitors of the fibroblast growth factor receptor.

The technical problem is solved by provision of the embodiments characterized in the claims.

Accordingly, the present invention relates to a method for predicting the responsiveness of a mammalian tumor cell or cancer cell to an inhibitor of a fibroblast growth factor receptor (FGFR), said method comprising evaluating the status of FGFR and the status of c-myc in said cell, wherein said status of FGFR and of c-myc is indicative of the responsiveness of said cell to the inhibitor.

Alternatively, a method for predicting the responsiveness of an individual to an inhibitor of a fibroblast growth factor receptor (FGFR) is provided, said method comprising evaluating the status of FGFR and the status of c-myc in a sample of an individual suspected to suffer from cancer, suffering from cancer or being prone to suffer from cancer, wherein said status of FGFR and of c-myc is indicative of a responsive individual to the inhibitor.

The present invention solves the above identified technical problem since, as documented herein below and in the appended examples, it was surprisingly found that the evaluation of the status of FGFR, like FGFR1, and in addition the status of c-myc provides for the prediction of the responsiveness of a tumor/cancer cell or an individual to cancer/ tumor treatment with an FGFR inhibitor.

FGFR1 is a member of the fibroblast growth factor receptor (FGFR) family, where amino acid sequence is highly conserved between members and throughout evolution. FGFR family members differ from one another in their ligand affinities and tissue distribution. A full-length representative protein consists of an extracellular region, composed of two or three immunoglobulin-like domains, a single hydrophobic membrane-spanning segment and a cytoplasmic tyrosine kinase domain. The extracellular portion of the protein interacts with fibroblast growth factors, setting in motion a cascade of downstream signals. Mutations in this gene have been associated with Pfeiffer syndrome, Jackson-Weiss syndrome, Antley-Bixler syndrome, osteoglophonic dysplasia, and autosomal dominant Kallmann syndrome 2. Chromosomal aberrations involving this gene are associated with stem cell myeloproliferative disorder and stem cell leukemia lymphoma syndrome. Exemplary FGFR nucleic acid sequences and nucleic acid sequences are provided further below and can also be retrieved from the respective databases like NCBI.

In the prior art it was suggested that tumors with high-level Fibroblast Growth Factor Receptor 1 (FGFR1) gene amplification may respond better to FGFR inhibitors compared with tumors having no such FGFR1 gene amplification (see Schildhaus (2012) Modem Pathology, 1-8). The prior art was, however, silent on a potential role of the status of c-myc in predicting the response to an FGFR inhibitor.

Using a xenograft mouse model, it was surprisingly found that expression of FGFR1 alone does not sufficiently or reliably predict responsiveness to an FGFR1 inhibitor, as explained below.

In order to provide an animal model of FGFR1 expressing cancer, FGFR1 expressing NIH3T3 cells were introduced in mice. In a further experiment, NIH3T3 cells expressing FGFR1 and c-myc were introduced in mice. These cells formed tumors in the mice (see Figures 3, 5), thus demonstrating the usefulness of the xenograft mice as an animal model for individuals/patients suffering from cancer/tumors with FGFR expression (like FGFR1 expression) or likewise as an animal model for individuals/patients suffering from cancer/tumors with FGFR amplification, like FGFR1 amplification. Exemplary cancer/tumors with FGFR expression or FGFR amplification are solid cancers/tumors like lung cancer/lung tumor, breast cancer/breast tumor or bladder cancer/bladder tumor as further defined below

Furthermore, as documented in the appended examples and figures FGFR1 expression induces the expression of c-myc at an intermediate level; see Fig. 4. left column. In other words, mice into which FGFR1 expressing NIH3T3 cells were introduced (i.e. cells generated for overexpression of FGFR1, not for overexpression of c-myc) developed tumors which expressed FGFR1 (as expected) and in addition c-myc at an intermediate level.

Mice into which NIH3T3 cells expressing FGFR1 and expressing c-myc were introduced developed a more aggressive tumor type. This tumor type showed besides FGFR1 expression a high expression level of c-myc; see Figure 4, right column, bottom. It is believed that this high expression level is due to the c-myc overexpressing NIH3T3 cells and the c-myc expression induced by the FGFR1 expression.

The xenograft mice were treated with an FGFR inhibitor and the tumor volume was determined at the start and the end of the treatment. Unexpectedly, different results were obtained depending on the status of c-myc. Mice that did not show any (or very low) c-myc expression did not show a response to treatment with an FGFR inhibitor, i.e. the tumor volume increased likewise in treated and untreated mice.

Unexpectedly, mice with tumors expressing FGFR1 and, at an intermediate level, c-myc showed a stable disease phenotype, i.e. tumor growth was stopped and the tumor volume did not substantially change over the course of the treatment;; see Figures 3, 5 and 6.

Even more surprisingly, it was found that mice with tumors expressing FGFR1 and, at an high level, c-myc showed a significant reduction of the tumor volume, i.e. a tumor shrinkage; see Figure 3 and 5. The tumor completely disappeared in mice expressing both FGFR1 and, at a high level, c-myc after 10 days of treatment with the FGFR inhibitor; see Figure 3 and 5.

In vitro cell culture experiments confirmed that a FGFR inhibitor induces apoptosis of cells co-expressing FGFR1 and c-myc, if c-myc is expressed at an intermediate or high expression level, indicating that these cells are responsive to FGFR1 inhibitors; see Figure 1 and 2. A more pronounced induction of apoptosis was observed in cell cultures with or a high expression of c-myc.

The prior art did not suggest that the status of c-myc in addition to that of FGFR might have any impact on the "responsiveness of a tumor/cancer cell or an individual to an FGFR inhibitor. Indeed, no mechanistic link was made in the art between Myc and FGFR. Myc is a known multifunctional, nuclear phosphoprotein that plays a role in cell cycle progression, apoptosis and cellular transformation. It is known to function as a transcription factor that regulates transcription of specific target genes. Mutations, overexpression, rearrangement and translocation of this gene have been associated with a variety of hematopoietic tumors, leukemias and lymphomas, including Burkitt lymphoma (http://www.ncbi.nlm.nih.gov/). Thus, Myc is known as an oncogene which is often expressed in tumor cells; see Wolfer et al (2009) PNAS 107, 3698-3703.

Without being bound by theory, it is believed that the inhibition of the FGFR-Myc-mediated cell proliferation or tumor growth by an FGFR inhibitor is the reason why tumor/cancer cells or individuals with FGFR expression and intermediate or high c-myc expression are highly responsive to FGFR inhibitors, while cells or individuals expressing only FGFR and expressing no or only weakly c-myc do not show responsiveness; see the following explanations. It has

been shown in the appended examples that cells expressing only c-myc die probably due to the toxic or apoptotic activity of Myc. In contrast, it has been found that cells which co-express both FGFR1 and, at a high expression level, c-myc proliferate; they massively form colonies in soft agar or, if injected into a mouse model, they form tumours with a more aggressive phenotype than tumours with FGFR1 expression and intermediate c-myc expression. Thus, it is believed that FGFR inhibits the toxic or apoptotic activity of Myc and drives proliferation of the tumourous/cancerous cells and the tumour, respectively.

It has been observed herein that cells co-expressing FGFR1 and, at a high expression level, c-myc form more colonies in soft agar and more aggressive tumours than cells expressing FGFR1 and, at an intermediate level, c-myc, i.e. the effect of said co-expression is more pronounced than one would expect if only the toxic/apopoptotic activity of Myc was counterbalanced. In any event, the FGFR1 inhibitor completely abolished the proliferative effect seen in cells/ tumours with FGFR1/c-myc co-expression, wherein c-myc was expressed at a high level. This indicates that the FGFR1 inhibitor interfered with the proliferative effect which appears to be mediated by FGFR1 and Myc. None of the herein provided results on a potential role of both FGFR and Myc in proliferation of tumour cells or tumour formation was anticipated, let alone proposed in the art. The art did also not provide any hint that an FGFR1 inhibitor might interfere with FGFR and Myc in said cell proliferation or tumour formation. Also for this reason, the herein provided finding that cells or individuals with FGFR expression or gene amplification and intermediate or high c-myc expression respond to treatment with an FGFR1 inhibitor is surprising.

As mentioned, the present invention relates to a method for predicting the responsiveness of a mammalian tumor cell or cancer cell to an inhibitor of a fibroblast growth factor receptor (FGFR), said method comprising evaluating the status of FGFR and the status of c-myc in said cell, wherein said status of FGFR and of c-myc is indicative of the responsiveness of said cell to the inhibitor. Alternatively, the present invention relates to a method for predicting the responsiveness of a mammalian tumor cell or mammalian cancer cell to an inhibitor of a fibroblast growth factor receptor (FGFR), said method comprising obtaining a tumor cell sample (e.g. a sample of an individual suspected to suffer from cancer, suffering from cancer or being prone to suffer from cancer), evaluating the status of FGFR and the status of c-myc in said tumor cell sample, and identifying cells found to comprise such a status of FGFR and of c-myc as responsive to the inhibitor.

Further, a method for predicting the responsiveness of an individual to an inhibitor of a fibroblast growth factor receptor (FGFR) is provided, said method comprising evaluating the status of FGFR and the status of c-myc in a sample of an individual suspected to suffer from cancer, suffering from cancer or being prone to suffer from cancer, wherein said status of FGFR and of c-myc is indicative of a responsive individual to the inhibitor. Alternatively, a method for predicting the responsiveness of an individual to an inhibitor of a fibroblast growth factor receptor (FGFR) is provided, said method comprising obtaining a sample of the individual (e.g. a sample of an individual suspected to suffer from cancer, suffering from cancer or being prone to suffer from cancer), evaluating the status of FGFR and the status of c-myc in said sample, and identifying individuals of which the sample was obtained as responsive to the inhibitor, if the sample comprises said status of FGFR and of c-myc.

As used herein the term "responsiveness to the inhibitor" or "responsive individual to the inhibitor" means in the context of the present invention that a mammalian tumor cell (or mammalian tumor) or mammalian cancer cell (or mammalian cancer) or an individual suffering from cancer, suspected to suffer from or being prone to suffer from cancer shows a response to an inhibitor of FGFR (or treatment with an inhibitor of FGFR). An artisan will readily be in the position to confirm that (a) cell(s) or individual(s) predicted to be responsive to an inhibitor of FGFR (i.e. identified in accordance with the present invention) shows indeed a response to the inhibitor of FGFR (or to treatment with an inhibitor of FGFR). For example, a response to an inhibitor may be reflected in a decreased suffering from cancer, such as a diminished and/or halted growth of a tumor and/or a reduction of the size of a tumor, the prevention of the formation of metastases or a reduction of number or size of metastases.

As demonstrated in the appended examples, the herein provided method for predicting the responsiveness to an FGFR inhibitor allows for the identification of a novel patient group which is highly responsive to FGFR inhibitors. It was found herein that responsive tumor (cells), cancer (cells) or individuals identified in accordance with the present invention (e.g. individuals having (increased) FGFR gene amplification and increased (like intermediate or high) c-myc-expression; or (increased) FGFR expression and increased (like intermediate or high) c-myc-expression), show a response to an FGFR inhibitor, whereas individuals having only (increased) FGFR gene amplification or (increased) FGFR expression but absent or only low c-myc-expression do not respond. In other words, FGFR1 amplification together with c-myc expression exhibits the dependence of the tumor on FGFR signalling.

The "response" may be reflected in a "complete response" or "partial response" of the herein identified individuals/patients or tumor(s) (cell(s))/cancer(s) (cell(s) to an inhibitor of FGFR (like BGJ 398 or PD173074). Such a "complete response" or "partial response" can primarily be seen in tumor cells or individuals with FGFR gene amplification and high c-myc-expression (like a c-myc score "3" as determined by IHC); or, likewise, FGFR expression and high c-myc-expression, like a c-myc score "3" as determined by IHC. Accordingly, a "complete response" or "partial response", in particular a "complete response", is preferred.

In this context, the term "response" can refer to a "tumor shrinkage". "Tumor shrinkage" can refer to a reduced volume of the tumor upon treatment with the FGFR inhibitor compared to the initial volume at the start of (i.e. prior to) the treatment with the FGFR inhibitor.

If the tumor volume is, for example, less than about 65 % of the initial volume, the tumor has shrunk upon treatment. Such a strong reduction of the tumor volume can primarily be seen in tumor cells or individuals with FGFR gene amplification and high c-myc-expression (like a c-myc score "3" as determined by IHC); or, likewise, FGFR expression and high c-myc-expression like a c-myc score "3" as determined by IHC. A tumor volume of, for example, less than 65 % (like of from about 1 % to 64 %) compared to the initial tumor volume (100 %) at the start of (i.e. prior to) the treatment with the FGFR inhibitor can represent a "partial response" (or partial remission; e.g. a decrease in the size of a tumor, or in the extent of cancer in the body, in response to treatment with the FGFR inhibitor). A "partial response" may encompass a (temporarily) tumor shrinkage/reduction in tumor volume during the course of the treatment compared to the initial tumor volume at the start of the treatment (i.e. prior to treatment).

Preferably, the novel patient group shows a "complete response". The term "complete response" as used herein can refer to the disappearance of all signs of cancer in response to the inhibitor of FGFR (or treatment with an inhibitor of FGFR). The term "complete response" and the term "complete remission" can be used interchangeably herein. For example, a "complete response" may be reflected in the continued shrinkage of the tumor (as shown in the appended example) until the tumor has disappeared. A tumor volume of, for example, 0 % compared to the initial tumor volume (100 %) at the start of (i.e. prior to) the treatment with the FGFR inhibitor can represent a "complete response". Thus, the terms "responsiveness to the inhibitor" or "responsive individual to the inhibitor" as used herein may indicate the "complete response" /"complete responsiveness" of the tumor (cells), cancer (cells) or individuals predicted to respond in accordance with the present invention.

Tumor cells or cancer cells or individuals predicted to respond in accordance with the present invention with a FGFR amplification or FGFR expression, and intermediate c-myc-expression (like a c-myc score "2" as determined by IHC) may, for example, not show a continued or strong shrinkage of the tumor (e.g. they do not show a "complete response" or "partial response"). As demonstrated herein, such tumor (cells)/or cancer (cells)/individuals may show a "stable disease" after treatment with an inhibitor of FGFR (like BGJ 398 or PD173074), i.e. the tumor growth is essentially stopped or halted. In other words, the tumor volume does substantially not change during the course of the treatment. Such tumor cells or cancer cells or individuals (i.e. tumor (cells) or cancer (cells) or individuals having FGFR gene amplification and intermediate c-myc-expression (like a c-myc score "2" as determined by IHC), or likewise FGFR expression and intermediate c-myc-expression (like a c-myc score "2" as determined by IHC) can, therefore, show a "stable disease" phenotype. In some instances, inviduals or tumor cell(s) as defined above with an intermediate c-myc-expression can show a partial response.

Such a "stable disease" may encompass a (temporary) tumor shrinkage/reduction in tumor volume during the course of the treatment compared to the initial tumor volume at the start of the treatment (i.e. prior to treatment). In this context, "tumor shrinkage" can refer to a reduced volume of the tumor upon treatment with the FGFR inhibitor compared to the initial volume at the start of (i.e. prior to) the treatment with the FGFR inhibitor. A tumor volume of, for example, less than 100 % (e.g. of from 99 to 66 % of the initial volume at the start of the treatment) can represent a "stable disease".

A "stable disease" may alternatively encompass a (temporary) tumor growth/increase in tumor volume during the course of the treatment compared to the initial tumor volume at the start of the treatment (i.e. prior to treatment). In this context, "tumor growth" can refer to a increased volume of the tumor upon treatment with the FGFR inhibitor compared to the initial volume at the start of (i.e. prior to) the treatment with the FGFR inhibitor. A tumor volume of, for example, more than 100 % (e.g. of from 101 to 135 % of the initial volume, preferably of from 101 to 110 % of the initial volume at the start of the treatment) can represent a "stable disease".

The term "stable disease" may include the following aspects: For example, the tumor volume does, for example, either not shrink after treatment (i.e. tumor growth is halted) or it does, for example, shrink at the start of the treatment but does not continue to shrink until the tumor has disappeared (i.e. tumor growth is first reverted but, before the tumor has, for example, less than 65 % of the initial volume, the tumor grows again.

In contrast to tumor cells or cancer cells or individuals predicted to respond in accordance with the present invention (in particular cancer/tumor cells or individuals with a FGFR amplification or FGFR expression, and intermediate or high c-myc-expression (like a c-myc score ("2" or "3", respectively, in particular "3", as determined by IHC), tumor (cells) or cancer (cells) or individuals having only FGFR gene amplification or FGFR expression but no or low c-myc-expression (like a c-myc score of "0" or "1" as determine by IHC), do, for example, not show a stop/halt of tumor growth (e.g. they do not show a "stable disease" phenotype as defined above), or do, for example, not show a tumor shrinkage of less than e.g. 65 % of the initial tumor volume (e.g. they do not show a "partial response"), let alone a continued shrinkage of the tumor until disappearance (e.g. they do not show a "complete response"). Rather, tumor(s) (cells) or cancer (cells) or individuals with tumors having FGFR gene amplification and or low c-myc-expression (like a c-myc score of "0" or "1" as determined by IHC) or, likewise, FGFR expression and no or low c-myc-expression (like a c-myc score of "0" or "1" as determined by IHC), show, for example, a continued tumor growth, i.e. an increase of the tumor

volume during the course of the treatment with an FGFR inhibitor compared to the intial tumor volume at the start of the treatment (i.e. prior to treatment). For example, the tumor volume upon treatment can exceed 135 % of the initial tumor volume in tumor(s) (cells) or cancer (cells) or individuals with tumors having FGFR gene amplification and no or low c-myc-expression (like a c-myc score of "0" or "1" as determined by IHC) or, likewise, FGFR expression and no or low c-myc-expression (like a c-myc score of "0" or "1" as determined by IHC) and no or low c-myc-expression (like a c-myc score of "0" or "1" as determined by IHC). For example, the non-responsive tumor(s) (cells) or cancer (cells) or individuals as defined above show a tumor growth/increase in tumor volume upon treatment with an FGFR inhibitor that is not (preferably statistically significantly) different to a control (control being e.g. tumor(s) (cells) or cancer (cells) or individual (s) not treated with an FGFR inhibitor).

As shown in the appended example, a person skilled in the art is readily in the position to determine tumor growth or tumor shrinkage. For example, the tumor volume at the start of the treatment (i.e. prior to the treatment) with an FGFR inhibitor can be determined taking advantage of e.g. Positron emission tomography (PET) or x-ray radiograph. The tumor growth or tumor shrinkage can be monitored during the course of the treatment by taking advantage of e.g. Positron emission tomography (PET) or x-ray radiograph. Thus, the tumor volume at any given time (e.g. during treatment and/or at the end of the treatment) can be determined and the relative increase or reduction be calculated e.g. in %. Thus, it is easily possible to confirm that a tumor or cancer or a tumor/cancer patient (or individual suffering from a cancer) predicted to respond in accordance with the invention does indeed respond.

Again, cancer (cells) or tumor (cells) or individuals having FGFR1 amplification or FGFR1 expression and having an intermediate or high c-myc expression (e.g. "increased c-myc expression" as defined herein, like c-myc score "2" or "3", respectively, in particular c-myc score "3") will likely "respond" as explained above, as compared to cancer (cells) or tumor (cells) or individuals having FGFR amplification (like FGFR1 amplification) and having a low (or even absent) c-myc expression (like a c-myc score "0" or "1" determined by IHC or, likewise, having FGFR1 expression and having a low (or even absent) c-myc expression (like a c-myc score "0" or "1" determined by IHC ("0" reflects no c-myc expression and "1" reflects a low c-myc expression level).

Further, a response may be reflected in the prevention of the development of a tumor or metastases, for example after resection of a tumor, in the prolongation of time to disease progression, or in the reduction of the size of (a) tumor (s) (as explained above) and/or (a) metastases, for example in neoadjuvant therapy. A responsive individual may, for example, not suffer from cancer after treatment with the FGFR inhibitor. For example, (a) tumor(s) and/or (a) metastasis (es) which has been resected will not recur within 1 year after termination of the treatment with the inhibitor, or within 2 years, 3 years, 4 years, 5 years, 10 years or, within 15 year after termination of the treatment.

A person skilled in the art appreciates that a positive test for FGFR expression or FGFR amplification and a positive test for c-myc-expression (like intermediate or high c-myc expression (such as a score of "2" or "3", respectively, determined by IHC) (or c-myc amplification) in the novel patient sub-group identified by the herein provided methods, necessarily translates 1:1 into a successful treatment. However, by these methods sub-groups of patients are identified that have a higher chance of response (= show a better response rate) to a treatment with a FGFR inhibitor as compared to the sub-group of patients not showing these positive test results. With other words a positive result indicates that the individual or patient has a higher chance to respond to treatment with a FGFR inhibitor as compared to a patient having, for example, only (increased) FGFR expression or gene amplification and no increased expression or gene amplification above normal of the c-myc gene (e.g. no gene amplification of the c-myc gene or absent/low expression of the c-myc gene).

For example, a patient or individual having FGFR amplification and intermediate or high c-myc expression or a patient or individual having FGFR expression and intermediate high c-myc expression in a sample will likely respond better to the treatment with an FGFR-inhibitor compared to a patient or individual having only FGFR amplification or FGFR expression, and having no (or only low) c-myc expression in a sample.

It is estimated that about 20 % of all individuals or cancer patients with FGFR amplification (like FGFR1 amplification) (which usually correlates with FGFR expression, like FGFR1 expression) show high c-myc expression (e.g. highly "increased c-myc expression" as defined herein, like c-myc score " "3"). It is believed that this novel patient group can show a "partial response", like tumor shrinkage (tumor volume after treatment less than 65 % compared to initial tumor volume prior to treatment) or even or "complete response" (e.g. disappearance of the tumor).

Further, it is estimated that about 15 % of all individuals or cancer patients with FGFR amplification (like FGFR1 amplification) (which usually correlates with FGFR expression, like FGFR1 expression) show intermediate c-myc expression (e.g. a moderately "increased c-myc expression" as defined herein, like c-myc score " "2"). It is believed that this novel patient group can show a "stable disease" phenotype as defined above.

Thus, overall a novel patient group identified herein of 35 % of all individuals or cancer patients with FGFR amplification (like FGFR1 amplification) (which usually correlates with FGFR expression, like FGFR1 expression) showing intermediate or high c-myc expression (e.g. moderately or highly "increased c-myc expression" as defined herein, like c-myc score "2" or "3", in particular "3") will benefit from/respond to the treatment with an FGFR inhibitor, whereas the remaining 65 % of all individuals or cancer patients with FGFR amplification (like FGFR1 amplification) (which usually correlates with FGFR expression, like FGFR1 expression) will not benefit/not respond to the treatment.

In accordance with the above, the expression level of c-myc, like the protein expression level to be assessed in accordance with the present invention, can be intermediate or high. Preferably the expression level, like the protein expression level is high. The protein expression level of c-myc is, for example high, if it is scored "3" in immunohistochemistry (IHC). The expression level, like the protein expression level can be intermediate. The protein expression level of c-myc is, for example intermediate, if it is scored "2" in immunohistochemistry (IHC).

The expression level (like the protein expression level) of c-myc of tumors/tumor cells, cancer/cancer cells or sample of (an) individuals (e.g. cancer patient) which are not responsive (or predicted not to respond) is low or absent. The protein expression level of c-myc is, for example, low, if it is scored "0" in immunohistochemistry (IHC). The protein expression level of c-myc is, for example low, if it is scored "1" in immunohistochemistry (IHC).

As used herein, a "FGFR inhibitor" or "inhibitor of FGFR", like an FGFR1 inhibitor or inhibitor of FGFR1, refers to any compound capable of downregulating, decreasing, reducing, suppressing, inactivating or otherwise regulating the amount and/or activity of (an) FGFR(s), like FGFR1. Inhibition of these FGFRs can be achieved by any of a variety of mechanisms known in the art, including, but not limited to binding directly to the FGFR polypeptide, denaturing or otherwise inactivating the FGFR polypeptide, or inhibiting the expression of the FGFR gene (e.g., transcription to mRNA, translation to a nascent polypeptide, and/or final polypeptide modifications to a mature protein), which encodes the FGFR. Generally, FGFR inhibitors may be small molecules, proteins (e.g. antibodies), polypeptides, nucleic acids, or other chemical moieties.

As used herein the term "inhibiting" or "inhibition" refers to the ability of a compound to downregulate, decrease, reduce, suppress, inactivate, or inhibit at least partially the activity of (an) FGFR(s) polypeptide, or the expression of (an) FGFR gene(s)

The term "inhibitor of FGFR" refers accordingly to a compound capable of inhibiting the expression and/or activity of FGFR as defined herein. An "inhibitor of FGFR" may, for example, interfere with transcription of a gene encoding such FGFR, processing (e.g. splicing, export from the nucleus and the like) of the gene product (e.g. unspliced or partially spliced mRNA) and/or translation of the gene product (e.g. mature mRNA). The inhibitor of FGFR may also interfere with further modification (like phosphorylation) of the polypeptide/protein encoded by the FGFR gene and thus completely or partially inhibit the activity of FGFR. Furthermore, the inhibitor of a FGFR may interfere with interactions of FGFR with other proteins.

The following inhibitors of FGFR are preferably used in accordance with the present invention: BGJ398 or PD173074 and/or pharmaceutically acceptable salts, solvates, and/or hydrates of these inhibitors. Most preferably, BGJ398 or a pharmaceutically acceptable salt, solvate, and/or hydrate thereof is used. These and further exemplary inhibitors and pharmaceutically acceptable salts, solvates, and/or hydrates of these inhibitors to be used herein are described in more detail below. The following inhibitors are "pan FGFR inhibitors"; i.e. they are non-selective FGFR inhibitors and can, accordingly, be used to inhibit FGFR1, FGFR2, FGFR3 or FGFR4. Thus, these inhibitors are exemplary FGFR inhibitors and could likewise be termed FGFR1 inhibitor, FGFR2 inhibitor, FGFR3 inhibitor or FGFR4 inhibitor.

**Brand Name: BGJ398**

Structure

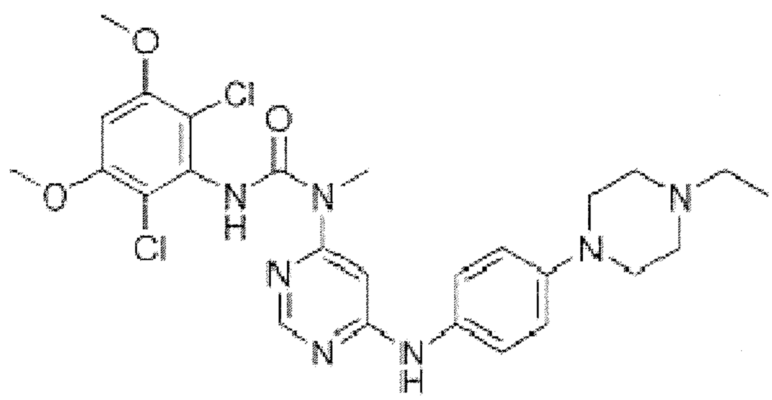

BGJ398 is commercially available e.g. from Selleckchem.

Brand Name: PD173074

Structure

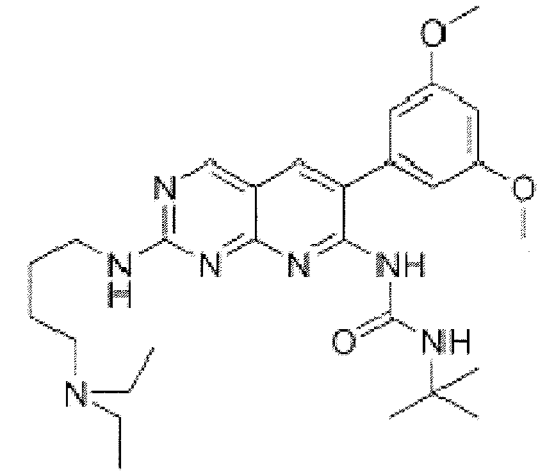

PD173074 is commercially available e.g. from Selleckchem.

Brand Name: AP24534

Structure

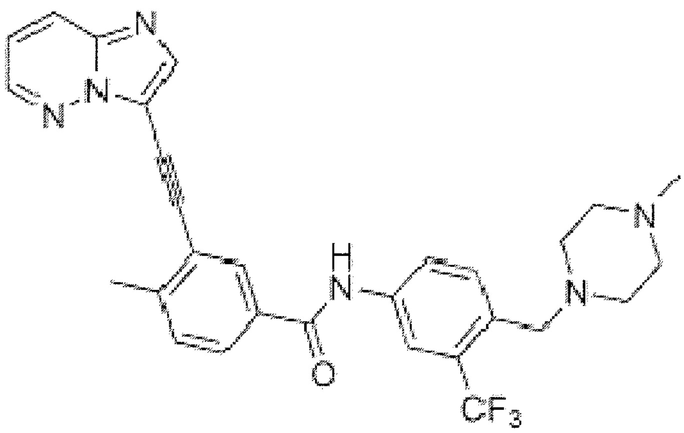

AP24534 is commercially available e.g. from Selleckchem.

**Brand Name: AZD-4547**

Structure

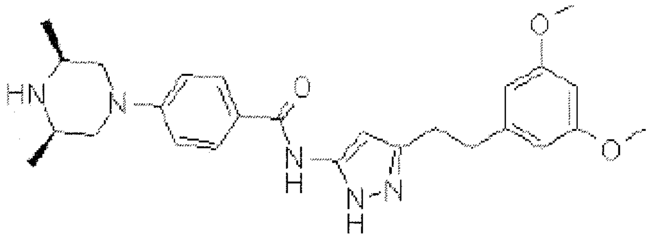

AZD-4547 is commercially available e.g. from Activebiochem.

**Brand Name: E-3810**

Structure

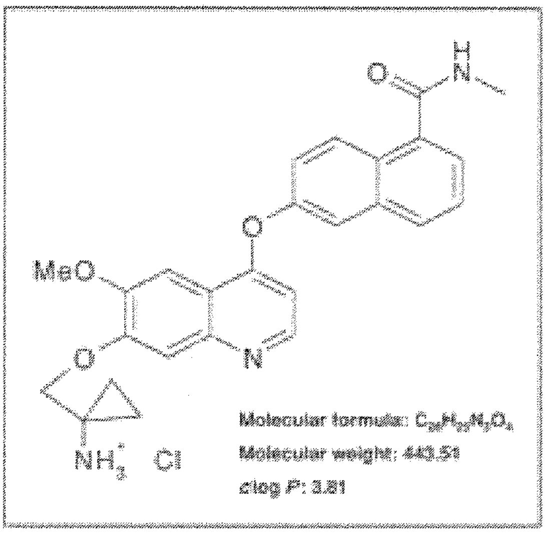


E-3810 is described in Bello et al. Cancer Res. 2011 (E-3810 Is a Potent Dual Inhibitor of VEGFR and FGFR that Exerts Antitumor Activity in Multiple Preclinical Models)

**Brand Name:** Brivanib

Structure

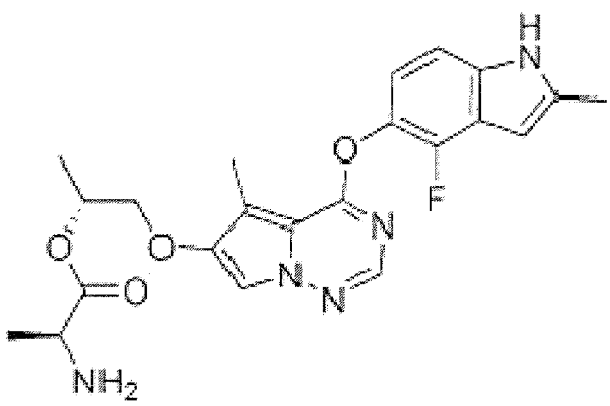

Brivanib is commercially available e.g. from Selleckchem.

**Brand Name:** Dovitinib

Structure

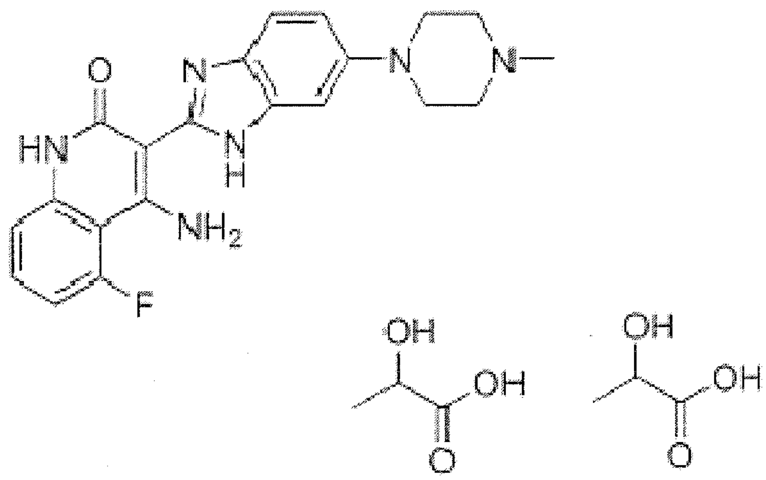

Dovitinib is commercially available e.g. from Selleckchem.

**Brand Name:** BIBF 1120

Structure

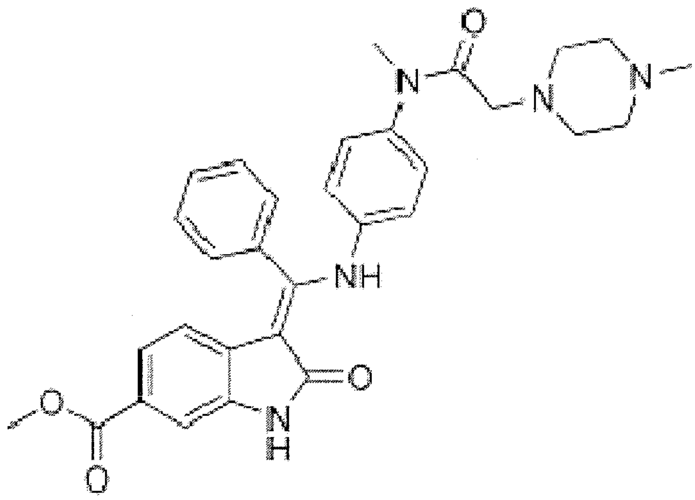

BIBF 1120 is commercially available e.g. from Selleckchem.

**Brand Name:** SU-6668

Structure

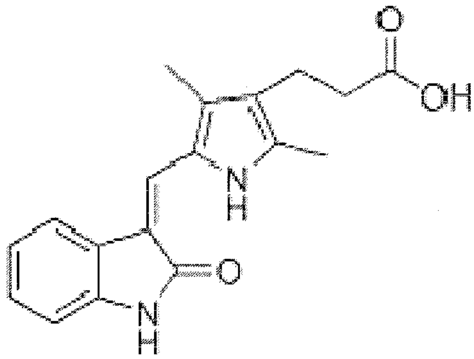

SU-6668 is commercially available e.g. from Selleckbio.

As mentioned, the above exemplary inhbitors to be used in accordance with the presnt invention are non-selective, i.e. pan FGFR-inhibitors. Also envisaged herein is the use of selective FGFR inhibitors.

Selectivity expresses the biologic fact that at a given compound concentration enzymes (or proteins) are affected to different degrees. In the case of enzymes selective inhibition can be defined as preferred inhibition by a compound at a given concentration. Or in other words, an enzyme is selectively inhibited over another enzyme when there is a concentration which results in inhibition of the first enzyme whereas the second enzyme is not affected (or second enzyme is covalent bound to the first enzyme). To compare compound effects on different enzymes it is crucial to employ similar assay formats.

The FGFR inhibitors to be used herein are, for example, specific for FGFR1, i.e. the compounds specifically inhibit FGFR1 and are therefore, selective FGFR1 inhibitors. This explanation applies mutatis mutandis to selective FGFR2, FGFR3 or FGFR4 inhibitors. In relation to predicting the responsiveness of a mammalian tumor cell or cancer cell or individual to an inhibitor of a selective FGFR inhibitor, it is to be understood that the status of the respective FGFR is to be evaluated. Any further definitions and explanations given herein applies mutatis mutandis in this context.

As described in more detail below, use of inhibitors of FGFR in accordance with the present invention is not limited to the herein described FGFR inhibitors. Also further inhibitors may be used, for example, small (binding) molecules, aptamers, intramers, antisense molecules, extracellular binding-partners, or antibody molecules such as a full antibody (immunoglobulin), a F(ab)-fragment, a F‘ab)$_2$-fragment, a single-chain antibody, a chimeric antibody, a CDR-grafted antibody, a bivalent antibody-construct, a synthetic antibody, a bispecific single chain antibody or a cross-cloned antibody. One example of an FGFR inhibitor to be used herien is FP-1039 (commercially available from Five Prime Therapeutics Inc.). FP-1039 is a soluble fusion protein consisting of the extracellular domain of human fibroblast growth factor 1c (FGFR1) linked to the Fc portion of human IgG1. FP-1039 prevent FGFR1 ligands from binding to any of their cognate receptors.

Accordingly, also yet unknown inhibitors may be used in accordance with the present invention. Such inhibitors may be identified by the methods described and provided herein and methods known in the art, like high-throughput screening using biochemical assays for inhibition of FGFR(s). Assays for screening of potential inhibitors and, in particular, for identifying inhibitors as defined herein, comprise, for example, in vitro competition binding assays to quantitatively measure interactions between test compounds and recombinantly expressed FGFR(s). Competition with immobilized capture compounds and free test compounds is performed. Test compounds that bind the FGFR active site will reduce the amount of FGFR captured on solid support, whereas test molecules that do not bind FGFR have no effect on the amount of FGFR captured on the solid support. Furthermore, inhibitor selectivity can also be assessed in parallel enzymatic assays for a set of recombinant FGFR(s). These assays are based on the measurement of the inhibitory effect of a an FGFR inhibitor and determination of the concentration of compound required for 50% inhibition of the FGFR. Proteomics methods are also an efficient tool to identify cellular targets of FGFR inhibitors.

Assays for screening of potential inhibitors and, in particular, for identifying inhibitors as defined herein, are, for example, described in the Bachovchin et. al. (2009) Nature biotechnology 27, 387-394. Based on his general knowledge a person skilled in the art is in the position to identify inhibitors or verify the inhibiting activity of compounds suspected of being inhibitors. These tests may be employed on cell(s) or cell culture(s) described in the appended example, but also further cell(s)/tissue(s)/cell culture(s) may be used, such as cell(s)/ tissue(s)/cell culture(s) derived from biopsies.

The herein provided methods for predicting or determining the responsiveness of a mammalian tumor or cancer cell to an inhibitor of FGFR or methods for predicting the responsiveness of an individual to an inhibitor of FGFR comprise the evaluation of the status of FGFR and the status of c-myc. The status is indicative of the responsiveness of said cell to the inhibitor (or to treatment with the inhibitor) or of a responsive individual to the inhibitor (or to treatment with the inhibitor), respectively.

The status of FGFR may be determined using mammalian tumor cells or mammalian cancer cells, preferably human tumor cells or human cancer cells. The cancer cell(s) or tumor cell(s) may be solid cancer cell(s) or solid tumor cell(s), like (a) lung cancer cell(s) or (a) lung tumor cell(s), such as (a) small cell lung cancer/tumor cell(s) tumor cell(s) or non-small cell lung cancer/tumor cell(s), like (a) squamous cell lung cancer cell(s) or (a) squamous cell lung tumor cell(s), or beast cancer cell(s) or breast tumor cell(s), or (a) bladder cancer cell(s) or (a) bladder tumor cell(s).

The cancer cell(s) or tumor cell(s) may also be (a) glioblastoma cancer cell(s) or (a) glioblastoma tumor cell (s), (a) melanoma cancer cell(s) or (a) melanoma tumor cell(s), (an) oral squamous carcinoma cell(s), (an) esophageal squamous cell carcinoma cell(s), (an) ovarian cancer cell(s) or (an) ovarian tumor cell(s), (an) prostate cancer cell(s) or (a) prostate tumor cell(s), or (a) rhabodomyosarcoma cell(s).

The cancer can be a solid cancer, such as lung cancer, like small cell lung cancer or non small cell lung cancer, in particular squamous cell lung cancer, breast cancer or bladder cancer.

The cancer may further be oral squamous carcinoma, esophageal squamous cell carcinoma, ovarian cancer, prostate cancer, rhabodomyosarcoma; or glioblastoma cancer or melanoma cancer.

For example, FGFR1 is found to be mutated in lung tumors/lung carcinomas, breast tumors/breast carcinomas, bladder tumors/bladder carcinomas, overian cancer, rhabdomyosarcoma, glioblastoma, melanoma; see Turner and Grose (2010) Nature 10, 116-129. and Greulich and Pollock (2011) Trends in Molecular Medicine. Furthermore, amplification or activation of FGFR1 was reported in oral squamous carcinoma, esophageal squamous cell carcinoma, ovarian cancer, prostate cancer, or rhabdomyosarcoma; see, Dutt (2011) Plos One 6, e20351 Accordingly, the cancer may be any of the above exemplary cancer types.

FGFR2 is known to be amplified in gastric cancer and breast cancer. FGFR3 is known to be amplified in bladder cancer and salivary adenoid cystic cancer. Accordingly, such cancer cell(s) or samples of an individual suffering from said cancer are to be assessed in accordance with the present invention, for example, in relation to the status of FGFR2 and FGFR3, respectively.

Squamous cell lung cancer (or squamous cell lung carcinoma which can be used interchangeably herein) is often smoking-associated and accounts for about 25 % of new lung carcinoma cases and 40 000 deaths in the United States (Schildhaus (2012), loc. cit.). About 20 % of squamous cell carcinomas show FGFR1 high- or low-level amplification; see Schildhaus loc. cit.

The status of FGFR and c-myc can be determined using a sample of an individual suspected to suffer from cancer, suffering from cancer or being prone to suffer from cancer. Said sample may, for example, be obtained by (a) biopsy (biopsies). The sample comprises mammalian tumor cells or mammalian cancer cells, preferably human tumor cells or human cancer cells as defined above, for example, (a) lung cancer cell(s) or lung tumor cell(s), such as a squamous lung cancer cell(s) or / squamous lung tumor cell(s) or (a) non-small cell lung cancer cell(s), (a) breast cancer cell(s) or (a) breast tumor cell(s), or (a) bladder cancer cell(s) or (a) bladder tumor cell(s).

The sample may alternatively comprise ((an) oral squamous carcinoma cell(s), (an) esophageal squamous cell carcinoma cell(s). (an) ovarian cancer cell(s) or (an) ovarian tumor cell(s), (an) prostate cancer cell(s) or (a) prostate tumor cell(s) (a) rhabdomyosarcoma cell(s), (a) glioblastoma cancer cell(s) or (a) glioblastoma tumor cell(s), (a) melanoma cancer cell(s) or (a) melanoma tumor cell(s).

The term "mammalian tumor cell(s)" used herein refers to (a) tumor cell(s) which is derived from or is a tumor cell from a mammal, preferably a human. The "mammalian tumor cells" may be obtained from a sample, like a biopsy, in particular a biopsy/biopsies from a patient/subject/individual suffering from cancer or suspected to suffer from cancer or, though less preferred a patient/subject/indivdual being prone to suffer from cancer. The term "tumor cell" also relates to "cancer cells". In particular, the term "mammalian tumor cell(s)" or "mammalian tumor cell(s)" can refer to a tumor, i.e. the method can be used to predict the responsiveness of a tumor to an inhibitor of a fibroblast growth factor, said method comprising evaluating the status of FGFR and the status of c-myc in a tumor cell or tumor cells (e.g. in a sample (like biopsy) of said tumor. As mentioned, such a sample can be obtained from a cancer patient. Thus, the method for predicting the responsiveness of a mammalian tumor or cancer cell (or mammalian tumor or cancer) can be used to predict whether a patient/subject/individual suffering from cancer, suspected to suffer from cancer or being prone to suffer from cancer is responsive to an FGFR inhibitor.

Preferably, said sample is from an individual suffering from, suspected to suffer from or being prone to suffer from cancer or the sample is obtained from an individual suffering from, suspected to suffer from or being prone to suffer from cancer. The cancer may be a solid cancer, such as lung cancer, like small cell lung cancer or non-small cell lung cancer, such as squamous cell lung cancer, breast cancer or bladder cancer.

The cancer can be oral squamous carcinoma, esophageal squamous cell carcinoma, ovarian cancer, prostate cancer, rhabodomyosarcoma, glioblastoma cancer or melanoma cancer. FGFR1 was found to be amplified in breast, bladder and lung tumors. FGFR1 is found to be mutated in glioblastoma, melanoma and lung. It is preferred herein that said sample is obtained from (a) tumor(s) and, accordingly, is (a) tumor cell(s) or (a) tumor tissue(s) suspected of being for example a lung tumour, like small cell lung cancer or non-small cell lung cancer, such as squamous cell lung cancer, breast cancer or bladder cancer. A person skilled in the art is in the position to identify such tumors and/or individuals/patients suffering from corresponding cancer (i.e. cancer/tumors characterised by FGFR amplifications, FGFR expression and/or activating FGFR mutations using standard techniques known in the art and methods disclosed herein. Such cancer are also described in the art, see Greulich (2011) Trends in Molecular Medicine 17, 283 and Dutt (2011) loc. cit. (as well as references therein) which are incorporated in their entirety herein.

It is also envisaged herein that mammalian tumor cells or mammalian cancer cells or samples may be tested in relation to the responsiveness to more than one FGFR inhibitor, for example, in relation to the responsiveness to two or more different FGFR inhibitors (i.e. inhibitors having different chemical formulae, optionally non-structurally related inhibitors). However, the methods of the present invention encompass the option that only the responsiveness to one inhibitor is tested at one time. Inhibitors the responsiveness to which is to be predicted in the present invention are described herein. In accordance with the above, the term "an inhibitor of FGFR" may encompass the simultaneous or subsequent test of two or more inhibitors of FGFR.

As mentioned, it has been found herein that the expression level of FGFR and the expression level of c-myc can be used to evaluate whether (a) mammalian tumor cell(s) or (a) mammalian cancer cell(s) or an individual and the like respond to an inhibitor of FGFR. Usually, the expression level of FGFR and the expression level of c-myc correlate with the gene amplification status of FGFR and c-myc, respectively; see Example 1 and Figure 8. For example, a high expression level of the FGFR gene often correlates with a high gene amplification status of the FGFR gene. Likewise, a high expression level of the c-myc gene often correlates with a high gene amplification status of the c-myc gene. Therefore, it is possible and envisaged herein that the responsiveness to an inhibitor of FGFR may be predicted by the evaluation of expression level of FGFR and the expression level of c-myc; or by the evaluation of the gene amplification status of FGFR and the expression level of c-myc; or by the evaluation of the expression level of FGFR and the gene amplification status of c-myc; or by the evaluation of the gene amplification status of FGFR and the gene amplification status of c-myc. The evaluation of the gene amplification status of FGFR or c-myc is usually sufficiently reliable for predicting the responsiveness to an inhibitor of FGFR. Yet, as shown in the appended Example 1 Figure 8, in rare instances the expression level of FGFR may not correlate with the gene amplification status of FGFR (the same holds true for the expression level of c-myc). For example, the FGFR gene may be amplified, while the FGFR gene is not expressed. In such a case, the FGFR inhibitor can (due to the absence of expressed FGFR product) not act on its FGFR target. Thus, in few instances the methods of the present invention may, by evaluation of the gene amplification level of FGFR (and/or of c-myc), indicate responsiveness of (a) mammalian tumor cell(s) or mammalian cancer cell(s) or (an) individual(s) to an FGFR inhibitor, while the mammalian tumor cell(s) or mammalian cancer cell(s) or (an) individual(s) are, in fact, not responsive. In other words, the evaluation of the gene amplification level of FGFR (and/or of c-myc) may give rise to false positives. Therefore, the evaluation of the expression level of FGFR (and/or of c-myc, particularly the expression level of FGFR and the expression level of c-myc) is believed to be advantageous as it excludes such potential false positives.

Preferably, the "FGFRs" and "c-myc" to be evaluated herein are human FGFRs (like human FGFR1) and human c-myc, respectively. Most preferably, the "FGFR" to be evaluated is human "FGFR1". Exemplary nucleic acid sequences or amino acid sequences of human FGFRs or human c-myc for the evaluation of the gene amplification status of FGFR or the expression level of FGFR and the expression level of c-myc (or the gene amplification status of c-myc) are provided herein and shown in SEQ ID NO: 1 to 12.

In accordance with the above, the present invention relates to a method for predicting the responsiveness of a mammalian tumor cell or cancer cell to an inhibitor of a fibroblast growth factor receptor (FGFR), said method comprising evaluating the expression level of FGFR and the expression level of c-myc in said cell, wherein said expression level of FGFR and of c-myc is indicative of the responsiveness of said cell to the inhibitor.

Further, the present invention relates to a method for predicting the responsiveness of an individual to an inhibitor of a fibroblast growth factor receptor (FGFR), said method comprising evaluating the expression level of FGFR and the expression level of c-myc in a sample of an individual suspected to suffer from cancer, suffering from cancer or being prone to suffer from cancer, wherein said expression level of FGFR and of c-myc is indicative of a responsive individual to the inhibitor.

The present invention relates to a method for predicting the responsiveness of a mammalian tumor cell or cancer cell to an inhibitor of a fibroblast growth factor receptor (FGFR), said method comprising evaluating the gene amplification status of FGFR and the expression level of c-myc in said cell, wherein said gene amplification status of FGFR and expression level of c-myc is indicative of the responsiveness of said cell to the inhibitor.

Further, the present invention relates to a method for predicting the responsiveness of an individual to an inhibitor of a fibroblast growth factor receptor (FGFR), said method comprising evaluating the gene amplification status of FGFR and the expression level of c-myc in a sample of an individual suspected to suffer from cancer, suffering from cancer or being prone to suffer from cancer, wherein said gene amplification status of FGFR and expression level of c-myc is indicative of a responsive individual to the inhibitor.

In accordance with the above, the present invention relates to a method for predicting the responsiveness of a mammalian tumor cell or cancer cell to an inhibitor of a fibroblast growth factor receptor (FGFR), said method comprising

evaluating the expression level of FGFR and the gene amplification status of c-myc in said cell, wherein said expression level of FGFR and gene amplification status of c-myc is indicative of the responsiveness of said cell to the inhibitor. Further, the present invention relates to a method for predicting the responsiveness of an individual to an inhibitor of a fibroblast growth factor receptor (FGFR), said method comprising evaluating the expression level of FGFR and the gene amplification status of c-myc in a sample of an individual suspected to suffer from cancer, suffering from cancer or being prone to suffer from cancer, wherein said expression level of FGFR and gene amplification status of c-myc is indicative of a responsive individual to the inhibitor.

In accordance with the above, the present invention relates to a method for predicting the responsiveness of a mammalian tumor cell or cancer cell to an inhibitor of a fibroblast growth factor receptor (FGFR), said method comprising evaluating the gene amplification status of FGFR and the gene amplification status of c-myc in said cell, wherein said amplifcation status of FGFR and gene amplification status of c-myc is indicative of the responsiveness of said cell to the inhibitor.

Further, the present invention relates to a method for predicting the responsiveness of an individual to an inhibitor of a fibroblast growth factor receptor (FGFR), said method comprising evaluating the gene amplification status of FGFR and the gene amplification status of c-myc in a sample of an individual suspected to suffer from cancer, suffering from cancer or being prone to suffer from cancer, wherein said gene amplification status of FGFR and gene amplification status of c-myc is indicative of a responsive individual to the inhibitor.

Accordingly, the status of FGFR can be the expression level of FGFR. The status of c-myc can be the expression level of c-myc. For example, an increase in said expression level of FGFR in comparison to the control and an increase in said expression level of c-myc in comparison to the control can, therefore, be indicative of the responsiveness of said cell to the inhibitor of FGFR or can be indicative of a responsive individual to the inhibitor of FGFR. Preferably, the increase is statistically significant.

The term "increase in the expression level of c-myc in comparison to the control" as used herein can refer to an intermediate expression level of c-myc or an high expression level of c-myc, for example, an intermediate protein expression level of c-myc (like a score "2" determined by IHC) or an high expression level of c-myc (like a score "3" determined by IHC). Thus, the term "increase in the expression level of c-myc in comparison to the control" as used herein can be replaced by the term "intermediate expression level of c-myc" or, preferably, by the term "high expression level of c-myc", for example, by the term "an intermediate protein expression level of c-myc" (like a score "2" determined by IHC) or, preferably, by the term "a high protein expression level of c-myc" (like a score "3" determined by IHC). All further explanations given herein in relation to the methods of the present invention apply mutatis mutandis in this context.

In accordance with the above, the present invention relates to a method for predicting the responsiveness of a mammalian tumor cell or cancer cell to an inhibitor of a fibroblast growth factor receptor (FGFR), said method comprising evaluating the expression level of FGFR and the expression level of c-myc in said cell, wherein said expression level of FGFR and of c-myc is indicative of the responsiveness of said cell to the inhibitor, and wherein an increase in said expression level of FGFR in comparison to the control and an increase in said expression level of c-myc in comparison to the control is indicative of the responsiveness of said cell to the inhibitor or indicative of a responsive individual to the inhibitor.

Further, the present invention relates to a method for predicting the responsiveness of an individual to an inhibitor of a fibroblast growth factor receptor (FGFR), said method comprising evaluating the expression level of FGFR and the expression level of c-myc in a sample of an individual suspected to suffer from cancer, suffering from cancer or being prone to suffer from cancer, wherein said expression level of FGFR and of c-myc is indicative of a responsive individual to the inhibitor, and wherein an increase in said expression level of FGFR in comparison to the control and an increase in said expression level of c-myc in comparison to the control is indicative of the responsiveness of said cell to the inhibitor or indicative of a responsive individual to the inhibitor.

In accordance with the above, the present invention relates to a method for predicting the responsiveness of a mammalian tumor cell or cancer cell to an inhibitor of a fibroblast growth factor receptor (FGFR), said method comprising evaluating the expression level of FGFR and the expression level of c-myc in said cell, wherein said expression level of FGFR and of c-myc is indicative of the responsiveness of said cell to the inhibitor, and wherein an increase in said expression level of FGFR in comparison to the control and a high protein expression level of c-myc (like a score "3" determined by IHC) is indicative of the responsiveness of said cell to the inhibitor or indicative of a responsive individual to the inhibitor.

Further, the present invention relates to a method for predicting the responsiveness of an individual to an inhibitor of a fibroblast growth factor receptor (FGFR), said method comprising evaluating the expression level of FGFR and the expression level of c-myc in a sample of an individual suspected to suffer from cancer, suffering from cancer or being prone to suffer from cancer, wherein said expression level of FGFR and of c-myc is indicative of a responsive individual to the inhibitor, and wherein an increase in said expression level of FGFR in comparison to the control and a high expression level of c-myc (like a score "3" determined by IHC) is indicative of the responsiveness of said cell to the inhibitor or indicative of a responsive individual to the inhibitor.

In accordance with the above, the present invention relates to a method for predicting the responsiveness of a mammalian tumor cell or cancer cell to an inhibitor of a fibroblast growth factor receptor (FGFR), said method comprising evaluating the expression level of FGFR and the expression level of c-myc in said cell, wherein said expression level of FGFR and of c-myc is indicative of the responsiveness of said cell to the inhibitor, and wherein an increase in said expression level of FGFR in comparison to the control and an intermediate protein expression level of c-myc (like a score "2" determined by IHC) is indicative of the responsiveness of said cell to the inhibitor or indicative of a responsive individual to the inhibitor.

Further, the present invention relates to a method for predicting the responsiveness of an individual to an inhibitor of a fibroblast growth factor receptor (FGFR), said method comprising evaluating the expression level of FGFR and the expression level of c-myc in a sample of an individual suspected to suffer from cancer, suffering from cancer or being prone to suffer from cancer, wherein said expression level of FGFR and of c-myc is indicative of a responsive individual to the inhibitor, and wherein an increase in said expression level of FGFR in comparison to the control and an intermediate expression level of c-myc (like a score "2" determined by IHC) is indicative of the responsiveness of said cell to the inhibitor or indicative of a responsive individual to the inhibitor.

In the simplest case, an increase in the expression level of FGFR in comparison to the control and/or an increase in the expression level of c-myc in comparison to the control refers to a detectable expression of FGFR and intermediate or high expression of c-myc, respectively, in a responsive cell or in a sample of an individual, whereas no expression of FGFR and no or only low expression of c-myc, respectively, can be detected in the control. In other words, an expression product of FGFR and an expression product of c-myc at an intermediate or high expression level, respectively, is present in a responsive cell or sample of a responsive individual whereas no expression product of FGFR and no or low level of expression product of c-myc is present in the control.

Likewise, an increase in the amplification status of the FGFR gene in comparison to the control and/or an increase in the amplification status of the c-myc gene in comparison to the control, respectively, refers to a detectable amplification of FGFR and/or of c-myc, respectively, in a responsive cell or in a sample of an individual, whereas no amplification of FGFR and/or of c-myc, respectively, can be detected in the control.

For the purpose of the present invention it is believed that the presence of expression product of FGFR and intermediate or high level of expression product of c-myc, respectively, or the presence of the amplification of the respective genes as defined and explained herein will suffice to predict responsiveness of cells or individuals to an FGFR inhibitor. It is believed that cells or individuals with a intermediate or high c-myc expression (or amplification of the c-myc gene) and a detectable expression (or amplification) of the FGFR gene are highly responsive to an FGFR inhibitor (in the sense of a response as explained above, like stable disease or tumor shrinkage). Accordingly, it is preferred herein that the increase in the expression level of c-myc in comparison to the control refers to a high expression of c-myc. Likewise, it is preferred herein that the increase in the amplification status of c-myc in comparison to the control refers to a high amplification status of the c-myc gene.

A "high expression" of c-myc may refer to a a protein expression level of a score of "3" as determined by immunohistochemistry (IHC).,. A "intermediate expression" of c-myc may refer to a a protein expression level of a score of "2" as determined by immunohistochemistry (IHC). As shown herein, such a score can be easily determined following routine and standardized immunohistochemical protocols, like that of Lifespan Biosciences Inc; see Example 1. An exemplary protocol according to Lifespan Biosciences Inc which can be used to determine the expression level of c-myc is provided further below.

For example, Fig 4 and 7 provides staining of samples by immunohistochemistry in order to reliably determine the protein expression level of c-myc.

The samples/cells in Fig 4, left column, upper and lower Figure show an intermediate c-myc protein expression level of score "2". A score "2" can be present if 50 % to 75 % of the cells stain positive for c-myc protein in the cytoplasm (e.g. show a brown colouring) or if up to 25 % of the cells stain positive for c-myc protein in the cytoplasm and in the nucleus. A score "2" indicates that the tumor (cells) or the cancer patient will respond to the treatment with an FGFR inhibitor, e.g. a response in form of a "stable disease" phenotype as defined herein.

The samples/cells in Fig 4, right column, lower Figure show a c-myc protein expression level of score "3", A score "3" can be present if 75 % to 100 % of the cells stain positive for c-myc protein in the cytoplasm (e.g. show a brown colouring) or if more than 25 % of the cells stain positive for c-myc protein in the nucleus. A high protein expression of c-myc, such as a score "3" determined in IHC is preferred in the methods of the present invention. A score "3" indicates that the tumor (cells) or the cancer patient will respond to the treatment with an FGFR inhibitor, e.g. a pronounced tumor shrinkage in form of a "partial response" (like a tumor volume of less than 65 % upon treatment compared with the initial tumor volume prior to treatment) or in form of a "complete response" (e.g. disappearance of the tumor).

In contrast, the control sample/cells (Fig. 4, right column, upper Figure or Fig. 7, upper Figure) does not show any staining for c-myc protein (e.g. no brown colouring). The latter sample can be therefore be regarded as a reference control herein. Said control does not show any detectable c-myc expression and has therefore a c-myc protein expression level of score "0".

A score "1" can be present if up to 50 % of the cells stain positive for c-myc protein in the cytoplasm (e.g. show a brown colouring). A score "1" indicates a low expression level of c-myc protein.

Because the expression level of c-myc and its amplification status usually correlate, it is considered that an at least twice amplification of the c-myc gene (i.e. 4 copies of the c-myc gene per cell) is an intermediate to high amplification status of the c-myc gene, i.e. corresponding to a score of "2" or "3" at the protein expression level, in particular to a score of "3" at the protein expression level.

The following is an exemplary protocol for determining the expression level of the c-myc gene (it may also be used to determine the expression level of the FGFR gene).

Tissue Preparation

- Formalin fixation and embedding in paraffin wax

*Tissue Sectioning*

- Make 4-μm sections and place on pre-cleaned and charged microscope slides.
- Heat in a tissue-drying oven for 45 minutes at 60°C

*Deparaffinization*

- Wash slides in 3 changes of xylene - 5 minutes each at room temperature.

*Rehydration*

- Wash slides in 3 changes of 100% alcohol - 3 minutes each at room temperature.
- Wash slides in 2 changes of 95% alcohol - 3 minutes each at room temperature.
- Wash slides in 1 change of 80% alcohol - 3 minutes at room temperature.
- Rinse slides in gentle running distilled water - 5 minutes at room temperature.

*Antigen retrieval*

- Steam slides in 0.01 M sodium citrate buffer, pH 6.0 at 99-100°C - 20 minutes
- Remove from heat and let stand at room temperature in buffer - 20 minutes
- Rinse in 1X TBS with Tween (TBST) - 1 minute at room temperature.

*Immunostaining*

- Do not allow tissues to dry at any time during the staining procedure.
- Apply a universal protein block - 20 minutes at room temperature.
- Drain protein block from slides, apply diluted primary antibody - 45 minutes at room temperature.
- Rinse slides in 1X TBST - 1 minute at room temperature.
- Apply a biotinylated secondary antibody (specific to the host of the primary antibody) - 30 minutes at room temperature.
- Rinse slides 1X TBST - 1 minute at room temperature.
- Apply alkaline phosphatase streptavidin - 30 minutes at room temperature.
- Rinse slides in 1X TBST - 1 minute at room temperature.

- Apply alkaline phosphatase chromogen substrate - 30 minutes at room temperature.
- Wash slides in distilled water - 1 minute at room temperature.

*Dehydrate*

- This method should only be used if the chromogen substrate is alcohol insoluble.
- Wash slides in 2 changes of 80% alcohol - 1 minute each at room temperature.
- Wash slides in 2 changes of 95% alcohol - 1 minute each at room temperature.
- Wash slides in 3 changes of 100% alcohol - 1 minute each at room temperature.
- Wash slides in 3 changes of xylene - 1 minute each at room temperature.
- Apply coverslip

In accordance with the above, the present invention relates to a method for predicting the responsiveness of a mammalian tumor cell or cancer cell to an inhibitor of a fibroblast growth factor receptor (FGFR), said method comprising evaluating the expression level of FGFR and the expression level of c-myc in said cell, wherein said expression level of FGFR and of c-myc is indicative of the responsiveness of said cell to the inhibitor, and wherein a detectable expression level of FGFR and a high protein expression level of c-myc like a score "3"as determined by IHC is indicative of the responsiveness of said cell to the inhibitor.
Further, the present invention relates to a method for predicting the responsiveness of an individual to an inhibitor of a fibroblast growth factor receptor (FGFR), said method comprising evaluating the expression level of FGFR and the expression level of c-myc in a sample of an individual suspected to suffer from cancer, suffering from cancer or being prone to suffer from cancer, wherein said expression level of FGFR and of c-myc is indicative of a responsive individual to the inhibitor, and wherein a detectable expression level of FGFR and a high protein expression level of c-myc like a score of "3" is indicative of a responsive individual to the inhibitor.
Likewise, the present invention relates to a method for predicting the responsiveness of a mammalian tumor cell or cancer cell to an inhibitor of a fibroblast growth factor receptor (FGFR), said method comprising evaluating the amplification status of the FGFR gene and the expression level of c-myc in said cell, wherein said amplification status of the FGFR gene and the expression level of c-myc is indicative of the responsiveness of said cell to the inhibitor, and wherein amplification of the FGFR gene and a high protein expression level of c-myc like a score of "3" is indicative of the responsiveness of said cell to the inhibitor.
Further, the present invention relates to a method for predicting the responsiveness of an individual to an inhibitor of a fibroblast growth factor receptor (FGFR), said method comprising evaluating the amplification status of the FGFR gene and the expression level of c-myc in a sample of an individual suspected to suffer from cancer, suffering from cancer or being prone to suffer from cancer, wherein said amplification status of the FGFR gene and the expression level of c-myc is indicative of a responsive individual to the inhibitor, and wherein an amplified FGFR gene and a high protein expression level of c-myc like a score of or "3" is indicative of a responsive individual to the inhibitor.
Further, the present invention relates to a method for predicting the responsiveness of a mammalian tumor cell or cancer cell to an inhibitor of a fibroblast growth factor receptor (FGFR), said method comprising evaluating the expression level of FGFR and the expression level of c-myc in said cell, wherein said expression level of FGFR and of c-myc is indicative of the responsiveness of said cell to the inhibitor, and wherein a detectable expression level of FGFR and an intermediate protein expression level of c-myc like a score "2" as determined by IHC is indicative of the responsiveness of said cell to the inhibitor.
Further, the present invention relates to a method for predicting the responsiveness of an individual to an inhibitor of a fibroblast growth factor receptor (FGFR), said method comprising evaluating the expression level of FGFR and the expression level of c-myc in a sample of an individual suspected to suffer from cancer, suffering from cancer or being prone to suffer from cancer, wherein said expression level of FGFR and of c-myc is indicative of a responsive individual to the inhibitor, and wherein a detectable expression level of FGFR and an intermediate protein expression level of c-myc like a score of "2" is indicative of a responsive individual to the inhibitor.
Likewise, the present invention relates to a method for predicting the responsiveness of a mammalian tumor cell or cancer cell to an inhibitor of a fibroblast growth factor receptor (FGFR), said method comprising evaluating the amplification status of the FGFR gene and the expression level of c-myc in said cell, wherein said amplification status of the FGFR gene and the expression level of c-myc is indicative of the responsiveness of said cell to the inhibitor, and wherein amplification of the FGFR gene and an intermediate protein expression level of c-myc like a score of "2" is indicative of the responsiveness of said cell to the inhibitor.
Further, the present invention relates to a method for predicting the responsiveness of an individual to an inhibitor of a fibroblast growth factor receptor (FGFR), said method comprising evaluating the amplification status of the

FGFR gene and the expression level of c-myc in a sample of an individual suspected to suffer from cancer, suffering from cancer or being prone to suffer from cancer, wherein said amplification status of the FGFR gene and the expression level of c-myc is indicative of a responsive individual to the inhibitor, and wherein an amplified FGFR gene and an intermediate protein expression level of c-myc like a score of or "2" is indicative of a responsive individual to the inhibitor.

As mentioned, a detectable expression of FGFR or a detectable amplification of the FGFR gene in combination with high or intermediate c-myc expression (or c-myc amplification) as defined herein is sufficient for a reliable prediction of the responsiveness. A detectable amplification of the FGFR gene as used herein refers to a positive test for FGFR gene amplification as defined further below (e.g. the FGFR gene is at least twice amplified, i.e. four copies per cell). It is envisaged herein that the expression level of FGFR may refer to a high expression level.

For example, said expression level of FGFR may be at least 1.5, 2.5-fold, or at least 5-fold increased in comparison to the control. The (protein) expression level of FGFR, may, for example, be at least 1.1-fold, 1.2-fold, 1.3-fold, 1.4-fold, 1.5-fold, 1.6 -fold, 1.7 -fold, 1.8-fold, 1.9-fold, 2.0-fold, 2.1-fold, 2.2-fold, 2.3-fold, 2.4-fold, 2.5-fold, 2.6-fold, 2.7-fold 2.8-fold, 2.9-fold, 3.0-fold, 3.1-fold, 3.2-fold; 3.3-fold, 3.4-fold, 3.5-fold, 4.0-fold, 5-fold, 5.5-fold, 6-fold, 7-fold, 8-fold, 9-fold, 10-fold, 11-fold, 12-fold, 13-fold, 14-fold, 15-fold or more increased in comparison to the control.

For example, said expression level of c-myc may be at least 1.5, 2.5-fold, preferably at least 5-fold increased in comparison to the control. The (protein) expression level of c-myc, may, for example, be at least 1.1-fold, 1.2-fold, 1.3-fold, 1.4-fold, 1.5-fold, 1.6-fold, 1.7-fold, 1.8-fold, 1.9-fold, 2.0-fold, 2.1-fold, 2.2-fold, 2.3-fold, 2.4-fold, even more preferably at least 2.5-fold, 2.6-fold, 2.7-fold 2.8-fold, 2.9-fold, at least 3.0-fold, 3.1-fold, 3.2-fold, 3.3-fold, 3.4-fold, 3.5-fold, 4.0-fold, 5-fold, 5.5-fold, 6-fold, at least 7-fold, 8-fold, 9-fold, 10-fold, 11-fold, 12-fold, 13-fold, 14-fold, 15-fold or more increased in comparison to the control.

It is envisaged herein that the expression level may be reflected in the activity of the gene product/protein. Accordingly, also the activity of the FGFR gene product/protein and Myc gene product/protein can be measured and evaluated in addition or in the alternative to the expression level of FGFR and c-myc, respectively, in accordance with the present invention. An increased activity of the FGFR protein is for example observed if activating mutations in the FGFR gene are present. Accordingly, and without deferring from the gist of the present invention, the "status of FGFR" to be evaluated can refer to the determination of the presence of activating mutations of the FGFR gene in a mammalian tumor cell or cancer cell or sample (like a biopsy as defined herein). All explanations and definitions given herein in relation to the methods for predicting the responsiveness of a mammalian tumor cell or cancer cell or individual/patient apply mutatis mutandis in this context. Activating mutations of the FGFR gene and methods for detecting same are well known in the art and, for example, described in Greulich (2011), loc. cit, which is incorporated herein in its entirety.

The following exemplary activating mutations of human FGFR1, FGFR2, FGFR3 or FGFR4 can be evaluated in accordance with the present invention (e.g. in the place of or in addition to the amplification status of FGFR gene or the expression of FGFR) and indicate likewise in combination with an increased c-myc expression, like an intermediate or high expression of c-myc responsiveness to an FGFR inhibitor:

FGFR1: P252S, P252T, N546K, K656E
FGFR2: S252W, P253R, S267P, W290C, K660E, K660M, K660N
FGFR3: R248C, S249C, K650E, K650M, K650N
FGFR4: N535D, V550E

FGFR proteins carrying the mutations can easily be contemplated by reference to the know and herein described respective amino acid sequences of human FGFR1, FGFR2, FGFR3 and FGF4. The mutated sequences can, for example, be derived from the Sanger database (http://www.sanger.ac.uk/genetics/CGP/cosmic/)

The expression level of FGFR may be the protein expression level of FGFR, as its evaluation provides a more reliable prediction of the responsiveness to (a) mammalian tumor cell(s) or (a) mammalian cancer cell(s) or (an) individual (s) in comparison to its RNA/mRNA expression level. The expression level of c-myc may be the protein expression level of c-myc, as its evaluation provides a more reliable prediction of the responsiveness to (a) mammalian tumor cell(s) or (a) mammalian cancer cell(s) or (an) individual(s) in comparison to its RNA/mRNA expression level As the determination (i.e. quantification) of the expression level of c-myc, for example, of the protein expression level, is routine and standardized practice in the art, the quantification of the expression level of c-myc, for example its protein expression level, is advantageous in the herein provided methods. Quantification of the protein expression level can be performed by taking advantage of the well known techniques such as IHC (immunohistochemistry). Also Western blotting techniques, immunoassays, gel- or blot-based methods, mass spectrometry, flow cytometry, FACS (fluorescence activated cell sorting) and the like may be used.

In accordance with this invention, the protein expression level of FGFR may be determined by immunohistochemical (IHC) methods employing antibodies against the FGFR protein. The patient group with an high protein expression level of FGFR (or high gene amplification status of FGFR) and high or intermediate protein expression level of c-myc (or high gene amplification status of c-myc) can easily be separated from patients with e.g. low (or absent) protein

expression level of FGFR (or low gene amplification status of FGFR) and low (or absent) (protein) expression level of c-myc (or low gene amplification status of c-myc), since a person skilled in the art is aware of standard tests, in particular of immunohistochemical tests, for such a determination of the expression level and/or gene amplification status of FGFR and c-myc, respectively. Such a test has been employed in the appended example and is described herein.

In accordance with the above, the present invention relates to a method for predicting the responsiveness of a mammalian tumor cell or cancer cell to an inhibitor of a fibroblast growth factor receptor (FGFR), said method comprising evaluating the protein expression level of FGFR and the protein expression level of c-myc in said cell, wherein said protein expression level of FGFR and of c-myc is indicative of the responsiveness of said cell to the inhibitor, and wherein an increase in said protein expression level of FGFR in comparison to the control and an increase in said protein expression level of c-myc in comparison to the control is indicative of the responsiveness of said cell to the inhibitor or indicative of a responsive individual to the inhibitor.

Further, the present invention relates to a method for predicting the responsiveness of an individual to an inhibitor of a fibroblast growth factor receptor (FGFR), said method comprising evaluating the protein expression level of FGFR and the protein expression level of c-myc in a sample of an individual suspected to suffer from cancer, suffering from cancer or being prone to suffer from cancer, wherein said protein expression level of FGFR and of c-myc is indicative of a responsive individual to the inhibitor, and wherein an increase in said protein expression level of FGFR in comparison to the control and an increase in said protein expression level of c-myc in comparison to the control is indicative of the responsiveness of said cell to the inhibitor or indicative of a responsive individual to the inhibitor.

Generally, a person skilled in the art is aware of methods for the quantitation of (a) polypeptide(s)/protein(s). Amounts of purified polypeptide in solution can be determined by physical methods, e.g. photometry. Methods of quantifying a particular polypeptide in a mixture may rely on specific binding, e.g of antibodies. Specific detection and quantitation methods exploiting the specificity of antibodies comprise for example immunohistochemistry *(in situ).* Western blotting combines separation of a mixture of proteins by electrophoresis and specific detection with antibodies. Electrophoresis may be multi-dimensional such as 2D electrophoresis. Usually, polypeptides are separated in 2D electrophoresis by their apparent molecular weight along one dimension and by their isoelectric point along the other direction. Alternatively, protein quantitation methods may involve but are not limited to mass spectrometry or enzyme-linked immunosorbant assay methods.

Also the mRNA expression level can be used in the evaluation of the expression level of FGFR and c-myc, respectively. However, the evaluation of the mRNA expression level is somewhat less reliable as compared to the herein above described protein expression level. Accordingly, the expression level of FGFR may be the mRNA expression level of FGFR. The expression level of c-myc may be the mRNA expression level of c-myc. The mRNA expression level may be assessed by methods known in the art, such as in situ hybridization, micro-arrays, RealTime PCR or RNAseq.

If the gene product is an RNA, in particular an mRNA (e.g. unspliced, partially spliced or spliced mRNA), determination can be performed by taking advantage of northern blotting techniques, in situ hybridization, hybridization on microarrays or DNA chips equipped with one or more probes or probe sets specific for mRNA transcripts or PCR techniques, like, quantitative PCR techniques, such as Real time PCR These and other suitable methods for binding (specific) mRNA are well known in the art and are, for example, described in Sambrook and Russell (2001, loc. cit.). A skilled person is capable of determining the amount of the component, in particular said gene products, by taking advantage of a correlation, preferably a linear correlation, between the intensity of a detection signal and the amount of the gene product to be determined.

As explained above, also the gene amplification status of FGFR and/or c-myc, especially of FGFR, may be evaluated in context of the herein provided methods. Accordingly, the status of FGFR may be the gene amplification status of FGFR. As mentioned, it may, however, be advantageous to evaluate the expression level of FGFR and of c-myc, such as the protein expression level of FGFR and of c-myc.

In accordance with the above, the present invention relates to a method for predicting the responsiveness of a mammalian tumor cell or cancer cell to an inhibitor of a fibroblast growth factor receptor (FGFR), said method comprising evaluating the gene amplification status of FGFR and the protein expression level of c-myc in said well, wherein said gene amplification status of FGFR and the protein expression level of c-myc is indicative of the responsiveness of said cell to the inhibitor.

Further, the present invention relates to a method for predicting the responsiveness of an individual to an inhibitor of a fibroblast growth factor receptor (FGFR), said method comprising evaluating the gene amplification status of FGFR and the protein expression level of c-myc in a sample of an individual suspected to suffer from cancer, suffering from cancer or being prone to suffer from cancer, wherein said gene amplification status of FGFR and of c-myc is indicative of a responsive individual to the inhibitor.

In accordance with the present invention, an increase in said gene amplification status of FGFR in comparison to the control and an increase in said expression level of c-myc in comparison to the control is indicative of the responsiveness of said cell to the inhibitor or indicative of a responsive individual to the inhibitor.

In accordance with the above, the present invention relates to a method for predicting the responsiveness of

a mammalian tumor cell or cancer cell to an inhibitor of a fibroblast growth factor receptor (FGFR), said method comprising evaluating the gene amplification status of FGFR and the protein expression level of c-myc in said cell, wherein said gene amplification status of FGFR and the protein expression level of c-myc is indicative of the responsiveness of said cell to the inhibitor, and wherein an increase in said gene amplification status of FGFR in comparison to the control and an increase in said expression level of c-myc in comparison to the control is indicative of the responsiveness of said cell to the inhibitor or indicative of a responsive individual to the inhibitor.

Further, the present invention relates to a method for predicting the responsiveness of an individual to an inhibitor of a fibroblast growth factor receptor (FGFR), said method comprising evaluating the gene amplification status of FGFR and the protein expression level of c-myc in a sample of an individual suspected to suffer from cancer, suffering from cancer or being prone to suffer from cancer, wherein said gene amplification status of FGFR and of c-myc is indicative of a responsive individual to the inhibitor, and wherein an increase in said gene amplification status of FGFR in comparison to the control and an increase in said expression level of c-myc in comparison to the control is indicative of the responsiveness of said cell to the inhibitor or indicative of a responsive individual to the inhibitor.

The term "gene amplification status of FGFR" as used herein may amplification of the FGFR gene. For example, the FGFR gene may be at least twice amplified (4 copies of the FGFR gene) Exemplary nucleic acid sequences of FGFR genes, for example human FGFR genes, like the human FGFR1 gene, are provided herein and shown in SEQ ID NO: 1 to 4.

It is envisaged herein that the expression level of FGFR is detected by an immunohistochemical method, whereas said FGFR gene amplification status can be determined with in situ hybridization methods, like fluorescence in situ hybridization techniques (FISH). Corresponding assays and kits are well known in the art, for protein expression assays as well as for the detection of gene amplifications. As mentioned, the evaluation of the FGFR gene amplification status may, in rare cases, produce false positive responders. Therefore, the evaluation of the gene amplification status may be followed or supplemented with the evaluation of the expression level of FGFR, such as the protein expression level. If both the assessment of FGFR expression level and FGFR gene amplification status is performed, the order of these tests can, generally, be changed, i.e. FGFR expression level tested first and vice versa, and these tests (or only the test of FGFR expression or FGFR gene amplification, if only one FGFR test is performed) can be preceded or followed by testing of c-myc expression, such as c-myc protein expression (or c-myc gene amplification). For example, c-myc expression may be tested first, followed by testing of FGFR expression level (like FGFR protein expression level e.g. determined by IHC) and, in addition or in the alternative, FGFR testing of the FGFR gene amplification status e.g. by ISH. In sum, all these tests can be performed in various orders and combinations, wherein the assessment of FGFR expression level and/or FGFR gene amplification level of the FGFR gene and the expression level of c-myc (optionally/alternatively the gene amplification level of c-myc) is mandatory for the identification of the responsive tumor (cell(s))/cancer (cell(s))/individual/individuals (e.g. patient group) in accordance with the present invention.

The FGFR gene amplification can be assessed by methods known in the art, which comprise, but are not limited to the determination of the average FGFR gene copy number in cells of a given sample or the determination of the FGFR/CEN8 ratio. Details on representative methods are provided herein below.

One exemplary scoring system to evaluate the FGFR1 gene amplification status is as follows (cells or samples being "FGFR1 gene amplification status positive" as defined below can be considered in context of the present invention as having an "increased FGFR gene amplification status"):

| FGFR1 positive ("amplified") |
|---|
| (1) the FGFR1/CEN8 ratio is ≥2.0; and/or |
| (2) the average number of FGFR1 signals per tumor cell nucleus is ≥6; and/or |
| (3) the percentage of tumor cells containing ≥15 FGFR1 signals or large clusters is ≥10 %; and/or |
| (4) the percentage of tumor cells containing ≥5 FGFR1 signals is ≥50 % |

The above system may be used in accordance with the present invention. "Positive" FGFR1 (or likewise "amplified FGFR1") according to the table above, indicates responsiveness of a mammalian tumor cell or mammalian cancer cell or of an individual. "Positive" FGFR1 (or likewise "amplified FGFR1") according to the table above relates to an "increased gene amplification status of FGFR1 as compared to the control". Points (1) to (3) represent a high level and (4) represents a low level amplification of the FGFR1 gene. Both the "low level" and "high level" of gene amplification of FGFR1 are considered to be essentially equivalent to a high expression level of FGFR1 indicating responsiveness to an FGFR inhibitor. The above scoring system is described in Schildhaus (2012, loc. cit.) in relation to the assessment of squamous cell lung cancer. Schildhaus (2012, loc. cit.) is incorporated by reference herein. It is believed that the above scoring system may be used for the assessmemt of other cancer types characterized by FGFR1 amplifications as defined and described herein, and to the assessment of cancer types characterized by amplifications of FGFR isoforms

other than FGFR1, like FGFR2, FGFR3 or FGFR4. All explanation given herein above apply mutatis mutandis to such other FGFR isoforms like FGFR2, FGFR3 or FGFR4.

In other words, the gene amplification status of FGFR indicates responsiveness to an FGFR inhibitor, if the average FGFR gene copy number is higher than 4 or higher than 5 copies or higher than 6 copies of the FGFR gene per tumor cell or cancer cell (for those test systems without an internal centromere control probe). Alternatively, the gene amplification status of FGFR indicates responsiveness if the FGFR/CEN8 ratio is higher than 2 per copy (for those test systems using an internal chromosome 8 centromere control probe). An FGFR gene copy number of 4 may, for example, arise from a duplication of 2 copies of the FGFR gene (e.g. duplication of two genes on two chromosomes or by duplication of the chromosomes carrying a copy of the FGFR gene).

In accordance with the herein provided method for predicting the responsiveness to an inhibitor of FGFR, the c-myc expression level is assessed and is increased in comparison with a control As mentioned above, c-myc expression may be classified according to 4 expression levels (scores 0, 1, 2, and 3, respectively). Figure 4 and 7 shows an example for c-myc score 0, 2 and 3. In the methods of the present invention the (protein) expression level of FGFR (e.g. FGFR1) or the gene amplification status of FGFR (e.g. FGFR1) may be evaluated, for example, by in situ hybridization (e.g. FISH) and the c-myc protein expression level may be evaluated (e.g. by immunohistochemistry (IHC).

In the alternative to the expression level of c-myc, the c-myc gene amplification status may be evaluated in the methods of the present invention in order to determine responsiveness to an FGFR inhibitor. For example, the c-myc gene may be amplified above normal. For example, the c-myc gene may be at least twice (2x; i.e. 4 copies) amplified above normal in tumor (cells), cancer (cells), or in a sample obtained from an individual suffering from, suspected to suffer from or being prone to suffer from cancer. The c-myc gene may be at least 3 times or at least 4-times amplified above normal or is at least 5-times amplified above normal. A person skilled in the art knows the normal, non-amplified status of the c-myc gene and may therefore easily determine, for example, a double /(2-times), a 3-times, a 4-times or a 5-times gene amplification status above normal of the c-myc gene. Such an amplification of the c-myc gene indicates responsiveness to an FGFR inhibitor as defined herein.

The c-myc amplification status in accordance with one of the in situ hybridization methods described herein can be determined as follows: c-myc can be considered as amplified when the ratio between the c-Myc signals and the centromer signals of chromosome 8 is above 2.0, or if the c-Myc signal per nucleus is 4 or above.

The gene amplification status of c-myc may be evaluated by in situ hybridization, such as fluorescent in situ hybridization (FISH), chromogenic in situ hybridization (CISH) and silver in situ hybridization (SISH). These methods are known to the skilled artisan. The principles of these methods can be deduced from standard text books. Commercial kits for the determination of the c-myc gene amplification status by in situ) hybridization are easily available. For example, a FISH test to be used for the determination of the gene amplification status of c-myc is the Kit "MYC/CEN-8 FISH Probe Mix" in combination with the "Histology FISH Accessory Kit" (both from the company DAKO).

The gene amplification status of the FGFR gene can be detected by an in situ hybridization method or whole genome shotgun sequencing. For example, the in situ hybridization may be fluorescent in situ hybridization (FISH), chromogenic in situ hybridization (CISH) and silver in situ hybridization (SISH).

As mentioned, for example, an increased gene amplification level of FGFR and an increased expression level of c-myc (e.g. protein expression level); or an increased expression level of FGFR (e.g. protein expression level) and an increased expression level of c-myc (e.g. protein expression level) in comparison to a control indicates responsiveness of a cell or an individual to an inhibitor of FGFR such as BGJ398 or PD173074.

As used in context of the methods of the present invention, a non-limiting example of a "control" is preferably a "non-responder" control, for example a sample/cell/tissue obtained from one or more healthy individuals or one or more patients that suffer from a cancer/tumor and are known to be not responsive to an inhibitor of FGFR. Another example for a "non-responder" control is a cell line/sample/cell/tissue that shows no response to an inhibitor of FGFR in an ex-vivo test. For example, cell line H520, SBC-7, HCC95 or H358 as used in Example 1 and Figure 8 may be considered as an exemplary "non-responder" control. Another non-limiting example of a "control" is an "internal standard", for example a mixture of purified or synthetically produced proteins and/or peptides, where the amounts of each protein/ peptide is gauged by using the "non-responder" control described above. In particular, this mixture can contain the protein of FGFR or the protein of c-myc as desribed and defined herein.

A further non-limiting example of a "control" may be a "healthy" control, for example a sample/cell/tissue obtained from a healthy individual or patient that is not suffering from a cancer/tumor or a cell obtained from such a subject. In accordance with the above, the reference or control status e.g. of FGFR or of c-myc is that determined in (a sample of) the corresponding healthy control individual/patient, i.e. it is the "normal" status of e.g. FGFR or of c-myc. The control may also be a sample/cell/tissue obtained from the individual or patient suspected of suffering from the cancer provided that the sample/cell/tissue does not contain tumor or cancer cells. In a further alternative, the "control" may be a sample/ cell/tissue obtained from an individual or patient suffering from the cancer, that has been obtained prior to the development or diagnosis of said cancer.

The meaning of the terms "cell(s)", "tissue(s)" and "cell culture(s)" is well known in the art and may, for example,

be deduced from "The Cell" (Garland Publishing, Inc., third edition). Generally, the term "cell(s) used herein refers to a single cell or a plurality of cells. The term "plurality of cells" means in the context of the present invention a group of cells comprising more than a single cell. Thereby, the cells out of said group of cells may have a similar function. Said cells may be connected cells and/or separate cells. The term "tissue" in the context of the present invention particularly means a group of cells that perform a similar function. The term "cell culture(s)" means in context of the present invention cells as defined herein above which are grown/cultured under controlled conditions. Cell culture(s) comprise in particular cells (derived/obtained) from multicellular eukaryotes, preferably animals as defined elsewhere herein. It is to be understood that the term "cell culture(s)" as used herein refers also "tissue culture (s)" and/or "organ culture(s)", an "organ" being a group of tissues which perform the same function.

Preferably, the cell(s), tissue(s) or cell culture(s) to be tested or to be used in accordance with the present invention comprise (a) mammalian tumor cell(s) or (a) mammalian cancer cell(s) or are (a) mammalian tumor cell(s) or (a) mammalian cancer cell(s). They may also be derived from (a) mammalian tumor cell(s) or (a) mammalian cancer cell(s). The tumor cells or cancer cells may, for example, be obtained from a biopsy, in particular a biopsy/biopsies from an individual suffering from cancer or from an individual prone to suffer from cancer or from an individual suspected to suffer from cancer. It is preferred herein that said individual is a human. In context of this invention particular useful cells, in particular tumor or cancer cells, are, accordingly, human cells. These human cells can be obtained from e.g. biopsies or from biological samples. The term "cell" also relates to in vitro cultured cell(s), cell culture(s) (as defined above) or cell line(s).

Generally, said tumor cell(s) or cancer cell(s) may be obtained from any biological source/organism, particularly any biological source/organism, suffering from the above-mentioned cancer, being prone to suffer from said cancer or being suspected of suffering from said cancer. Preferably, the (tumor) cell(s) or (cancer) cell to be contacted is (are) obtained/derived from an animal, more preferably, said (tumor)/(cancer) cell(s) is (are) derived from a mammal, especially of a human. Non-limiting examples for mammals are even-toed ungulates such as sheep, cattle and pig, odd-toed angulates such as horses as well as carnivores such as cats and dogs. In the context of this invention, it is envisaged that samples are derived from organisms that are economically, agronomically or scientifically important. Scientifically or experimentally important organisms include, but are not limited to, mice, rats, rabbits, guinea pigs and pigs. The tumor cell(s) may also be obtained from primates which comprise lemurs, monkeys and apes.

Preferably, the above cancer cell(s) or tumor cell(s) is/are (a) lung cancer or (a) lung tumor cell(s), such as solid tumor cells or solid cancer cells, like (a) lung cancer cell(s) or lung tumor cell(s), such as (a) small cell lung cancer cell(s) or (a) non-small cell lung cancer cell(s), like a squamous lung cancer cell(s) or / squamous lung tumor cell(s), (a) breast cancer cell(s) or (a) breast tumor cell(s) or (a) bladder cancer cell(s) or (a) bladder tumor cell(s).

The cancer cell(s) or tumor cell(s) can also be (an) oral squamous carcinoma cell(s), (an) esophageal squamous cell carcinoma cell(s), (an) ovarian cancer cell(s) or (an) ovarian tumor cell(s), (an) prostate cancer cell(s) or (a) prostate tumor cell(s), or (a) rhabodomyosarcoma cell(s) (a) glioblastoma cancer cell(s) or (a) glioblastoma tumor cell(s), (a) melanoma cancer cell(s) or (a) melanoma tumor cell(s).

Likewise, the sample preferably comprises mammalian tumor cells or mammalian cancer cells, preferably human tumor cells or human cancer cells as defined above, for example, solid tumor cells or solid cancer cells, like (a) lung cancer cell(s) or lung tumor cell(s), such as (a) small cell lung cancer cell(s) or (a) non-small cell lung cancer cell (s), like a squamous lung cancer cell(s) or / squamous lung tumor cell(s), or (a) breast cancer cell(s) or (a) breast tumor cell(s), or (a) bladder cancer cell(s) or (a) bladder tumor cell(s).

The sample can comprise or (an) oral squamous carcinoma cell(s), (an) esophageal squamous cell carcinoma cell(s), (an) ovarian cancer cell(s) or (an) ovarian tumor cell(s), (an) prostate cancer cell(s) or (a) prostate tumor cell(s), (a) rhabodomyosarcoma cell(s) or (a) glioblastoma cancer cell(s) or (a) glioblastoma tumor cell(s), (a) melanoma cancer cell(s) or (a) melanoma tumor cell(s).

The cancer is preferably a solid cancer, such as a lung cancer, like small cell lung cancer or non-small cell lung cancer, such as squamous cell lung cancer, breast cancer or bladder cancer.

The cancer can be oral squamous carcinoma, esophageal squamous cell carcinoma, ovarian cancer, prostate cancer, rhabodomyosarcoma or glioblastoma cancer, melanoma cancer. For example, FGFR1 is found to be mutated in glioblastoma, melanoma and lung. Furthermore FGFR1 was found to be amplified in breast, bladder and lung tumors.

The use of high throughput screening (HTS) is envisaged in the context of the present invention, in particular in the methods for predicting responsiveness of (a) mammalian tumor cell(s) or (a) mammalian cancer cell(s) or (an) individual(s) to an inhibitor of FGFR. HTS may be used in the evaluation of the cell(s) or sample(s) to rapidly determine the responsiveness to (an) FGFR inhibitor(s). Suitable HTS approaches are known in the art and a person skilled in the art is readily in the position to adapt such protocols or known HTS approaches to the performance of the methods of the present invention. Such approaches are also useful in the screening, identifying and/or validation of potential inhibitors of FGFR. Screening-assays are usually performed in liquid phase, wherein for each cell/tissue/cell culture to be tested at least one reaction batch is made. Typical containers to be used are micro titer plates having for example, 384, 1536, or 3456 wells (i.e. multiples of the "original" 96 reaction vessels). Robotics, data processing and control software, and

sensitive detectors, are further commonly used components of a HTS device. Often robot system are used to transport micro titer plates from station to station for addition and mixing of sample(s) and reagent(s), incubating the reagents and final readout (detection). Usually, HTS can be used in the simultaneous preparation, incubation and analysis of many plates.

The assay can be performed in a single reaction (which is usually preferred), may, however, also comprise washing and/or transfer steps. Detection can be performed taking advantage of radioactivity, luminescence or fluorescence, like fluorescence-resonance-energytransfer (FRET) and fluorescence polarisation (FP) and the like. The mammalian tumor cell(s) or mammalian cancer cell(s) or samples described herein can also be used in such a context. In particular cellular assays and in vivo assays can be employed in HTS. Cellular assays may also comprise cellular extracts, i.e. extracts from cells, tissues and the like. In the methods for predicting responsiveness of a mammalian tumor cell(s) or mammalian cancer cell(s) or (an) individual(s), the use of cell(s), tissue(s) comprising such (a) cell(s) or sample(s) comprising such a cell(s) and/or tissue(s) is envisaged (such as a sample obtained from an individual suffering from cancer, suspected from suffering from cancer or being prone to suffer from cancer). In vivo assays are useful in the validation of potential inhibitors of FGFR to be used herein.

Depending on the results of a first assay, follow up assays can be performed by re-running the experiment to collect further data on a narrowed set (e.g. samples found "positive" in the first assay), confirming and refining observations. For identifying/validation of potential inhibitor(s) of FGFR, the screening of compound libraries is envisaged herein. The screening of usually several hundred thousands of substances often takes between days and weeks. An experimental high throughput screen may be supplemented (or even be replaced) by a virtual screen. For example, if the structure of the target molecule (e.g. a kinase of FGFR) is known, methods can be employed, which are known under the term "docking". If the structure of several target-binding molecules is known (e.g. the herein described inhibitor of FGFR) methods for Pharmacophor-Modelling can be used aiming at the development new substances which also bind to the target molecule.

The FGFR whose status is to be evaluated herein may be FGFR1. Likewise, the inhibitor of FGFR may be an inhibitor of FGFR1. Accordingly, the present invention relates in accordance with the above to a method for predicting the responsiveness of a mammalian tumor cell or cancer cell to an inhibitor of fibroblast growth factor receptor 1 (FGFR1), said method comprising evaluating the status of FGFR1 and the status of c-myc in said cell, wherein said status of FGFR1 and the status of c-myc is indicative of the responsiveness of said cell to the inhibitor.

Further, the present invention relates to a method for predicting the responsiveness of an individual to an inhibitor of fibroblast growth factor receptor 1 (FGFR1), said method comprising evaluating the status of FGFR1 and the status of c-myc in a sample of an individual suspected to suffer from cancer, suffering from cancer or being prone to suffer from cancer, wherein said status of FGFR1 and of c-myc is indicative of a responsive individual to the inhibitor.

In accordance with the above, the present invention relates to a method for predicting the responsiveness of a mammalian tumor cell or cancer cell to an inhibitor of a fibroblast growth factor receptor 1 (FGFR1), said method comprising evaluating the gene amplification status of FGFR1 and the protein expression level of c-myc in said cell, wherein said gene amplification status of FGFR1 and the protein expression level of c-myc is indicative of the responsiveness of said cell to the inhibitor.

Further, the present invention relates to a method for predicting the responsiveness of an individual to an inhibitor of a fibroblast growth factor receptor 1 (FGFR1), said method comprising evaluating the gene amplification status of FGFR1 and the protein expression level of c-myc in a sample of an individual suspected to suffer from cancer, suffering from cancer or being prone to suffer from cancer, wherein said gene amplification status of FGFR1 and said protein expression level of c-myc is indicative of a responsive individual to the inhibitor.

In accordance with the above, the present invention relates to a method for predicting the responsiveness of a mammalian tumor cell or cancer cell to an inhibitor of a fibroblast growth factor receptor 1 (FGFR1), said method comprising evaluating the gene amplification status of FGFR1 and the protein expression level of c-myc in said cell, wherein said gene amplification status of FGFR1 and the protein expression level of c-myc is indicative of the responsiveness of said cell to the inhibitor, and wherein an increase in said gene amplification status of FGFR1 in comparison to the control and an increase in said expression level of c-myc in comparison to the control is indicative of the responsiveness of said cell to the inhibitor.

Further, the present invention relates to a method for predicting the responsiveness of an individual to an inhibitor of a fibroblast growth factor receptor 1 (FGFR1), said method comprising evaluating the gene amplification status of FGFR1 and the protein expression level of c-myc in a sample of an individual suspected to suffer from cancer, suffering from cancer or being prone to suffer from cancer, wherein said gene amplification status of FGFR1 and said protein expression level of c-myc is indicative of a responsive individual to the inhibitor, and wherein an increase in said gene amplification status of FGFR1 in comparison to the control and an increase in said expression level of c-myc in comparison to the control is indicative of a responsive individual to the inhibitor.

In accordance with the above, the present invention relates to a method for predicting the responsiveness of a mammalian tumor cell or cancer cell to an inhibitor of a fibroblast growth factor receptor 1 (FGFR1), said method

comprising evaluating the protein expression level of FGFR1 and the protein expression level of c-myc in said cell, wherein said protein expression level of FGFR1 and the protein expression level of c-myc is indicative of the responsiveness of said cell to the inhibitor.

Further, the present invention relates to a method for predicting the responsiveness of an individual to an inhibitor of a fibroblast growth factor receptor 1 (FGFR1), said method comprising evaluating the protein expression level of FGFR1 and the protein expression level of c-myc in a sample of an individual suspected to suffer from cancer, suffering from cancer or being prone to suffer from cancer, wherein said protein expression level of FGFR1 and of c-myc is indicative of a responsive individual to the inhibitor.

In accordance with the above, the present invention relates to a method for predicting the responsiveness of a mammalian tumor cell or cancer cell to an inhibitor of a fibroblast growth factor receptor 1 (FGFR1), said method comprising evaluating the protein expression level of FGFR1 and the protein expression level of c-myc in said cell, wherein said protein expression level of FGFR1 and the protein expression level of c-myc is indicative of the responsiveness of said cell to the inhibitor, and wherein an increase in said protein expression level of FGFR in comparison to the control and an increase in said protein expression level of c-myc in comparison to the control is indicative of the responsiveness of said cell to the inhibitor.

Further, the present invention relates to a method for predicting the responsiveness of an individual to an inhibitor of a fibroblast growth factor receptor 1 (FGFR1), said method comprising evaluating the protein expression level of FGFR1 and the protein expression level of c-myc in a sample of an individual suspected to suffer from cancer, suffering from cancer or being prone to suffer from cancer, wherein said protein expression level of FGFR1 and of c-myc, is indicative of a responsive individual to the inhibitor, and wherein an increase in said protein expression level of FGFR in comparison to the control and an increase in said protein expression level of c-myc in comparison to the control is indicative of the responsiveness of said cell to the inhibitor or indicative of a responsive individual to the inhibitor.

The term "FGFR" and "FGFR1" etc. are defined herein above and further below. Preferably the FGFR (like FGFR1 is a human FGFR (like a human FGFR1). Exemplary sequences are shown in SEQ ID NO: 1 to 4.

Furthermore, it was found herein that the responsive cell line H1581 shows wild type p53 expression. Accordingly, the herein provided methods may comprise a further step of evaluating the status of p53 in a tumor cell or in a sample of an individual suspected to suffer from cancer, suffering from cancer or being prone to suffer from cancer, wherein the status of p53 is indicative of the responsiveness of the cell to the inhibitor or is indicative of a responsive individual to the inhibitor. For example, the status of p53 indicating responsiveness may be the presence of wild type p53 expression product in said cell(s) or sample. The presence of a mutated p53 expression product (e.g. a loss of function mutated p53) can indicate that the cell or the sample is not responsive to the FGFR inhibitor. Exemplary nucleic acid sequences encoding a wild-type p53 and a mutated p53 are shown in SEQ ID NO. 13 and 14, respectively. The herein provided means and methods for detecting expression products can be applied in this context.

Also provided herein is FGFR and c-myc for use in detecting an individual responsive to an FGFR inhibitor, for example one or more of the following FGFR inhibitor(s): BGJ398, PD173074, AP24534, AZD-4547, and E-3810 and pharmaceutically acceptable salts, solvates, and/or hydrates of these inhibitors.

Further, the present invention provides the use of a nucleic acid or antibody capable of detecting the gene amplification status or the expression level of FGFR and/or the use of a nucleic acid or antibody capable of detecting the expression level of c-myc for predicting the responsiveness of a cancer or tumor cell (such as a mammalian cancer cell or mammalian tumor cell) or a responsive individual to an FGFR inhibitor like BGJ398, PD173074, AP24534, AZD-4547, and E-3810 and pharmaceutically acceptable salts, solvates, and/or hydrates of these inhibitors.

The oligonucleotide(s) may be about 15 to 100 nucleotides in length. A person skilled in the art is, based on his general knowledge and the teaching provided herein, easily in the position to identify and/or prepare (a) an oligo- or polynucleotide capable of detecting the expression level of FGFR and/or c-myc. These oligo- or polynucleotides may be used as probe(s) in the methods for predicting the responsiveness to an inhibitor of FGFR described herein. A skilled person will know, for example, computer programs which may be useful for the identification of corresponding probes to be used herein. For example, the nucleic acid sequence(s) of exemplary FGFR and/or c-myc coding sequences as disclosed herein (SEQ ID NO: 1 to 12) may be used in this context. Exemplary nucleic acid sequences are also available on corresponding databases, such as the Ensemble database (http://www.ensembl.org/index.html).

Furthermore, the present invention provides a kit useful for carrying out the method of the herein provided methods, the kit comprising a nucleic acid or an antibody capable of detecting the gene amplification status or the expression level of FGFR and/or comprising a nucleic acid or an antibody capable of detecting the expression level of c-myc. Also envisaged herein is the use of the herein described kit for carrying out the herein provided methods.

For example, said kit may comprise (a) compound(s) required for specifically determining the expression level or gene amplification status of FGFR and/or of c-myc. Moreover, the present invention also relates to the use of (a) compound(s) required for specifically determining the expression level or gene amplification status of FGFR and/or of c-myc for the preparation of a kit for carrying out the methods of this invention. On the basis of the teaching of this invention, the skilled person knows which compound(s) is (are) required for specifically determining the expression level

or gene amplification status of FGFR and/or of c-myc. For example, such compound(s) may be (a) "binding molecule (s)", like, for example, (a) "binding molecule(s)" Particularly, such compound(s) may be (a) (nucleotide) probe(s), (a) primer(s) (pair(s)), (an) antibody(ies) and/or (an) aptamer(s) specific for at least one marker gene as described herein or for a product thereof. The kit (to be prepared in context) of this invention may be a diagnostic kit.

The kit (to be prepared in context) of this invention may further comprise or be provided with (an) instruction manual(s). For example, said instruction manual(s) may guide the skilled person (how) to determine the (reference/control) expression level or gene amplification status of FGFR and/or of c-myc and/or (how) to diagnose responsiveness to an inhibitor of FGFR. Said instruction manual(s) may comprise guidance to use or apply the herein provided methods or uses. The kit (to be prepared in context) of this invention may further comprise substances/chemicals and/or equipment suitable/required for carrying out the methods and uses of this invention. For example, such substances/chemicals and/or equipment are solvents, diluents and/or buffers for stabilizing and/or storing (a) compound(s) required for specifically determining the gene amplification status or the expression level of FGFR and/or of c-myc.

Furthermore, the present invention relates to an inhibitor of FGFR (or likewise FGFR inhibitor) as described and defined herein above for use in the treatment of cancer in an individual or patient identified by the herein described methods. The present invention also provides a method for the treatment of cancer comprising administering an effective amount of an inhibitor of FGFR as defined herein to an individual or subject identified by the herein described methods in need of such a treatment. Preferably, said individual or said subject is a human. The present invention also relates to the use of an inhibitor of a kinase of the Src family as defined herein for the preparation of a pharmaceutical composition for the treatment of a patient identified by the herein provided methods.

As explained above, the herein provided methods allow for a reliable determination or prediction of the responsiveness to an inhibitor of FGFR. For such a reliable prediction it is sufficient that the status of FGFR and the status of c-myc is evaluated using (a) mammalian tumor cell(s) or (a) mammalian cancer cell(s) or a sample of an individual. By taking advantage of the herein provided methods, an individual or individuals (e.g. a patient group) which are responsive to an inhibitor of FGFR (or which is/are likely to respond to an inhibitor of FGFR) may, thus, be identified. Therefore, an "individual identified by the herein provided methods" is an individual that is predicted to respond to an inhibitor of FGFR in accordance with the herein provided methods. The terms "individual identified by the herein provided methods" and "individual predicted to respond to an inhibitor of FGFR in accordance with the herein provided methods" may be used herein interchangeably.

As mentioned above, individuals having an (increased) (protein) expression level of FGFR (or an (increased) gene amplification status of FGFR) in comparison to a control and an increased (protein) expression level of c-myc (or an increased gene amplification status of c-myc) in comparison to a control show a much better response to an inhibitor of FGFR (e.g. cancer therapy/ cancer treatment with an inhibitor of FGFR) than an individual having an (increased) (protein) expression level of FGFR (or an (increased) gene amplification status of FGFR) in comparison to a control but no (increased) (protein) expression level of c-myc (or no increased gene amplification status of c-myc), like no or low c-myc expression, in comparison to a control. Therefore, this individual or these individuals (e.g. group of patients) predicted to respond to an inhibitor of FGFR strongly benefit from treatment or therapy with an inhibitor of FGFR whereas other individuals do not respond. For example, it is expected that an individual/individuals (e.g. group of patients) predicted to respond to an inhibitor of FGFR show a tumor shrinkage or stable disease phenotype whereas an individual/individuals predicted not to respond to an inhibitor of FGFR ("non-responders") do not show such a tumor shrinkage or stable disease phenotype.

Preferably, the cancer to be treated is characterized by or associated with gene amplification of FGFR or (protein) expression of FGFR and (protein) expression of c-myc. The cancer may be characterized by or associated with FGFR protein overexpression and c-myc protein overexpression (i.e. the expression level of FGFR and c-myc in a tumor/cancer sample is (preferably statistically significantly higher) compared to a control sample, such as a "non-responder" or "non responsive" control (e.g. cell line H520, SBC-7, HCC95 and H358 as used in Example 1 and Figure 8 and 10).

Exemplary tumors / cancer to be treated in accordance with the present invention which are characterized by or associated with gene amplification of FGFR or (protein) expression of FGFR and (protein) expression of c-myc are solid cancer/tumors, such as a lung cancer, like small cell lung cancer or non-small cell lung cancer, such as squamous cell lung cancer, breast cancer or bladder cancer. Also the treatment of oral squamous carcinoma, esophageal squamous cell carcinoma, ovarian cancer, prostate cancer, rhabodomyosarcoma; or glioblastoma cancer or melanoma cancer is contemplated herein.For example, FGFR1 is found to be mutated in glioblastoma, melanoma and lung. Furthermore FGFR1 was found to be amplified in breast, bladder and lung tumors.

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a subject and includes: (a) preventing a disease related in a subject which may be predisposed to the disease; (b) inhibiting the disease,

i.e. arresting its development; or (c) relieving the disease, i.e. causing regression of the disease. In particular, "treatment" comprises a "better response" as defined and explained herein above, such as a "complete response" or a more rapid tumor shrinkage as compared to "non-responders" (like individuals with only (increased) FGFR amplification or (increased) FGFR expression but no (increased) c-myc expression (or (increased) c-myc gene amplification).

An "individual", "patient"' or "subject" for the purposes of the present invention includes both humans and other animals, particularly mammals, and other organisms. Thus, the methods are applicable to both human therapy and veterinary applications. Preferably, the "individual", "patient" or "subject" is a mammal, and the most preferably the "individual", "patient" or "subject" is human.

The inhibitor of FGFR may be administered as a single anti-tumor agent or in form of a combination therapy, for example, conventional therapies like surgery, radiotherapy and/or one or more additional chemotherapeutic agents. Surgery may comprise the step of partial or complete tumour resection, prior to, during or after the administration of the herein provided therapy of the responding individuals with one or more inhibitors of FGFR. The FGFR inhibitors to be used herein may be administered in a neoadjuvant or adjuvant setting

The therapy used in said combination therapy may be chemotherapy or an anti-hormonal therapy. The chemotherapy may be anthracycline/taxane chemotherapy, therapy with an antimetabolite agents, therapy with an anti-hormonal compound, therapy with an anti-estrogen, therapy with a tyrosine kinase inhibitor, therapy with a raf inhibitor, therapy with a ras inhibitor, therapy with a dual tyrosine kinase inhibitor, therapy with taxol, therapy with taxane, therapy with doxorubicin, therapy with adjuvant (anti-) hormone drugs, and/or therapy with cisplatin and the like. The inhibitor may be administered by any one of a parenteral route, oral route, intravenous route, subcutaneous route, intranasal route or transdermal route.

Accordingly, the herein provided therapy of responding individuals with an inhibitor of FGFR as defined herein (or a pharmaceutically acceptable salt, solvate and/or hydrate thereof) may be administered to a patient in need of such a treatment during or after a surgical intervention/resection of the cancerous tissue. Therefore, the present invention is useful in neoadjuvant therapy, i.e. the treatment with the herein defined combination therapy given to a patient/patient group in need thereof prior to surgery. It is also useful in adjuvant therapy (i.e. after surgery). In other words, the inhibitor of FGFR may be administered in a neoadjuvant or adjuvant setting (in particular neoadjuvant or adjuvant treatment of cancer).

The pharmaceutical composition will be formulated and dosed in a fashion consistent with good medical practice, taking into account the clinical condition of the individual patient, the site of delivery of the pharmaceutical composition, the method of administration, the scheduling of administration, and other factors known to practitioners. The "effective amount" of the pharmaceutical composition for purposes herein is thus determined by such considerations.

The skilled person knows that the effective amount of pharmaceutical composition administered to an individual will, inter alia, depend on the nature of the compound.

For example, if said inhibitor of FGFR is a small molecule like BGJ398, the total (pharmaceutically) effective amount of the inhibitor in the pharmaceutical composition administered orally per dose will be in the range of about 50 mg inhibitor per day to 1000 mg inhibitor per day of patient, although, as noted above, this will be subject to therapeutic discretion. More preferably, this dose is at least 50 mg inhibitor per day, and most preferably for humans between about 50mg and 600 mg inhibitor per day. For example, an inhibitor like BGJ398 may be administered at a dose of 15 mg/kg body weigth per day. If given continuously, the inhibitor is typically administered at a dose rate of about 50 mg per day to about 600 mg per day. An intravenous bag solution may also be employed. The length of treatment needed to observe changes and the interval following treatment for responses to occur appears to vary depending on the desired effect. The particular amounts may be determined by conventional tests which are well known to the person skilled in the art. The length of treatment needed to observe changes and the interval following treatment for responses to occur appears to vary depending on the desired effect. The particular amounts may be determined by conventional tests which are well known to the person skilled in the art.

The administration of the herein provided compositions may, inter alia, comprise an administration twice daily, every day, every other day, every third day, every forth day, every fifth day, once a week, once every second week, once every third week, once every month, etc.

For example, if said compound is a (poly)peptide or protein the total pharmaceutically effective amount of pharmaceutical composition administered parenterally per dose will be in the range of about 1 $\mu$g protein /kg/day to 15 mg protein /kg/day of patient body weight, although, as noted above, this will be subject to therapeutic discretion. More preferably, this dose is at least 0.01 mg protein /kg/day, and most preferably for humans between about 0.01 and 1 mg protein /kg/day. If given continuously, the pharmaceutical composition is typically administered at a dose rate of about 1 $\mu$g/kg/hour to about 50 $\mu$g/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect. The particular amounts may be determined by conventional tests which are well known to the person skilled in the art.

Pharmaceutical compositions of the invention may be administered orally, rectally, parenterally, intracisternally, intravaginally, intraperitoneally, topically (as by powders, ointments, drops or transdermal patch), bucally, or as an oral or nasal spray.

Pharmaceutical compositions of the invention preferably comprise a pharmaceutically acceptable carrier. By "pharmaceutically acceptable carrier" is meant a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The pharmaceutical composition is also suitably administered by sustained release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP 133,988). Sustained release pharmaceutical composition also include liposomally entrapped compound. Liposomes containing the pharmaceutical composition are prepared by methods known per se: DE 3,218,121; Epstein et al., Proc. Natl. Acad. Sci. (USA) 82: 3688-3692 (1985); Hwang et al., Proc. Natal. Acad. Sci. (USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, the pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multidose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection.

Inhibitors for use in accordance with the present invention are described herein and refer generally to known and/or commercially available inhibitors. However, also the use of inhibitors yet to be generated or known compounds to be tested for their inhibiting activity is envisaged in context of the present invention.

Therefore, the present invention provides a method for assessing the activity of a candidate molecule suspected of being an inhibitor of FGFR comprising the steps of:

(a) contacting a cell, tissue or a non-human animal comprising a FGFR with said candidate molecule;
(b) detecting a decrease in FGFR activity; and
(c) selecting a candidate molecule that decreases FGFR activity;
wherein a decrease of the FGFR activity is in particular indicative for the capacity of the selected molecule to be useful in the treatment of cancer.

Further, the present invention provides a method for identifying a candidate molecule suspected of being an

inhibitor of FGFR comprising the steps of:

(a) contacting a cell, tissue or a non-human animal comprising a FGFR with said candidate molecule;
(b) detecting a decrease in FGFR activity; and
(c) selecting a candidate molecule that decreases FGFR activity;
wherein a decrease of the FGFR activity is in particular indicative for the capacity of the selected molecule to be useful in the treatment of cancer.

It is to be understood that the detected activity of a FGFR is compared to a standard or reference value of the activity of a FGFR, respectively. The standard/reference value may be detected in a cell, tissue, or non-human animal as defined herein, which has not been contacted with a potential inhibitor or prior to the above contacting step. The decrease in the activity of the FGFR upon contacting with (a) candidate molecule(s) may also be compared to the decrease in activity of FGFR induced by (a) routinely used reference compound(s). A skilled person is easily in the position to determine/assess whether the activity and/or expression of a FGFR is (preferably statistically significant) decreased.

In accordance with this invention, in particular the screening or identifying methods described herein, a cell, tissue or non-human animal to be contacted with a candidate molecule comprises FGFR as defined herein. For example said cell, tissue or non-human animal may express a FGFR gene, in particular also (an) additional (copy) copies of such a gene, (a) mutated gene(s), a recombinant gene construct and the like. As explained herein below, the capability of a candidate molecule to inhibit/antagonize FGFR may, accordingly, be detected by measuring the expression level of such gene products of FGFR gene(s) or of corresponding gene constructs (e.g. mRNA or protein), wherein a low expression level (compared to a standard or reference value) is indicative for the capability of the candidate molecule to act as inhibitor/antagonist.

The term "comprising FGFR "may, for example, relate to to the EGFR gene(s) or proteins known in the art and described herein, but also to a reporter construct which comprises the FGFR (or a functional fragment thereof) and a "reporter". Exemplary reporters (reporter gene products), which can be used in the screening methods of the invention are luciferase, (green/red) fluorescent protein and variants thereof, EGFP (enhanced green fluorescent protein), RFP (red fluorescent protein, like DsRed or DsRed2), CFP (cyan fluorescent protein), BFP (blue green fluorescent protein), YFP (yellow fluorescent protein), β-galactosidase or chloramphenicol acetyltransferase. The skilled person is readily in the position to generate and use also other reporters/reporter constructs, which can be employed in accordance with the present invention. The use of fusion proteins containing a FGFR (or a functional fragment thereof) and a reporter gene product is also envisaged in the methods of the present invention.

Inhibitors of FGFR may interfere with the transcription of the FGFR reporter construct, in particular fusion proteins. For example, the inhibitor may bind to the promoter region of the FGFR gene or of the sequence encoding the fusion protein, thus preventing initiation of transcription or stopping the already initiated transcription process. The inhibitor may also bind to/interfere with components of the transcription machinery, thereby effectively inhibiting initiation of transcription or continuation of transcription. Such an interference with the transcription of the FGFR gene, or FGFR constructs or FGFR fusion proteins by a candidate molecule will be reflected in a decreased transcription activity and hence, a reduced transcript level (e.g. unspliced/partially spliced/spliced mRNA). It is also envisaged herein that a reporter construct to be used herein comprises the promoter of a FGFR gene linked to a reporter as described herein. Thus, activity of the FGFR may be reflected in an activation of its promoter and, hence, in turn reflected in the change/ decrease of the reporter signal associated with the reporter.

Due to the reduced transcript level also the level of the translated gene product (i.e. the protein level) will be decreased. The level of the above described fusion proteins preferably correlates with the signal strength of a detectable signal associated with the reporter gene product. Exemplary FGFR fusion proteins are proteins comprising FGFR (or a functional fragment thereof) and a reporter as described above (e.g. luciferase, (green/red) fluorescent protein and variants thereof, EGFP (enhanced green fluorescent protein), and the like).

Accordingly, a decrease in FGFR (promoter) activity (which may, for example, be reflected in a decrease in the (promoter) reporter signal) upon contacting the cell/tissue/non-human animal with a candidate molecule will indicate that the candidate molecule is indeed a FGFR inhibitor/antagonist and, thus, suitable in the treatment of cancer. The candidate molecules which decrease FGFR (promoter) activity as defined herein above are selected out of the candidate molecules tested, wherein those molecules are preferably selected which strongly decrease FGFR (promoter) activity (reflected, for example, in a pronounced decrease in the reporter signal). It is assumed that the FGFR antagonizing/ inhibiting activity of a candidate molecule is the stronger the more the reporter signal is decreased.

It is envisaged in the context of the present invention (in particular the screening/identifying methods disclosed herein) that also cellular extracts can be contacted (e.g. cellular extracts comprising FGFR as described and defined herein). For example, these cellular extracts may be obtained from the (transgenic/genetically engineered) cell(s), tissue (s) and/or non-human animal(s) to be used herein, in particular to be contacted with the candidate molecule. The use

of such cellular extracts is particular advantageous since it allows the assessment of the activity of a candidate molecule in vitro. The assessing/screening methods taking advantage of such (cellular) extracts can, for example, be used in prescreening candidate molecules, wherein the molecules selected in such a prescreen are then subject to subsequent screens, for example in the cell-based methods disclosed herein, in particular in methods wherein a (transgenic) cell(s), tissue(s) and/or non-human animal(s) are contacted with a candidate molecule. In this context, it is accordingly preferred that the candidate molecule has been selected in the in vitro pre-screening method, described herein above and below.

It is to be understood that in a high throughput screening routinely, many (often thousands of candidate molecules) are screened simultaneously, Accordingly, in a (first) screen candidate molecules are selected, which decrease FGFR activity.

Step (a) of the screening methods of the present invention, i.e. the "contacting step" may also be accomplished by adding a (biological) sample or composition containing said candidate molecule or a plurality of candidate molecules (i.e. various different candidate molecules) to the sample to be analyzed (e.g. (a) cell(s)/tissue(s)/non-human animal comprising FGFR or a functional fragment thereof).

Generally, the candidate molecule(s) or a composition comprising/containing the candidate molecule(s) may for example be added to a (transfected) cell, tissue or non-human animal comprising FGFR. As defined and disclosed herein, the term "comprising FGFR"refers not only to the FGFR gene(s) or proteins known in the art and described herein, but also to reporter constructs comprising a reporter and FGFR. Exemplary reporters (preferably associated with the reporter signals disclosed herein) are luciferase and fluorescent proteins, like GFP, RFP and the like. Also reporter constructs comprising a promoter and/or enhancer region of FGFR and a reporter as defined herein can be used in the screening/identifying methods. Accordingly, the cell(s), tissue(s) and/or non-human animals used in the context of the present invention, in particular in context of the screening/identifying methods can be stably or transiently transfected with the reporter constructs disclosed herein.

In particular the identification/assessment of candidate molecules which are capable of inhibiting/antagonizing FGFR may be, inter alia, performed by transfecting an appropriate host with a nucleic acid molecule encoding FGFR (or a functional fragment thereof) and contacting said host with (a) candidate molecule(s). The host (cell, tissue, non-human animal) can also be transfected with the above described reporter constructs, e.g. luciferase reporter constructs, such as, but not limited to, reporter constructs comprising a luciferase reporter. The host may comprise CHO-cell, HEK 293, HeLa, Cos 7, PC12 or NIH3T3 cell, frog oocytes or primary cells like primary cardiomyocytes, fibroblasts, muscle, endothelial or embryonic stem cells. Alternatively, it is also possible to use cell lines stably transfected with a nucleic acid molecule encoding FGFR or a functional fragment thereof. The explanations given herein above in respect of "cells" also apply to tissues/non-human animals comprising or derived from these cells. A sample to be analyzed may also be a biological, medical or pathological sample, for example fluids that comprise cells, tissues or cell cultures. Such a fluid may be a body fluid or also excrements and may also be a culture sample. The body fluids may comprise but are not limited to blood, serum, plasma, urine, saliva, synovial fluid, spinal fluid, cerebrospinal fluid, tears, stool and the like.

The (biological) sample or composition, comprising a plurality of candidate molecules are usually subject to a first screen. The samples/compositions tested positive in the first screen are often subject to subsequent screens in order to verify the previous findings and to select the most potent inhibitors/antagonists of the FGFR. Upon multiple screening and selection rounds those candidate molecules will be selected which show a pronounced capacity to inhibit/antagonize FGFR as defined and disclosed herein. For example, batches (i.e. compositions/samples) containing many candidate molecules will be rescreened and batches with no or insufficient inhibitory activity of candidate molecules be discarded without re-testing.

For example, if a (biological) sample or composition with many different candidate molecules is tested and one (biological) sample or composition is tested positive, then it is either possible in a second screening to screen, preferably after purification, the individual molecule(s) of the (biological) sample or composition. It may also be possible to screen subgroups of the (biological) sample or composition of the first screen in (a) subsequent screen(s). The screening of compositions with subgroups of those candidate molecules tested in previous screening rounds will thus narrow in on (an) potential potent FGFR inhibitor(s). This may facilitate and accelerate the screening process in particular when a large number of molecules is screened. Accordingly, the cycle number of screening rounds is reduced compared to testing each and every individual candidate molecule in (a) first (and subsequent) screen(s) (which is, of course, also possible). Thus, depending on the complexity or the number of the candidate molecules, the steps of the screening method described herein can be performed several times until the (biological) sample or composition to be screened comprises a limited number, preferably only one substance.

The term "decrease in FGFR activity" in step (b) of the screening method means that the "activity of the FGFR" is reduced upon contacting the cell, tissue, or non-human animal comprising a FGFR with the candidate molecule, preferably in comparison to a (control) standard or reference value, as defined herein.

Particularly preferred are optical measurement techniques that allow a resolution of fluorescence on the level of single cells or single cells of a tissue, preferably at the subcellular level. They may involve fluorescence, for example confocal microscopy, digital image recording, preferably a CCD camera and suitable picture analysis software. Preferably,

step (b) is carried out after the measurement of a standard response by performing a control experiment. For example, the activity of a FGFR is measured in a cell, tissue or a non-human animal comprising a FGFR without contacting a candidate molecule in a first screen. In a second screen, after contacting the candidate molecule, the activity of FGFR is measured. A difference in the activities will indicate whether the tested candidate molecule is indeed an antagonist of a FGFR.

The activity of a FGFR can be quantified by measuring, for example, the level of gene products (e.g. mRNA and/or protein of the FGFR and said component, respectively) by any of the herein described methods, activities, or other cellular functions, like inter alia, the involvement in signalling pathways or changes in intracellular localization.

As mentioned, a decreased "FGFR activity" and, accordingly, a decreased concentration/amount of FGFR proteins in a sample may be reflected in a decreased expression of the corresponding gene(s) encoding the FGFR protein(s). Therefore, a quantitative assessment of the gene product (e.g. protein or spliced, unspliced or partially spliced mRNA) can be performed in order to evaluate decreased expression of the corresponding gene(s) encoding the FGFR protein(s). Also here, a person skilled in the art is aware of standard methods to be used in this context or may deduce these methods from standard textbooks (e.g. Sambrook, 2001, loc. cit.). For example, quantitative data on the respective concentration/amounts of mRNA from the FGFR can be obtained by Northern Blot, Real Time PCR and the like. Preferably, the concentration/amount of the gene product (e.g. the herein above described FGFR mRNA or FGFR protein) may be decreased by at least about 10 %, 20 %, 30 %, 40 %, preferably by at least 50 %, 60 %, 70 %, 80 %, 90 %, 91 %, 92 %, 93 %, 94 %, 95 %, 96 %, 97 %, 98 %, 99 % or even 100 % compared to a control sample. A decrease by 100 % means that no gene product is detectable by herein described routine methods. It is preferred herein that FGFR proteins are (biologically) active or functional. Methods for determining the activity of a FGFR are described herein above. Since the FGFR proteins are preferably (biologically) active/functional (wherein it is preferred that at least 70 %, 75 %, preferably at least 80%, 85 %, 90 %, 95 %, 96, %, 97%, 98 % and most preferably, at least 99 % of FGFR proteins of a sample a (biologically) active/functional), a decreased concentration/amount of FGFR proteins in a sample reflects a decreased (biological) activity of the FGFR protein.

As mentioned, a person skilled in the art is aware of standard methods to be used for determining or quantitating expression of a nucleic acid molecule encoding, for example, the FGFR (or fragments thereof) as defined herein. For example, the expression can be determined on the protein level by taking advantage of immunoagglutination, immunoprecipitation (e.g. immunodiffusion, immunelectrophoresis, immune fixation), western blotting techniques (e.g. (in situ) immuno histochemistry, (in situ) immuno cytochemistry, affinity chromatography, enzyme immunoassays), and the like. Amounts of purified polypeptide in solution can be determined by physical methods, e.g. photometry. Methods of quantifying a particular polypeptide in a mixture rely on specific binding, e.g. of antibodies. Specific detection and quantitation methods exploiting the specificity of antibodies comprise for example immunohistochemistry (in situ). For example, concentration/amount of FGFR proteins in a cell, tissue or a non-human animal can be determined by enzyme linked-immunosorbent assay (ELISA). Alternatively, Western Blot analysis or immunohistochemical staining can be performed. Western blotting combines separation of a mixture of proteins by electrophoresis and specific detection with antibodies. Electrophoresis may be multi-dimensional such as 2D electrophoresis. Usually, polypeptides are separated in 2D electrophoresis by their apparent molecular weight along one dimension and by their isoelectric point along the other direction.

Expression can also be determined on the nucleic acid level (e.g. if the gene product/product of the coding nucleic acid sequence is an unspliced/partially spliced/spliced mRNA) by taking advantage of Northern blotting techniques or PCR techniques, like in-situ PCR or Real time PCR. Quantitative determination of mRNA can be performed by taking advantage of northern blotting techniques, hybridization on microarrays or DNA chips equipped with one or more probes or probe sets specific for mRNA transcripts or PCR techniques referred to above, like, for example, quantitative PCR techniques, such as Real time PCR. These and other suitable methods for detection and/or determination of the concentration/amount of (specific) mRNA or protein(s)/polypeptide(s) are well known in the art and are, for example, described in Sambrook (2001), loc. cit.).

A skilled person is capable of determining the amount of mRNA or polypeptides/proteins, in particular the gene products described herein above, by taking advantage of a correlation, preferably a linear correlation, between the intensity of a detection signal and the amount of, for example, the mRNA or polypeptides/proteins to be determined. Accordingly, the activity of a FGFR (or a functional fragment thereof) may be quantified based on the mRNA or protein level of the FGFR or a functional fragment thereof and vice versa.

Genetic readout systems are also envisaged. Analogously, the activity of a FGFR or of a functional fragment thereof may be quantified by any molecular biological method as described herein. A skilled person is also aware of standard methods to be used in determining the amount/concentration of FGFR expression products (in particular the protein and the nucleic acid level of FGFR) in a sample or may deduce corresponding methods from standard textbooks (e.g. Sambrook, 2001).

In samples obtained from (a) cell(s), tissue or a cell culture(s) transfected with appropriate constructs or obtained from transgenic animals or cell cultures derived from a non-human animal(s), the concentration/amount of FGFR protein can be determined by bioassays, if, for example, a FGFR -inducible promoter is fused to a reporter gene. Apparently,

decreased expression of the reporter gene/activity of the reporter gene product will reflect a decreased FGFR activity, in particular a decreased concentration/amount of FGFR protein. Alternatively, the effect of the FGFR protein on the expression of (a) reporter gene(s) may be evaluated by determining the amount/concentration of the gene product of the reporter gene(s) (e.g. protein or spliced, unspliced or partially spliced mRNA). Further methods to be used in the assessment of mRNA expression of a reporter gene are within the scope of a skilled person and also described herein below.

Also in this context, reporter constructs comprising a promoter and/or enhancer region of FGFR and a reporter as defined herein can be used in the screening/identifying methods. Exemplary reporters (preferably associated with the reporter signals disclosed herein) are luciferase and fluorescent proteins, like GFP, RFP and the like. Exemplary reporters (preferably associated with the reporter signals disclosed herein) are luciferase and fluorescent proteins, like GFP, RFP and the like. These and other reporters/reporter constructs/reporter signals are also described herein above and below.

The difference, as disclosed herein is statistically significant and a candidate molecule(s) is (are) selected, if the FGFR activity (or of a corresponding reporter signal) is strongly decreased, preferably is very low or non-dectable. For example, the FGFR activity (or of a corresponding reporter signal) may be decreased by at least 50%, 60%, 70%, 80%, more preferably by at least 90% compared to the (control) standard value. In a cell based method the cells can be transfected with one or more constructs encoding a FGFR or a functional fragment thereof as described above and optionally a reporter under the transcriptional control of the FGFR promoter or a functional fragment thereof as described above.

Preferably, the selected compound has a high FGFR inhibiting/antagonizing activity. This can be reflected in the capacity of the FGFR antagonist/inhibitor to potently decrease the activity of FGFR.

The above detected difference between the activity of a FGFR or the activity of a functional fragment of the FGFR in a cell, tissue or a non-human animal contacted with said candidate molecule and the activity in the (control) standard value (measured e.g. in the absence of said candidate molecule) may be reflected by the presence, the absence, the increase or the decrease of a specific signal in the readout system, as in the herein described fluorescence based system.

Preferably, candidate agents to be tested encompass numerous chemical classes, though typically they are organic compounds, preferably small (organic) molecules as defined herein above. Candidate agents may also comprise functional groups necessary for structural interaction with proteins, particularly hydrogen bonding, and typically include at least an amine, carbonyl, hydroxyl or carboxyl group, preferably at least two of the functional chemical groups. The candidate agents often comprise carbocyclic or heterocyclic structures and/or aromatic or polyaromatic structures substituted with one or more of the above functional groups.

Exemplary classes of candidate agents may include heterocycles, peptides, saccharides, steroids, and the like. The compounds may be modified to enhance efficacy, stability, pharmaceutical compatibility, and the like. Structural identification of an agent may be used to identify, generate, or screen additional agents. For example, where peptide agents are identified, they may be modified in a variety of ways to enhance their stability, such as using an unnatural amino acid, such as a D-amino acid, particularly D-alanine, by functionalizing the amino or carboxylic terminus, e.g. for the amino group, acylation or alkylation, and for the carboxyl group, esterification or amidification, or the like. Other methods of stabilization may include encapsulation, for example, in liposomes, etc.

As mentioned above, candidate agents are also found among other biomolecules including amino acids, fatty acids, purines, pyrimidines, nucleic acids and derivatives, structural analogs or combinations thereof. Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs.

The reporter constructs for detecting FGFR inhibition as described herein above may be comprised in a cell, tissue or a non-human animal. Methods for transfecting cells or tissues are known in the art. Accordingly, calcium phosphate treatment or electroporation may be used for transfecting cells or tissues to express said reporter constructs (see Sambrook (1989), loc. cit.). Furthermore, nucleic acid molecules expressing said reporter constructs can be reconstituted into liposomes for delivery to target cells. As a further alternative, cells may be transduced to express specific reporter construct using genetically engineered viral vectors.

The non-human animal comprising said reporter construct for detecting FGFR inhibition may be a transgenic non-human animal. The non-human organism to be used in the described screening assays is preferably selected from the group consisting of C. elegans, yeast, drosophila, zebrafish, guinea pig, rat and mouse. The generation of such a

transgenic animal is within the skill of a skilled artisan. Corresponding techniques are, inter alia, described in "Current Protocols in Neuroscience" (2001), John Wiley&Sons, Chapter 3.16. Accordingly, the invention also relates to a method for the generation of a non-human transgenic animal comprising the step of introducing a reporter construct for detecting FGFR inhibition as disclosed herein into an ES-cell or a germ cell. The non-human transgenic animal provided and described herein is particular useful in screening methods and pharmacological tests described herein above. In particular the non-human transgenic animal described herein may be employed in drug screening assays as well as in scientific and medical studies wherein antagonists/inhibitors of FGFR for the treatment of a cancer are tracked, selected and/or isolated.

The transgenic/genetically engineered cell(s), tissue(s), and/or non-human animals to be used in context of the present invention, in particular, the screening/identifying methods, preferably comprise the herein described and defined reporter constructs. Also in this context, reporter constructs may comprise a promoter and/or enhancer region of a FGFR and a reporter as defined herein. Exemplary reporters (preferably associated with the reporter signals disclosed herein) are luciferase and fluorescent proteins, like GFP, RFP and the like. Exemplary reporters (preferably associated with the reporter signals disclosed herein) are luciferase and fluorescent proteins, like GFP, RFP and the like. These and other reporters/reporter constructs/reporter signals are also described herein above and below.

The present invention relates to the use of a cell, tissue or a non-human animal for screening and/or validation of a compound suspected of being an inhibitor of FGFR. Correspondingly, the present invention relates to the use of a cell, tissue or a non-human animal for screening and/or validation of a compound suspected of being an agonist of a FGFR. Accordingly, herein envisaged is the use of a cell, tissue or a non-human animal for screening and/or validation of a compound suspected of being an antagonist of a FGFR.

The term "cell" as used in this context may also comprise a plurality of cell as well as cells comprised in a tissue. For example, a high and stable expression of FGFR may facilitate the detection of a decrease in FGFR activity. Since wild-type cells have sometimes a low or unstable FGFR expression, the use of (a) transgenic cell(s), tissue(s), non-human animal is particularly envisaged, if these cells have a high FGFR expression (reflected in a high protein or mRNA level). (Transgenic) cells(s), tissue(s) and non-human animals to be used in accordance with the present invention are also described herein above.

The used non-human animal or cell may be transgenic or non transgenic. In this context the term "transgenic" particularly means that at least one of the FGFR genes as described herein is overexpressed; thus the FGFR activity in the non-human transgenic animal or a transgenic animal cell is enhanced. Generally, it is preferred herein that FGFR is highly expressed in (a) cell(s), tissue(s), non-human animal to be used in the screening methods as described above.

The term "transgenic non-human-animal", "transgenic cell" or "transgenic tissue" as used herein refers to an non-human animal, tissue or cell, not being a human that comprises different genetic material of a corresponding wild-type animal, tissue or cell. The term "genetic material" in this context may be any kind of a nucleic acid molecule, or analogues thereof, for example a nucleic acid molecule, or analogues thereof as defined herein. The term "different" means that additional or fewer genetic material in comparison to the genome of the wild type animal or animal cell. An overview of different expression systems to be used for generating transgenic cell/animal refers for example to Methods in Enzymology 153 (1987), 385-516, in Bitter et al (Methods in Enzymology 153 (1987), 516-544) and in Sawers et al. (Applied Microbiology and Biotechnology 46 (1996), 1-9), Billman-Jacobe (Current Opinion in Biotechnology 7 (1996), 500-4), Hockney (Trends in Biotechnology 12 (1994), 456-463), Griffiths et al., (Methods in Molecular Biology 75 (1997), 427-440).

The (transgenic) non-human animal or (transgenic) cell may be a mammal or may be derived from a mammal. Non-limiting examples of the (transgenic) non-human animal or derived (transgenic) cell are selected from the group consisting of a mouse, a rat, a rabbit, a guinea pig and Drosophila. Generally, the (transgenic) cell may be a prokaryotic or eukaryotic cell. For example, the (transgenic) cell in accordance with the present invention may be but is not limited to bacterial, yeast, fungus, plant or animal cell. In general, the transformation or genetically engineering of a cell with a nucleic acid construct or a vector can be carried out by standard methods, as for instance described in Sambrook and Russell (2001), Molecular Cloning: A Laboratory Manual, CSH Press, Cold Spring Harbor, NY, USA; Methods in Yeast Genetics, A Laboratory Course Manual, Cold Spring Harbor Laboratory Press, 1990.

The term "Fibroblast growth factor receptor" (short "FGFR") as used herein refers to a member of the type 4 family of receptor tyrosine kinases. Presently, four members of said family are known. These members are termed FGFR1-4 (i.e. FGFR1, FGFR2, FGFR3, FGFR4). Due to alternate splicing of gene products of the genes of these four family members, more than 48 different isoforms of FGFR are produced. These isoforms vary in kinase domains and ligand-binding properties. However, they share the common extracellular region composed of two to three immunoglobulin (Ig)-like domains. Thus, they belong to the immunoglobulin superfamily.

Exemplary nucleic acid sequences and amino acid sequences of members of the type 4 family of receptor tyrosine kinases (and of the corresponding isoforms), such as FGFR1, are provided herein and shown in SEQ ID NO: s 1 to 4. SEQ ID NO: 1 and 3 shows the nucleic acid sequence encoding the human FGFR1 gene. SEQ ID NO: 7 to 12 show the nucleic acid sequence encoding the human FGFR2 gene, FGFR3 gene and FGFR4 gene, respectively.

SEQ ID NO: 1 and 3 show the nucleic acid sequence encoding isoform FGFR1α and FGFR1β of the human FGFR1, respectively. SEQ ID NO: 2 and 4 show the amino acid sequence of isoform FGFR1α and FGFR1β of the human FGFR1, respectively.

SEQ ID NO: 7 to 12 show the nucleic acid sequence encoding isoform FGFR2 7, FGFR3 9 and FGFR4 11 of the human FGFR2, FGFR3, and FGFR4, respectively. SEQ ID NO: 7 to 12 show the amino acid sequence of isoform FGFR2 8, FGFR3 10 and FGFR4 12 of the human FGFR2, FGFR3, and FGFR4, respectively.

Thus, an FGFR may be selected from the group consisting of

(a) a polypeptide comprising an amino acid encoded by a nucleic acid molecule having the nucleic acid sequence as depicted in SEQ ID NOs: 1, 3, 7, 9 or 11;
(b) a polypeptide having an amino acid sequence as depicted in SEQ ID NOs2, 4, 8, 10 or 12;
(c) a polypeptide encoded by a nucleic acid molecule encoding a peptide having an amino acid sequence as depicted in SEQ ID NOs2, 4, 8, 10 or 12
(d) a polypeptide comprising an amino acid encoded by a nucleic acid molecule hybridizing under stringent conditions to the complementary strand of nucleic acid molecules as defined in (a) or (c) and encoding a functional FGFR, or a fragment thereof;
(e) a polypeptide having at least 60 % homology to the polypeptide of any one of (a) to (d), whereby said polypeptide is a functional FGFR, or a fragment thereof; and
(f) a polypeptide comprising an amino acid encoded by a nucleic acid molecule being degenerate as a result of the genetic code to the nucleotide sequence of a nucleic acid molecule as defined in (a), (c) and (d).

For example, FGFR1 (or an isoform thereof) may be selected from the group consisting of

(a) a polypeptide comprising an amino acid encoded by a nucleic acid molecule having the nucleic acid sequence as depicted in SEQ ID NOs: 1 or 3;
(b) a polypeptide having an amino acid sequence as depicted in SEQ ID NOs: 2 or 4;
(c) a polypeptide encoded by a nucleic acid molecule encoding a peptide having an amino acid sequence as depicted in SEQ ID NOs: 2 or 4;
(d) a polypeptide comprising an amino acid encoded by a nucleic acid molecule hybridizing under stringent conditions to the complementary strand of nucleic acid molecules as defined in (a) or (c) and encoding a functional FGFR, or a fragment thereof;
(e) a polypeptide having at least 60 % homology to the polypeptide of any one of (a) to (d), whereby said polypeptide is a functional FGFR, or a fragment thereof; and
(f) a polypeptide comprising an amino acid encoded by a nucleic acid molecule being degenerate as a result of the genetic code to the nucleotide sequence of a nucleic acid molecule as defined in (a), (c) and (d).

The term "functional FGFR " used in context of the present invention refers to a polypeptide having at least 60 % homology to a polypeptide as defined in section (a) to (d) of the above-described specific aspect of the present invention which has essentially the same biological activity as a polypeptide having 100 % homology to a polypeptide as indicated in section (a) to (d), i.e. a polypeptide being essentially identical to a polypeptide having an amino acid sequence as depicted in SEQ ID NO: 2, 4, 8, 10 or 12. Methods for determining the activity of (a) polypeptide(s) are well known in the art and may, for example, be deduced from standard text books, such as Bioanalytik (Lottspeich/Zorbas (eds.), 1998, Spektrum Akademischer Verlag). Methods for determining the activity of a FGFR are also described herein below.

It is of note that a (functional) FGFR or a functional fragment thereof as described and defined herein may further comprise a heterologous polypeptide, for example, (an) amino acid sequence(s) for identification and/or purification of the recombinant protein (e.g. amino acid sequence from C-MYC, GST protein, FLAG peptide, HIS peptide and the like), an amino acid sequence used as reporter (e.g. green fluorescent protein, yellow fluorescent protein, red fluorescent protein, luciferase, and the like), or antibodies/antibody fragments (like scFV). A person skilled in the art knows that for determination of homology as described herein only a part of a polypeptide is to be used, whereby said part is FGFR (or a functional fragment thereof). Also further compounds (e.g. toxins or antibodies or fragments thereof) may be attached to FGFR (or a functional fragment thereof) by standard techniques. These compounds may, in particular, be useful in a medical setting as described herein, wherein the responsiveness to inhibitors of FGFR is tested and responsive individuals/patients are treated with FGFR inhibitors (or a functional fragment thereof). A skilled person is aware of compounds to be used/attached in this context.

Methods and assays for determining the activity of "FGFR", like FGFR1, are described herein. These methods also allow determining whether a polypeptide can be considered as a "functional FGFR". The activity exhibited by the following exemplary polypeptides can be considered as "reference activity" of a functional FGFR: a polypeptide having

an amino acid sequence as depicted in SEQ ID NO: 2, 4, 8, 10 or 12 ("human FGFR). For example, the activity exhibited by the following exemplary polypeptides can be considered as "reference activity" of a functional FGFR1: a polypeptide having an amino acid sequence as depicted in SEQ ID NO: 2 ... and 4 ("human FGFR1", isoform FGFR1α and FGFR1β, respectively).

The meanings of the term "polypeptide" and "nucleic acid sequence(s)/molecule(s)" are well known in the art and are used accordingly in context of the present invention. For example, "nucleic acid sequence(s)/molecule(s)" as used herein refer(s) to all forms of naturally occurring or recombinantly generated types of nucleic acids and/or nucleic acid sequences/molecules as well as to chemically synthesized nucleic acid sequences/molecules. This term also encompasses nucleic acid analogues and nucleic acid derivatives such as e. g. locked DNA, PNA, oligonucleotide thiophosphates and substituted ribo-oligonucleotides. Furthermore, the term "nucleic acid sequence(s)/molecules(s)" also refers to any molecule that comprises nucleotides or nucleotide analogues. Preferably, the term "nucleic acid sequence(s)/molecule(s)" refers to deoxyribonucleic acid (DNA) and ribonucleic acid (RNA). The "nucleic acid sequence (s)/molecule(s)" may be made by synthetic chemical methodology known to one of ordinary skill in the art, or by the use of recombinant technology, or may be isolated from natural sources, or by a combination thereof. The DNA and RNA may optionally comprise unnatural nucleotides and may be single or double stranded. "Nucleic acid sequence(s)/molecule (s)" also refers to sense and anti-sense DNA and RNA, that is, a nucleotide sequence which is complementary to a specific sequence of nucleotides in DNA and/or RNA. Furthermore, the term "nucleic acid sequence(s)/molecule(s)" may refer to DNA or RNA or hybrids thereof or any modification thereof that is known in the state of the art (see, e.g., US 5525711, US 4711955, US 5792608 or EP 302175 for examples of modifications). The nucleic acid molecule(s) may be single- or double-stranded, linear or circular, natural or synthetic, and without any size limitation. For instance, the nucleic acid molecule(s) may be genomic DNA, cDNA, mRNA, antisense RNA, ribozymal or a DNA encoding such RNAs or chimeroplasts (Colestrauss, Science (1996), 1386-1389. Said nucleic acid molecule(s) may be in the form of a plasmid or of viral DNA or RNA. "Nucleic acid sequence(s)/molecule(s)" may also refer to (an) oligonucleotide(s), wherein any of the state of the art modifications such as phosphothioates or peptide nucleic acids (PNA) are included.

The nucleic acid sequence of FGFR of other species than the herein provided human sequence can be identified by the skilled person using methods known in the art, e.g. by using hybridization assays or by using alignments, either manually or by using computer programs such as those mentioned herein below in connection with the definition of the term "hybridization" and degrees of homology.

The nucleic acid sequence encoding for orthologs of human FGFRs may be at least 40% homologous to the nucleic acid sequence as shown in SEQ ID NO. 1, 3, 7, 9 or 11. More preferably, the nucleic acid sequence encoding for orthologs of human FGFRs is at least 45%, 50%, 55%, 60%, 65%, 70%, 75%, 80%, 85%, 90%, 91%, 92%, 93%, 94%, 95%, 96%, 97% or 98% homologous to the nucleic acid sequence as shown in SEQ ID NOs. 1, 3, 7, 9 or 11, wherein the higher values are preferred. Most preferably, the nucleic acid sequence encoding for orthologues of FGFR is at least 99% homologous to the nucleic acid sequence as shown in SEQ ID NO. 1, 3, 7, 9 or 11. The term "orthologous protein" or "orthologous gene" as used herein refers to proteins and genes, respectively, in different species that are similar to each other because they originated from a common ancestor.

Hybridization assays for the characterization of orthologs of known nucleic acid sequences are well known in the art; see e.g. Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989). The term "hybridization" or "hybridizes" as used herein may relate to hybridizations under stringent or non-stringent conditions. If not further specified, the conditions are preferably non-stringent. Said hybridization conditions may be established according to conventional protocols described, e.g., in Sambrook (2001) loc. cit.; Ausubel (1989) loc. cit., or Higgins and Hames (Eds.) "Nucleic acid hybridization, a practical approach" IRL Press Oxford, Washington DC, (1985). The setting of conditions is well within the skill of the artisan and can be determined according to protocols described in the art. Thus, the detection of only specifically hybridizing sequences will usually require stringent hybridization and washing conditions such as, for example, the highly stringent hybridization conditions of 0.1 x SSC, 0.1% SDS at 65°C or 2 x SSC, 60°C, 0.1 % SDS. Low stringent hybridization conditions for the detection of homologous or not exactly complementary sequences may, for example, be set at 6 x SSC, 1% SDS at 65°C. As is well known, the length of the probe and the composition of the nucleic acid to be determined constitute further parameters of the hybridization conditions.

In accordance with the present invention, the terms "homology" or "percent homology" or "identical" or "percent identity" or "percentage identity" or "sequence identity" in the context of two or more nucleic acid sequences refers to two or more sequences or subsequences that are the same, or that have a specified percentage of nucleotides that are the same (preferably at least 40% identity, more preferably at least 45%, 50%, 55%, 60%, 65%, 70%, 75%, 80%, 85%, 90%, 91%, 92%, 93%, 94%, 95%, 96%, 97% or 98% identity, most preferably at least 99% identity), when compared and aligned for maximum correspondence over a window of comparison (preferably over the full length), or over a designated region as measured using a sequence comparison algorithm as known in the art, or by manual alignment and visual inspection. Sequences having, for example, 75% to 90% or greater sequence identity may be considered to

be substantially identical. Such a definition also applies to the complement of a test sequence. Preferably the described identity exists over a region that is at least about 15 to 25 nucleotides in length, more preferably, over a region that is at least about 50 to 100 nucleotides in length and most preferably, over a region that is at least about 800 to 1200 nucleotides in length, preferably over the full length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Although the FASTDB algorithm typically does not consider internal non-matching deletions or additions in sequences, i.e., gaps, in its calculation, this can be corrected manually to avoid an overestimation of the % identity. CLUSTALW, however, does take sequence gaps into account in its identity calculations. Also available to those having skill in this art are the BLAST and BLAST 2.0 algorithms (Altschul, (1997) Nucl. Acids Res. 25:3389-3402; Altschul (1993) J. Mol. Evol. 36:290-300; Altschul (1990) J. Mol. Biol. 215:403-410). The BLASTN program for nucleic acid sequences uses as defaults a word length (W) of 11, an expectation (E) of 10, M=5, N=4, and a comparison of both strands. The BLOSUM62 scoring matrix (Henikoff (1989) PNAS 89:10915) uses alignments (B) of 50, expectation (E) of 10, M=5, N=4, and a comparison of both strands.

In order to determine whether an nucleotide residue in a nucleic acid sequence corresponds to a certain position in the nucleotide sequence of e.g. SEQ ID NOs: 1, 3, 7, 9 or 11, respectively, the skilled person can use means and methods well-known in the art, e.g., alignments, either manually or by using computer programs such as those mentioned herein. For example, BLAST 2.0, which stands for Basic Local Alignment Search Tool BLAST (Altschul (1997), loc. cit.; Altschul (1993), loc. cit.; Altschul (1990), loc. cit.), can be used to search for local sequence alignments. BLAST, as discussed above, produces alignments of nucleotide sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cut-off score set by the user. The BLAST approach is to look for HSPs between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output.

Analogous computer techniques using BLAST (Altschul (1997), loc. cit.; Altschul (1993), loc. cit.; Altschul (1990), loc. cit.) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score, which is defined as:

$$\frac{\%\ \text{sequence identity} \times \%\ \text{maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those, which show product scores between 15 and 40, although lower scores may identify related molecules. Another example for a program capable of generating sequence alignments is the CLUSTALW computer program (Thompson (1994) Nucl. Acids Res. 2:4673-4680) or FASTDB (Brutlag (1990) Comp. App. Biosci. 6:237-245), as known in the art.

The explanations and definitions given herein above in respect of "homology of nucleic acid sequences" apply, mutatis mutandis, to "amino acid sequences" of FGFRs as depicted in SEQ ID NO: 2, 4, 8, 10 or 12 (human FGFRs), like SEQ ID NO: 2 or 4 (human FGFR1, isoform FGFR1$\alpha$ and FGFR1$\beta$, respectively) as explained below.

The polypeptide to be used in accordance with the present invention may have at least 40 % identity/similarity to the FGFRs having the amino acid sequence as, for example, depicted in SEQ ID NO: 2, 4, 8, 10 or 12, respectively (e.g. SEQ ID NO: 2 or 4 (human FGFR1, isoform FGFR1$\alpha$ and FGFR1$\beta$, respectively). More preferably, the polypeptide has at least 45%, 50%, 55%, 60%, 65%, 70%, 75%, 80%, 85%, 90%, 91%, 92%, 93%, 94%, 95%, 96%, 97% or 98% identity/similarity to FGFR depicted in SEQ ID NO: 2, 4, 8, 10 or 12, respectively (e.g. SEQ ID NO: 2 or 4 (human FGFR1, isoform FGFR1$\alpha$ and FGFR1$\beta$, respectively), wherein the higher values are preferred. Most preferably, the polypeptide has at least 99% homology to the FGFR as depicted in SEQ ID NO: 2, 4, 8, 10 or 12, respectively (e.g SEQ ID NO: 2 or 4 (human FG-FR1, isoform FGFR1$\alpha$ and FGFR1$\beta$, respectively).

In accordance with the above, the present invention relates to the following items:

1. A method for predicting the responsiveness of a mammalian tumor cell or cancer cell to an inhibitor of a fibroblast growth factor receptor (FGFR), said method comprising evaluating the status of FGFR and the status of c-myc in said cell, wherein said status of FGFR and of c-myc is indicative of the responsiveness of said cell to the inhibitor.

2. A method for predicting the responsiveness of an individual to an inhibitor of a fibroblast growth factor receptor (FGFR), said method comprising evaluating the status of FGFR and the status of c-myc in a sample of an individual suspected to suffer from cancer, suffering from cancer or being prone to suffer from cancer, wherein said status of FGFR and of c-myc is indicative of a responsive individual to the inhibitor.

3. The method of item 1 or 2, wherein said inhibitor of a fibroblast growth factor receptor (FGFR) is selected from the group consisting of BGJ398, PD173074, AP24534, AZD-4547, and E-3810 and pharmaceutically acceptable salts, solvates, and/or hydrates of these inhibitors.

4. The method of any one of items 1 to 3, wherein said status of FGFR is the gene amplification status of FGFR.

5. The method of any one of items 1 to 3, wherein said status of FGFR is the expression level of FGFR.

6. The method of any one of items 1 to 5, wherein said status of c-myc is the expression level of c-myc.

7. The method of item 6, wherein an increase in said gene amplification status of FGFR in comparison to the control and an increase in said expression level of c-myc in comparison to the control is indicative of the responsiveness of said cell to the inhibitor or indicative of a responsive individual to the inhibitor.

8. The method of any one of items 4 and 6 to 7, wherein said FGFR gene is at least twice amplified.

9. The method of any one of items 4 and 6 to 8, wherein said gene amplification status of the FGFR gene is detected by an in situ hybridization method or whole genome shotgun sequencing.

10. The method of item 9, wherein said in situ hybridization is selected from the group consisting of fluorescent in situ hybridization (FISH), chromogenic in situ hybridization (CISH) and silver in situ hybridization (SISH).

11. The method of item 6, wherein an increase in said expression level of FGFR in comparison to the control and an increase in said expression level of c-myc in comparison to the control is indicative of the responsiveness of said cell to the inhibitor or indicative of a responsive individual to the inhibitor.

12. The method of any one of items 5, 6 and 11, wherein said expression level of FGFR is at least 2.5-fold, preferably at least 5-fold increased in comparison to the control.

13. The method of any one of items 6 to 12, wherein said expression level of c-myc is at least 2.5-fold, preferably at least 5-fold increased in comparison to the control.

14. The method of any one of items 5, 6 and 11 to 13, wherein said expression level of FGFR is the protein expression level of FGFR.

15. The method of any one of items 6 to 14, wherein said expression level of c-myc is the protein expression level of c-myc.

16. The method of item 15, wherein said protein expression level of c-myc is intermediate.

17. The method of item 16, wherein said protein expression level is intermediate, if it is scored "2" in immunohistochemistry (IHC).

18. The method of item 15, wherein said protein expression level of c-myc is high.

19. The method of item 18, wherein said protein expression level of c-myc is high if it is scored "3" in immunohistochemistry (IHC).

20. The method of any one of items 14 to 19, wherein said protein expression level is assessed by IHC, immunoassay,

gel- or blot-based methods, mass spectrometry, flow cytometry, or FACS.

21. The method of any one of items 5, 6 and 11 to 13, wherein said expression level of FGFR is the mRNA expression level of FGFR.

22. The method of any one of items 6 to 13, wherein said expression level of c-myc is the mRNA expression level of c-myc.

23. The method of item 21 or 22, wherein the mRNA expression level is assessed by in situ hybridization, micro-arrays, RealTime PCR or RNAseq.

24. The method of any one of items 1 to 23, wherein the cancer/tumor cell is a solid cancer/solid tumor cell, like a lung cancer/tumor cell, such as a small cell lung cancer cell or non-small cell lung cancer cell, like a squamous cell lung cancer cell, or like a breast cancer cell or a bladder cancer cell;
or wherein said sample comprises a solid cancer/solid tumor cell, like a lung cancer/tumor cell, such as a small cell lung cancer cell or non-small cell lung cancer cell, like a squamous cell lung cancer cell, or like a breast cancer cell or a bladder cancer cell.

25. The method of any of one of items 2 to 23, wherein said cancer is a solid cancer.

26. The method of item 25, wherein said cancer is a lung cancer, such as small cell lung cancer or non-small cell lung cancer, like squamous cell lung cancer, or breast cancer or bladder cancer.

27. The method of any one of items 1 to 26, wherein said FGFR is FGFR1, like human FGFR1.

28. FGFR and c-myc for use in detecting an individual responsive to an FGFR inhibitor as defined in any of items 1 to 3.

29. Use of a nucleic acid or antibody capable of detecting the gene amplification status or the expression level of FGFR and/or the use of a nucleic acid or antibody capable of detecting the expression level of c-myc for predicting the responsiveness of a cancer or tumor cell or a responsive individual to an FGFR inhibitor as defined in any of items 1 to 3.

30. A kit useful for carrying out the method of any one of items 1 to 27 comprising a nucleic acid or an antibody capable of detecting the gene amplification status or the expression level of FGFR and/or comprising a nucleic acid or an antibody capable of detecting the expression level of c-myc.

31. An FGFR inhibitor as defined in any of items 1 to 3 for use in the treatment of cancer in an individual identified by the method of any one of items 2 to 27.

32. A method for the treatment of cancer comprising administering an effective amount of an FGFR inhibitor as defined in any of item 1 to 3 to an individual identified by the method of any one of items 2 to 27 in need of such a treatment.

33. The method of item 32, wherein said individual is a human.

34. The method of item 32 or 33, or the inhibitor of item31, wherein said inhibitor is administered as a single anti-tumor agent.

35. The method of item 32 or 33, or the inhibitor of item 31, wherein said inhibitor is administered in form of a combination therapy.

36. The method of item 35, or the inhibitor of item 35, wherein the therapy used in said combination therapy is chemotherapy or an anti-hormonal therapy.

37. The method of item 36, or the inhibitor of item 36, wherein said chemotherapy is selected from the group consisting of anthracycline/taxane chemotherapy, therapy with an antimetabolite agents, therapy with an anti-hormonal compound, therapy with an anti-estrogen, therapy with a tyrosine kinase inhibitor, therapy with a raf inhibitor, therapy with a ras inhibitor, therapy with a dual tyrosine kinase inhibitor, therapy with taxol, therapy with

taxane, therapy with doxorubicin, therapy with adjuvant (anti-) hormone drugs, therapy with cisplatin and the like.

38. The method of any one of items 32 to 37, or the inhibitor of any of items 31 and 34 to 37, wherein said inhibitor is administered by any one of a parenteral route, oral route, intravenous route, subcutaneous route, intranasal route or transdermal route.

39. The method of any one of items 32 to 38, or the inhibitor of any of items 31 and 34 to 37, wherein said inhibitor is to be administered in a neoadjuvant or adjuvant setting.

The present invention is further illustrated by reference to the following non-limiting figures and examples. Unless otherwise indicated, established methods of recombinant gene technology were used as described, for example, in Sambrook, Russell "Molecular Cloning, A Laboratory Manual", Cold Spring Harbor Laboratory, N.Y. (2001)) which is incorporated herein by reference in its entirety.

The Figures show:

**Figure 1. Colony formation in soft agar assay.**
Colony formation in soft agar assay using KRAS G12V (positive control), FGFR1 plus c-Myc, two different splice variants of FGFR1, c-Myc and empty Vector (negative control) and treatment with increasing concentrations of an FGFR1 inhibitor (PD173074).

**Figure 2. Apoptosis FACS assay.**
Apoptosis FACS assay shows the treatment of NIH3T3 cells expressing FGFR1 alone or in combination with Myc and treated with DMSO (control) and increased concentrations of FGFR inhibitor PD173074.

**Figure 3. Xenograft mouse model showing responsiveness of tumors with FGFR1 and c-myc expression.**
Xenograft mouse model experiment shows tumor size for treated and non-treated mice with FGFR inhibitor BGJ398. Tumors derived from NIH3T3 cells expressing KRas G12V (positive control) show strong tumor growth upon treatment with an FGFR inhibitor. Tumors derived from NIH3T3 cells expressing only FGFR1 (i.e. no myc expression) show a stable disease upon treatment with an FGFR inhibitor. Tumors derived from NIH3T3 cells expressing two different splice variants of FGFR1 plus Myc show strong reduction of tumor volume. The tumor disappeared within ten days of treatment.

**Figure 4. Iimmunohistochemical Myc staining of four Xenograft mouse tumors.**
Tumor expressing FGFR1α and FGFR1β isoform shows Myc score 2. Tumor expressing KRas G12V shows Myc score 0 and tumor expressing FGFR1β plus Myc shows Myc score 3.

**Figure 5. Xenograft mouse model treated with FGFR inhibitor BGJ398 shows responsiveness of tumors with FGFR1 and c-myc expression.**
Xenograft mouse model of FGFR1 and c-myc expressing tumor and xenograft mouse model of only FGFR1 expressing tumor. The Figure shows the influence of the treatment with FGFR inhibitor BGJ398 on the tumors. The tumors of the non-treated mouse showed enhanced tumor volume as expected. In relation to the treated mouse, the tumor expressing only FGFR1 did not shrink and even little grew, whereas the tumor with FGFR1 and c-myc expression completely disappeared after ten days of treatment.

**Figure 6. Xenograft mouse model experiments**
Xenograft mouse model experiments show tumor volume for treated and non-treated mice with FGFR inhibitor BGJ398. Tumors derived from NIH3T3 cells expressing EML4-Alk (positive control) show an increase in tumor volume. Tumors derived from NIH3T3 cells expressing two different splice variants of FGFR1 (no c-myc expression) show stable disease under treatment (i.e. no reduction of tumor volume).

**Figure 7. Iimmunohistochemical Myc staining of four Xenograft mouse tumors.**
The samples were stained for Myc protein. EML4-Alk tumors not responding to FGFR1 inhibition have a Myc score 0. FGFR1 tumors with stable disease show Myc score 2.

**Figure 8. RT-PCR data derived from different FGFR1-amplified and FGFR1 non-amplified cell lines.**
IC50 values are given in μM for PD173074 inhibitor. The left column of each cell lines shows expression of FGFR isoforms FGFR1, FGFR2, FGFR3 and FGFR4. The right column shows c-myc expression. Further, the copy number of the FGFR1 gene is indicated for each cell line. In general cell lines with FGFR1 amplification show high expression

of FGFR1, i.e. the FGFR1 expression correlates with FGFR1 amplification Cell lines expressing high amounts of FGFR and Myc show lower IC50 values, i.e. are more responsive to the FGFR inhibitor.

**Figure 9. RT-PCR data from different FGFR1-amplified and FGFR1 non-amplified cell lines.**
FGFR1-amplified and FGFR1 non-amplified cell lines express different amounts of FGFR1 splice variants. Respnsive cell lines H1581 and A427 expressing high amounts of FGFR, with a higher FGFR1β/FGFR1α ratio together with high Myc expression, show lower IC50 values (compare to Figure 8.).

**Figure 10. Western Blot shows cytochrome c relase in cells treated with an FGFR1 inhibitor.**
The Western Blot shows cytochrome c relase in responsive H1581 cells treated with an FGFR1 inhibitor PD173074 compared to control cell line H358 indicating the central role of Myc.

**Figure 11. Mitochondrial potential breakdown.**
Here the mitochondrial potential breakdown under 1μM PD173074 treatment over time is shown, indicating a c-myc induced apoptosis signalling.

**Figure 12. Knockdown of c-myc expression in cell line H1581 renders the cell line less responsive to the FGFR inhibitor PD173074.**
Two different H1581 cell lines are screened against different concentrations of PD173074. H1581 cell line is normally highly sensitive/responsive to FGFR inhibition. When Myc is downregulated, the same cell line shows less response to FGFR inhibition (marked as shMyc). These in vitro cell line data give a clear link to what is observed in the in-vitro model Figure 2 and in-vivo model Figure 3 and 5.

The Example illustrates the invention.

**<u>Example 1: FGFR1 expression or amplification and c-MYC expression as a marker for predicting responsiveness to a FGFR1 inhibitor treatment</u>**

Materials & Methods

*Reagents*

PD173074 (Tocris) and BGJ 398 (Activebiochem) was purchased from commercial suppliers, dissolved in dimethyl sulfoxide (DMSO) or vehicle solution, and stored at -20°C.

*Immunoblotting*

To assess protein levels and protein phosphorylation status, cells were plated in growth medium and lysed after 24h of incubation using 20 mM Tris-- - HCl (pH 7.5), 150 mM NaCl, 1 mM Na2EDTA, 1 mM EGTA, 1% Triton X-- - 100 2.5 mM sodium pyrophosphate, 1 mM beta-- glycerophosphate, 1 mM Na3VO4, 1 μg/ml leupeptin, 1 mM PMSF, complete Protease Inhibitors Cocktail and Phosphatase Inhibitors Cocktail Set II. Lysates were cleared after 15 minutes of incubation at 4°C by centrifugation (14000 rpm for 20 minutes, 4°C) and boiled with SDS sample buffer (5x SDS sample buffer: 250 mM Tris (pH 6.8), 50% glycerol,3.575 M b-- mercaptoethanol, 10% SDS and 0.05% bromophenolblue) for 10 minutes. Lysates were separated by 4-- 12% SDS-- - PAGE, transferred to nitrocellulose membranes and detected by immunoblotting. Western blotting was performed using the following antibodies: Myc, CDK-9, Cytochrome C and BAX from Cell Signaling, Actin from MP Biomedical, FGFR1 from Santa Cruz and Bim and BAD from BD Laboratories.

*Immunohistochemical*

Paraffin embedded samples were washed 2x2 minutes with PBS-Tween 20. Incubate sections with 1% Triton X-100 diluted in PBS for 30 minutes at room temperature. Incubate sections directly with normal goat serum blocking solution for 30 minutes at room temperature. Wash 3x2 minutes with PBS-Tween 20. Incubate sections with Unconjugated AffiniPure Fab Fragment Goat Anti-Mouse IgG (H+L) (Jackson ImmunoResearch Labs, Code Number: 115-007-003) for 1 hour at room temperature. Wash 3x2 minutes with PBS-Tween 20. Incubate sections with primary Myc antibody for 30 minutes at room temperature. Wash 2x2 minutes with PBS-Tween 20. Incubate sections with 3% H2O2 in PBS for 10 minutes to block endogenous peroxidase. Wash 3x2 minutes with PBS-Tween 20. Incubate sections with secondary Antibody for 20 minutes at room temperature. Wash 3x2 minutes with PBS-Tween 20. Incubate sections with HRP-Streptavidin in PBS for 20 minutes at room temperature. Wash 3x2 minutes with PBS-Tween 20. Incubate sections with

DAB for 5 minutes. Wash with tap water briefly. Dehydrate through 95% and 100% ethanol. Clear with xylene. Coverslip with permanent mounting medium.

*Cell-- - based screening*

All compounds were purchased from commercial suppliers, dissolved in DMSO, and stored at - 20°C. Cells were plated into sterile 96-- - well microtiter plates using a multichannel pipette and cultured overnight. Compounds were then added in serial dilutions. Cellular viability was determined after 96 hours of treatment by measuring cellular ATP content using the CellTiter-- Glo Assay (Promega). Plates were measured on a Mithras LB 940 Plate Reader (Berthold Technologies). GI50 values were determined using the following R package (Frommolt P, Thomas RK, 2008, Standardized high-- - throughput evaluation of cell-- - based compound screens. BMC Bioinformatics. 9:475)

*Mitochondrial Membrane Potential Detection*

Breakdown of the mitochondrial potential was assessed by JC-1 staining. After treatment with VX680 cells were incubated with 2μM Tetrachloro-tetraethylbenzimidazolylcarbocyanine-iodide (JC-1, Biomol) for 30 minutes at 37°C. Cells were washed twice with cold PBS, incubated with accutase solution (Sigma Aldrich) for one minute and resuspended in Annexin-V binding buffer. For the analysis on a FACS Gallios Flow Cytometer (Beckman Coulter) FL-1 and FL-2 channels were used to compensate and detect changes of mitochondrial membrane potential, respectively. Results were calculated using FACS Kaluza analysis software (Beckman Coulter). At least 50,000 cells were measured per measurement.

*Apoptosis*

For determination of apoptosis, cells were seeded in six-well plates, incubated for 24 hours, treated with either DMSO (control) or 0.5 and 1.0 μM PD173074 for 96 hours, and stained with annexin V and propidium iodide (PI). Finally, the cells were analyzed on a FACSCanto flow cytometer (BD Biosciences).

*Cloning of FGFR1, production of retroviruses and transductions*

Total RNA was extracted from H1581 cells (ATCC). RNA was extracted using Qiagen RNeasy Mini Kit (Qiagen) and 1 μg RNA was reverse transcribed using the SuperScript III Reverse Transcriptase kit (Invitrogen) according to the manufacturer's instructions. cDNA was cleaned by using the Nucleo Spin Extract II Kit (Macherey-Nagel) according to the manufacturer's instructions. FGFR1 was amplified by RT-PCR from the cDNA using FGFR1 specific primers (forward 5'- ATGTGGAGCTGGAAGTGC - 3' (SEQ ID No: 15)), and reverse 5'-TCAGCGGCGTTTGAGTC - 3' (SEQ ID No: 16)) and under followed conditions: 95°C 2min., 30 cycles (95°C 30sec., 57°C 30sec., 72°C 3min.), 72°C 5min. The product was cleaned again by using the Nucleo Spin Extract II Kit (Macherey-Nagel) according to the manufacturer's instructions. Resulting FGFR1 DNA fragments were amplified by PCR again using FGFR1 specific primers with attB overhangs (Invitrogen) (forward 5'-AAAAAGCAGGCTTCACC ATGTGGAGCTGGAAGTGC- 3' (SEQ ID No:17)), and reverse 5'-AGAAAGCTGGGTC TCAGCGGCGTTTGAGTC- 3' (SEQ ID No: 18)) and under followed conditions: 94°C 2min., 10 cycles (94°C 45sec., 54°C 45sec., 72°C 2min. 30sec.), 72°C 5min. 10μl of PCR product was used for second step PCR with attB Primers (forward 5'-GGGGACAAGTTTGTACAAAAAAGCAGGCT- 3' (SEQ ID No: 19), and reverse 5'-GGGGACCACTTTGTACAAGAAAGCTGGGT- 3' (SEQ ID No: 20)) and under followed conditions: 98°C 1min., 5 cycles (98°C 15sec., 45°C 30sec., 72°C 2min. 30sec.), 20 cycles (98°C 15sec., 55°C 30sec., 72°C 2min. 30sec.), 72°C 10min. Product was cleaned again by using the Nucleo Spin Extract II Kit (Macherey-Nagel) according to the manufacturer's instructions. Resulting FGFR1 fragments were fliped into pDONR221 vector using the BP-Clonase (Invitrogen), sequenced and fliped into pBabe puro by using LR-Clonase (Invitrogen). 4μg pBabe puro FGFR1 vector together with 4μg pCL vector were co-transfected into Hek293T packaging cells (ATCC), using TransIT-TKO transfection agent (Mirus). Same was done with pBabe zeo c-Myc (from Addgene). After 48 hours, FGFR1 viral supernatants were filtered, mixed with medium containing total concentration of 8μg/ml polybrene (Sigma) and incubated with NIH3T3 cells (both from ATCC) for 10 hours. After 12 hours, cells were transferred into medium containing 3μg/ml puromycin. After selection the whole process was repeated with c-Myc viral supernatant (After puromycin selection, myc viral supernatants were filtered, mixed with medium containing total concentration of 8μg/ml polybrene (Sigma) and incubated with NIH3T3 FGFR1 cells for 10 hours. After 12 hours, cells were transferred into medium containing 500μg/ml zeocin).

*Soft agar assay*

Cells were suspended in growth media containing 10% calf serum (CS) and 0.6% agar and plated in triplicate

on 50 μl of solidified growth medium (10% CS; 1.0% agar). Growth medium containing indicated compound concentrations was added on top. Colonies were analyzed with the Scanalyzer imaging system (LemnaTec) or counted by hand.

*Xenografts*

All animal procedures were approved by the local animal protection committee and the local authorities. Xenograft-experiments were performed in 10 to 15 week old male athymic NMRI-nude-mice (Janvier, Europe). Mice were hold in a 12 hour light-dark-cycle. They got water and standard food ad libitium.

NIH3T3-cells transduced with empty Vector (puro), empty Vector (puro+zeo), KRAS G12V, FGFR1-α, FGFR1-β, FGFR1-β + cMyc were cultured as described before.
For implantation they were resuspended in plain RPMI in a concentration of 50*10^6 cells/ml. 5*10^6 cells were injected subcutaneously in mice, under 2.5% Isolflurane-anaesthesia. Tumorgrowth was monitored every 2nd day by calimetric measurement.
Tumorvolumes were calculated by V= (long diameter)$^2$ x (short diameter).

Therapy was started when tumors reached a size of 100mm$^3$. They were treated daily by oral gavage either with 10mg/kg NVP-BGJ 398 (Novartis) dissolved in vehicle (PEG300, 5%Glucose; 2:1) or with the vehicle-detergent alone.
After 8 (KRas), 12 (FGFR1-α and FGFR1-β + cMyc), days of therapy mice were sacrificed by intraperitoneal injection of Ketamin/Xylazine (300/60 mg/kg) and tumors were resected and fixed in 4% formaldehyde for histology.

**Results**

The present inventors detected in a subset of 436 squamos cell lung cancer tumors frequent and focal fibroblast growth factor receptor 1 (FGFR1) amplification (up to 20% of cases). Further, it was found that c-Myc amplification co-occurs with FGFR1 amplification with a p-value < 0.001 in squamos cell lung cancers. NIH3T3 cells expressing only FGFR1, do not form colonies in soft agar (tested as well for different splice forms), while co-expressing FGFR1 together with c-Myc in NIH3T3 cells form massive colony formation in soft agar. This effect was invertible by treating the cells with an FGFR inhibitor (PD173074); see Figure 1.

Furthermore, we were able to show that NIH3T3 cells massively die if they just express c-Myc (data not shown). NIH3T3 cells are perfectly happy if they express just FGFR1 or FGFR1 in combination with Myc. When FGFR1 and Myc is co-expressed and treated with an FGFR1 inhibitor the cells massively run into apoptosis, while the FGFR inhibitor treatment of NIH3T3 cells expressing just FGFR1, does not harm the cells at all; see Figure 2.

Bringing NIH3T3 cells into a xenograft mouse model, it was found that NIH3T3 cells expressing just FGFR1 form tumors in mice. NIH3T3 cells expressing FGFR1 and Myc form tumours in mice as well with an even more aggressive phenotype resulting in bigger tumor. The tumors in mice in a xenograft mouse model were treated with an FGFR inhibitor (BGJ398) and the effect of the treatment on tumor volume was investigated..

Tumor types expressing only FGFR1 showed a stable disease upon treatment (i.e. the tumor volume did not decrease or even continued growing at a reduced rate); see Figure 3, 5 and 6. Tumors expressing FGFR1 in combination with high Myc expression showed a clear shrinkage of the tumor under inhibitor treatment; see Figure 3 and 5.

An upregulation of Myc under FGFR1 expression was found, i.e. in tumor types with FGFR1 expression (i.e. tumors derived from NIH3T3 cells expressing only FGFR1), FGFR1 induced c-myc expression at a score "2"; see Figure 4 and 7. in other words, tumors derived from NIH3T3 cells expressing only FGFR1) are expected to show an intermediate c-myc expression.

Tumor types expressing FGFR1 and c-myc (i.e. tumors derived from NIH3T3 cells expressing FGFR1 and c-myc), i.e. tumors with an FGFR independent upregulation of Myc show an evenbetter response to FGFR inhibition; see Figure 1, 2, 3 and 5. These tumors shrank upon treatment with an FGFR inhibitor and disappeared.

Comparing these data with cell line models, it was shown that cell lines with FGFR1 amplification expressing high amounts of FGFR and Myc respond to FGFR inhibition in a much better way. They show a Myc induced mitochondrial potential breakdown (by cytochrome c relase); see Figure 8, 10 and 11. In opposite, down regulation of Myc, in a cell line highly sensitive to FGFR inhibition, leads to less sensitivity to FGFR inhibition; see Figure 12.

Thereby a Myc score was identified which is predictive for the response to FGFR inhibition in FGFR1 amplified squamous cell lung cancer; see Figure 4 and 7. It is expected that 35% of all patients with squamous cell lung cancer and FGFR1 amplification show Myc score 2 and 3. It is expected that 20 % of all patients with squamous cell lung cancer and FGFR1 amplification show a Myc score 3.

The present invention refers to the following nucleotide and amino acid sequences: The sequences provided herein are available in the Ensembl database and can be retrieved from www.ensembl.org; Theses sequences also relate to annotated and modified sequences. The present invention also provides techniques and methods wherein homologous sequences, and variants of the concise sequences provided herein are used. Preferably, such "variants"

are genetic variants.

SEQ ID No. 1:

Nucleotide sequence encoding homo sapiens Fibroblast Growth Factor Receptor 1 β isoform (FGFR1) >ENST00000356207

ATGTGGAGCTGGAAGTGCCTCCTCTTCTGGGCTGTGCTGGTCACAGCCACACTCTGCACC
GCTAGGCCGTCCCCGACCTTGCCTGAACAAGATGCTCTCCCCTCCTCGGAGGATGATGAT
GATGATGATGACTCCTCTTCAGAGGAGAAAGAAACAGATAACACCAAACCAAACCGTATG
CCCGTAGCTCCATATTGGACATCCCCAGAAAAGATGGAAAAGAAATTGCATGCAGTGCCG
GCTGCCAAGACAGTGAAGTTCAAATGCCCTTCCAGTGGGACCCCAAACCCCACACTGCGC
TGGTTGAAAAATGGCAAAGAATTCAAACCTGACCACAGAATTGGAGGCTACAAGGTCCGT
TATGCCACCTGGAGCATCATAATGGACTCTGTGGTGCCCTCTGACAAGGGCAACTACACC
TGCATTGTGGAGAATGAGTACGGCAGCATCAACCACACATACCAGCTGGATGTCGTGGAG
CGGTCCCCTCACCGGCCCATCCTGCAAGCAGGGTTGCCCGCCAACAAAACAGTGGCCCTG
GGTAGCAACGTGGAGTTCATGTGTAAGGTGTACAGTGACCCGCAGCCGCACATCCAGTGG
CTAAAGCACATCGAGGTGAATGGGAGCAAGATTGGCCCAGACAACCTGCCTTATGTCCAG
ATCTTGAAGACTGCTGGAGTTAATACCACCGACAAAGAGATGGAGGTGCTTCACTTAAGA
AATGTCTCCTTTGAGGACGCAGGGGAGTATACGTGCTTGGCGGGTAACTCTATCGGACTC
TCCCATCACTCTGCATGGTTGACCGTTCTGGAAGCCCTGGAAGAGAGGCCGGCAGTGATG
ACCTCGCCCCTGTACCTGGAGATCATCATCTATTGCACAGGGGCCTTCCTCATCTCCTGC
ATGGTGGGGTCGGTCATCGTCTACAAGATGAAGAGTGGTACCAAGAAGAGTGACTTCCAC
AGCCAGATGGCTGTGCACAAGCTGGCCAAGAGCATCCCTCTGCGCAGACAGGTAACAGTG
TCTGCTGACTCCAGTGCATCCATGAACTCTGGGGTTCTTCTGGTTCGGCCATCACGGCTC
TCCTCCAGTGGGACTCCCATGCTAGCAGGGGTCTCTGAGTATGAGCTTCCCGAAGACCCT
CGCTGGGAGCTGCCTCGGGACAGACTGGTCTTAGGCAAACCCCTGGGAGAGGGCTGCTTT
GGGCAGGTGGTGTTGGCAGAGGCTATCGGGCTGGACAAGGACAAACCCAACCGTGTGACC
AAAGTGGCTGTGAAGATGTTGAAGTCGGACGCAACAGAGAAAGACTTGTCAGACCTGATC
TCAGAAATGGAGATGATGAAGATGATCGGGAAGCATAAGAATATCATCAACCTGCTGGGG
GCCTGCACGCAGGATGGTCCCTTGTATGTCATCGTGGAGTATGCCTCCAAGGGCAACCTG
CGGGAGTACCTGCAGGCCCGGAGGCCCCCAGGGCTGGAATACTGCTACAACCCCAGCCAC
AACCCAGAGGAGCAGCTCTCCTCCAAGGACCTGGTGTCCTGCGCCTACCAGGTGGCCCGA
GGCATGGAGTATCTGGCCTCCAAGAAGTGCATACACCGAGACCTGGCAGCCAGGAATGTC
CTGGTGACAGAGGACAATGTGATGAAGATAGCAGACTTTGGCCTCGCACGGGACATTCAC
CACATCGACTACTATAAAAAGACAACCAACGGCCGACTGCCTGTGAAGTGGATGGCACCC
GAGGCATTATTTGACCGGATCTACACCCACCAGAGTGATGTGTGGTCTTTCGGGGTGCTC
CTGTGGGAGATCTTCACTCTGGGCGGCTCCCCATACCCCGGTGTGCCTGTGGAGGAACTT
TTCAAGCTGCTGAAGGAGGGTCACCGCATGGACAAGCCCAGTAACTGCACCAACGAGCTG
TACATGATGATGCGGGACTGCTGGCATGCAGTGCCCTCACAGAGACCCACCTTCAAGCAG
CTGGTGGAAGACCTGGACCGCATCGTGGCCTTGACCTCCAACCAGGAGTACCTGGACCTG
TCCATGCCCCTGGACCAGTACTCCCCCAGCTTTCCCGACACCCGGAGCTCTACGTGCTCC
TCAGGGGAGGATTCCGTCTTCTCTCATGAGCCGCTGCCCGAGGAGCCCTGCCTGCCCCGA
CACCCAGCCCAGCTTGCCAATGGCGGACTCAAACGCCGCTGA

SEQ ID No. 2:

Amino acid sequence of homo sapiens Fibroblast Growth Factor Receptor 1 (FGFR1), β isoform >ENST00000356207 peptide: ENSP00000348537

MWSWKCLLFWAVLVTATLCTARPSPTLPEQDALPSSEDDDDDDDSSSEEKETDNTKPN
RMPVAPYWTSPEKMEKKLHAVPAAKTVKFKCPSSGTPNPTLRWLKNGKEFKPDHRIGG
YKVRYATWSIIMDSVVPSDKGNYTCIVENEYGSINHTYQLDVVERSPHRPILQAGLPAN
KTVALGSNVEFMCKVYSDPQPHIQWLKHIEVNGSKIGPDNLPYVQILKTAGVNTTDKE
MEVLHLRNVSFEDAGEYTCLAGNSIGLSHHSAWLTVLEALEERPAVMTSPLYLEIIIYCT
GAFLISCMVGSVIVYKMKSGTKKSDFHSQMAVHKLAKSIPLRRQVTVSADSSASMNSG
VLLVRPSRLSSSGTPMLAGVSEYELPEDPRWELPRDRLVLGKPLGEGCFGQVVLAEAIG
LDKDKPNRVTKVAVKMLKSDATEKDLSDLISEMEMMKMIGKHKNIINLLGACTQDGPL
YVIVEYASKGNLREYLQARRPPGLEYCYNPSHNPEEQLSSKDLVSCAYQVARGMEYLA
SKKCIHRDLAARNVLVTEDNVMKIADFGLARDIHHIDYYKKTTNGRLPVKWMAPEALF
DRIYTHQSDVWSFGVLLWEIFTLGGSPYPGVPVEELFKLLKEGHRMDKPSNCTNELYM
MMRDCWHAVPSQRPTFKQLVEDLDRIVALTSNQEYLDLSMPLDQYSPSFPDTRSSTCSS
GEDSVFSHEPLPEEPCLPRHPAQLANGGLKRR*.

SEQIDNo.3:

Nucleotide sequence encoding homo sapiens Fibroblast Growth Factor Receptor 1 α isoform (FGFR1) >ENST00000397091

ATGTGGAGCTGGAAGTGCCTCCTCTTCTGGGCTGTGCTGGTCACAGCCACACTCTGC
ACCGCTAGGCCGTCCCCGACCTTGCCTGAACAAGCCCAGCCCTGGGGAGCCCCTGTG
GAAGTGGAGTCCTTCCTGGTCCACCCCGGTGACCTGCTGCAGCTTCGCTGTCGGCTG
CGGGACGATGTGCAGAGCATCAACTGGCTGCGGGACGGGGTGCAGCTGGCGGAAA
GCAACCGCACCCGCATCACAGGGGAGGAGGTGGAGGTGCAGGACTCCGTGCCCGCA
GACTCCGGCCTCTATGCTTGCGTAACCAGCAGCCCCTCGGGCAGTGACACCACCTAC
TTCTCCGTCAATGTTTCAGATGCTCTCCCCTCCTCGGAGGATGATGATGATGATGAT
GACTCCTCTTCAGAGGAGAAAGAAACAGATAACACCAAACCAAACCCCGTAGCTCC
ATATTGGACATCCCCAGAAAAGATGGAAAAGAAATTGCATGCAGTGCCGGCTGCCA
AGACAGTGAAGTTCAAATGCCCTTCCAGTGGGACCCCAAACCCCACACTGCGCTGG
TTGAAAAATGGCAAAGAATTCAAACCTGACCACAGAATTGGAGGCTACAAGGTCCG
TTATGCCACCTGGAGCATCATAATGGACTCTGTGGTGCCCTCTGACAAGGGCAACTA
CACCTGCATTGTGGAGAATGAGTACGGCAGCATCAACCACACATACCAGCTGGATG
TCGTGGAGCGGTCCCCTCACCGGCCCATCCTGCAAGCAGGGTTGCCCGCCAACAAA
ACAGTGGCCCTGGGTAGCAACGTGGAGTTCATGTGTAAGGTGTACAGTGACCCGCA
GCCGCACATCCAGTGGCTAAAGCACATCGAGGTGAATGGGAGCAAGATTGGCCCAG
ACAACCTGCCTTATGTCCAGATCTTGAAGACTGCTGGAGTTAATACCACCGACAAAG
AGATGGAGGTGCTTCACTTAAGAAATGTCTCCTTTGAGGACGCAGGGGAGTATACG
TGCTTGGCGGGTAACTCTATCGGACTCTCCCATCACTCTGCATGGTTGACCGTTCTGG
AAGCCCTGGAAGAGAGGCCGGCAGTGATGACCTCGCCCCTGTACCTGGAGATCATC

ATCTATTGCACAGGGGCCTTCCTCATCTCCTGCATGGTGGGGTCGGTCATCGTCTAC
AAGATGAAGAGTGGTACCAAGAAGAGTGACTTCCACAGCCAGATGGCTGTGCACAA
GCTGGCCAAGAGCATCCCTCTGCGCAGACAGGTAACAGTGTCTGCTGACTCCAGTGC
ATCCATGAACTCTGGGGTTCTTCTGGTTCGGCCATCACGGCTCTCCTCCAGTGGGAC
TCCCATGCTAGCAGGGGTCTCTGAGTATGAGCTTCCCGAAGACCCTCGCTGGGAGCT
GCCTCGGGACAGACTGGTCTTAGGCAAACCCCTGGGAGAGGGCTGCTTTGGGCAGG
TGGTGTTGGCAGAGGCTATCGGGCTGGACAAGGACAAACCCAACCGTGTGACCAAA
GTGGCTGTGAAGATGTTGAAGTCGGACGCAACAGAGAAAGACTTGTCAGACCTGAT
CTCAGAAATGGAGATGATGAAGATGATCGGGAAGCATAAGAATATCATCAACCTGC
TGGGGGCCTGCACGCAGGATGGTCCCTTGTATGTCATCGTGGAGTATGCCTCCAAGG
GCAACCTGCGGGAGTACCTGCAGGCCCGGAGGCCCCCAGGGCTGGAATACTGCTAC
AACCCCAGCCACAACCCAGAGGAGCAGCTCTCCTCCAAGGACCTGGTGTCCTGCGC
CTACCAGGTGGCCCGAGGCATGGAGTATCTGGCCTCCAAGAAGTGCATACACCGAG
ACCTGGCAGCCAGGAATGTCCTGGTGACAGAGGACAATGTGATGAAGATAGCAGAC
TTTGGCCTCGCACGGGACATTCACCACATCGACTACTATAAAAAGACAACCAACGG
CCGACTGCCTGTGAAGTGGATGGCACCCGAGGCATTATTTGACCGGATCTACACCCA
CCAGAGTGATGTGTGGTCTTTCGGGGTGCTCCTGTGGGAGATCTTCACTCTGGGCGG
CTCCCCATACCCCGGTGTGCCTGTGGAGGAACTTTTCAAGCTGCTGAAGGAGGGTCA
CCGCATGGACAAGCCCAGTAACTGCACCAACGAGCTGTACATGATGATGCGGGACT
GCTGGCATGCAGTGCCCTCACAGAGACCCACCTTCAAGCAGCTGGTGGAAGACCTG
GACCGCATCGTGGCCTTGACCTCCAACCAGGAGTACCTGGACCTGTCCATGCCCCTG
GACCAGTACTCCCCCAGCTTTCCCGACACCCGGAGCTCTACGTGCTCCTCAGGGGAG
GATTCCGTCTTCTCTCATGAGCCGCTGCCCGAGGAGCCCTGCCTGCCCCGACACCCA
GCCCAGCTTGCCAATGGCGGACTCAAACGCCGCTGA

The coding region ranges from nucleotide ATG to nucleotide TGA.

SEQ ID No. 4:

Amino acid sequence of homo sapiens Fibroblast Growth Factor Receptor 1 (FGFR1), α isoform >ENST00000397091 peptide: ENSP00000380280

MWSWKCLLFWAVLVTATLCTARPSPTLPEQAQPWGAPVEVESFLVHPGDLLQLRCRLR
DDVQSINWLRDGVQLAESNRTRITGEEVEVQDSVPADSGLYACVTSSPSGSDTTYFSVN
VSDALPSSEDDDDDDDSSSEEKETDNTKPNPVAPYWTSPEKMEKKLHAVPAAKTVKFK
CPSSGTPNPTLRWLKNGKEFKPDHRIGGYKVRYATWSIIMDSVVPSDKGNYTCIVENEY
GSINHTYQLDVVERSPHRPILQAGLPANKTVALGSNVEFMCKVYSDPQPHIQWLKHIEV
NGSKIGPDNLPYVQILKTAGVNTTDKEMEVLHLRNVSFEDAGEYTCLAGNSIGLSHHSA
WLTVLEALEERPAVMTSPLYLEIIIYCTGAFLISCMVGSVIVYKMKSGTKKSDFHSQMAV
HKLAKSIPLRRQVTVSADSSASMNSGVLLVRPSRLSSSGTPMLAGVSEYELPEDPRWELP
RDRLVLGKPLGEGCFGQVVLAEAIGLDKDKPNRVTKVAVKMLKSDATEKDLSDLISEM
EMMKMIGKHKNIINLLGACTQDGPLYVIVEYASKGNLREYLQARRPPGLEYCYNPSHNP
EEQLSSKDLVSCAYQVARGMEYLASKKCIHRDLAARNVLVTEDNVMKIADFGLARDIH
HIDYYKKTTNGRLPVKWMAPEALFDRIYTHQSDVWSFGVLLWEIFTLGGSPYPGVPVEE
LFKLLKEGHRMDKPSNCTNELYMMMRDCWHAVPSQRPTFKQLVEDLDRIVALTSNQE

YLDLSMPLDQYSPSFPDTRSSTCSSGEDSVFSHEPLPEEPCLPRHPAQLANGGLKRR*.

SEQ ID No.5:

Nucleotide sequence encoding homo sapiens myc. >ENST00000377970

CTGGATTTTTTTCGGGTAGTGGAAAACCAGCAGCCTCCCGCGACGATGCCCCTCAAC
GTTAGCTTCACCAACAGGAACTATGACCTCGACTACGACTCGGTGCAGCCGTATTTC
TACTGCGACGAGGAGGAGAACTTCTACCAGCAGCAGCAGCAGAGCGAGCTGCAGCC
CCCGGCGCCCAGCGAGGATATCTGGAAGAAATTCGAGCTGCTGCCCACCCCGCCCC
TGTCCCCTAGCCGCCGCTCCGGGCTCTGCTCGCCCTCCTACGTTGCGGTCACACCCTT
CTCCCTTCGGGGAGACAACGACGGCGGTGGCGGGAGCTTCTCCACGGCCGACCAGC
TGGAGATGGTGACCGAGCTGCTGGGAGGAGACATGGTGAACCAGAGTTTCATCTGC
GACCCGGACGACGAGACCTTCATCAAAAACATCATCATCCAGGACTGTATGTGGAG
CGGCTTCTCGGCCGCCGCCAAGCTCGTCTCAGAGAAGCTGGCCTCCTACCAGGCTGC
GCGCAAAGACAGCGGCAGCCCGAACCCCGCCCGCGGCCACAGCGTCTGCTCCACCT
CCAGCTTGTACCTGCAGGATCTGAGCGCCGCCGCCTCAGAGTGCATCGACCCCTCGG
TGGTCTTCCCCTACCCTCTCAACGACAGCAGCTCGCCCAAGTCCTGCGCCTCGCAAG
ACTCCAGCGCCTTCTCTCCGTCCTCGGATTCTCTGCTCTCCTCGACGGAGTCCTCCCC
GCAGGGCAGCCCCGAGCCCCTGGTGCTCCATGAGGAGACACCGCCCACCACCAGCA
GCGACTCTGAGGAGGAACAAGAAGATGAGGAAGAAATCGATGTTGTTTCTGTGGAA
AAGAGGCAGGCTCCTGGCAAAAGGTCAGAGTCTGGATCACCTTCTGCTGGAGGCCA
CAGCAAACCTCCTCACAGCCCACTGGTCCTCAAGAGGTGCCACGTCTCCACACATCA
GCACAACTACGCAGCGCCTCCCTCCACTCGGAAGGACTATCCTGCTGCCAAGAGGG
TCAAGTTGGACAGTGTCAGAGTCCTGAGACAGATCAGCAACAACCGAAAATGCACC
AGCCCCAGGTCCTCGGACACCGAGGAGAATGTCAAGAGGCGAACACACAACGTCTT
GGAGCGCCAGAGGAGGAACGAGCTAAAACGGAGCTTTTTTGCCCTGCGTGACCAGA
TCCCGGAGTTGGAAAACAATGAAAAGGCCCCCAAGGTAGTTATCCTTAAAAAAGCC
ACAGCATACATCCTGTCCGTCCAAGCAGAGGAGCAAAAGCTCATTTCTGAAGAGGA
CTTGTTGCGGAAACGACGAGAACAGTTGAAACACAAACTTGAACAGCTACGGAACT
CTTGTGCGTAA

The coding region ranges from nucleotide CTG to nucleotide TAA.

SEQ ID No. 6:

Amino acid sequence of homo sapiens myc >ENST00000377970 peptide: ENSP00000367207

LDFFRVVENQQPPATMPLNVSFTNRNYDLDYDSVQPYFYCDEEENFYQQQQQSELQPP
APSEDIWKKFELLPTPPLSPSRRSGLCSPSYVAVTPFSLRGDNDGGGGSFSTADQLEMVT
ELLGGDMVNQSFICDPDDETFIKNIIIQDCMWSGFSAAAKLVSEKLASYQAARKDSGSPN
PARGHSVCSTSSLYLQDLSAAASECIDPSVVFPYPLNDSSSPKSCASQDSSAFSPSSDSLLS
STESSPQGSPEPLVLHEETPPTTSSDSEEEQEDEEEIDVVSVEKRQAPGKRSESGSPSAGG
HSKPPHSPLVLKRCHVSTHQHNYAAPPSTRKDYPAAKRVKLDSVRVLRQISNNRKCTSP
RSSDTEENVKRRTHNVLERQRRNELKRSFFALRDQIPELENNEKAPKVVILKKATAYILS

VQAEEQKLISEEDLLRKRREQLKHKLEQLRNSCA*.

SEQ ID No. 7:

Nucleotide sequence encoding homo sapiens Fibroblast Growth Factor Receptor 2 (FGFR2) >ENST00000358487

ATGGTCAGCTGGGGTCGTTTCATCTGCCTGGTCGTGGTCACCATGGCAACCTTGTCCCTG
GCCCGGCCCTCCTTCAGTTTAGTTGAGGATACCACATTAGAGCCAGAAGAGCCACCAACC
AAATACCAAATCTCTCAACCAGAAGTGTACGTGGCTGCGCCAGGGGAGTCGCTAGAGGTG
CGCTGCCTGTTGAAAGATGCCGCCGTGATCAGTTGGACTAAGGATGGGGTGCACTTGGGG
CCCAACAATAGGACAGTGCTTATTGGGGAGTACTTGCAGATAAAGGGCGCCACGCCTAGA
GACTCCGGCCTCTATGCTTGTACTGCCAGTAGGACTGTAGACAGTGAAACTTGGTACTTC
ATGGTGAATGTCACAGATGCCATCTCATCCGGAGATGATGAGGATGACACCGATGGTGCG
GAAGATTTTGTCAGTGAGAACAGTAACAACAAGAGAGCACCATACTGGACCAACACAGAA
AAGATGGAAAAGCGGCTCCATGCTGTGCCTGCGGCCAACACTGTCAAGTTTCGCTGCCCA
GCCGGGGGGAACCCAATGCCAACCATGCGGTGGCTGAAAAACGGGAAGGAGTTTAAGCAG
GAGCATCGCATTGGAGGCTACAAGGTACGAAACCAGCACTGGAGCCTCATTATGGAAAGT
GTGGTCCCATCTGACAAGGGAAATTATACCTGTGTAGTGGAGAATGAATACGGGTCCATC
AATCACACGTACCACCTGGATGTTGTGGAGCGATCGCCTCACCGGCCCATCCTCCAAGCC
GGACTGCCGGCAAATGCCTCCACAGTGGTCGGAGGAGACGTAGAGTTTGTCTGCAAGGTT
TACAGTGATGCCCAGCCCCACATCCAGTGGATCAAGCACGTGGAAAAGAACGGCAGTAAA
TACGGGCCCGACGGGCTGCCCTACCTCAAGGTTCTCAAGGCCGCCGGTGTTAACACCACG
GACAAAGAGATTGAGGTTCTCTATATTCGGAATGTAACTTTTGAGGACGCTGGGGAATAT
ACGTGCTTGGCGGGTAATTCTATTGGGATATCCTTTCACTCTGCATGGTTGACAGTTCTG
CCAGCGCCTGGAAGAGAAAAGGAGATTACAGCTTCCCCAGACTACCTGGAGATAGCCATT
TACTGCATAGGGGTCTTCTTAATCGCCTGTATGGTGGTAACAGTCATCCTGTGCCGAATG
AAGAACACGACCAAGAAGCCAGACTTCAGCAGCCAGCCGGCTGTGCACAAGCTGACCAAA
CGTATCCCCCTGCGGAGACAGGTAACAGTTTCGGCTGAGTCCAGCTCCTCCATGAACTCC
AACACCCCGCTGGTGAGGATAACAACACGCCTCTCTTCAACGGCAGACACCCCCATGCTG
GCAGGGGTCTCCGAGTATGAACTTCCAGAGGACCCAAAATGGGAGTTTCCAAGAGATAAG
CTGACACTGGGCAAGCCCCTGGGAGAAGGTTGCTTTGGGCAAGTGGTCATGGCGGAAGCA
GTGGGAATTGACAAAGACAAGCCCAAGGAGGCGGTCACCGTGGCCGTGAAGATGTTGAAA
GATGATGCCACAGAGAAAGACCTTTCTGATCTGGTGTCAGAGATGGAGATGATGAAGATG
ATTGGGAAACACAAGAATATCATAAATCTTCTTGGAGCCTGCACACAGGATGGGCCTCTC
TATGTCATAGTTGAGTATGCCTCTAAAGGCAACCTCCGAGAATACCTCCGAGCCCGGAGG
CCACCCGGGATGGAGTACTCCTATGACATTAACCGTGTTCCTGAGGAGCAGATGACCTTC
AAGGACTTGGTGTCATGCACCTACCAGCTGGCCAGAGGCATGGAGTACTTGGCTTCCCAA
AAATGTATTCATCGAGATTTAGCAGCCAGAAATGTTTTGGTAACAGAAAACAATGTGATG
AAAATAGCAGACTTTGGACTCGCCAGAGATATCAACAATATAGACTATTACAAAAAGACC
ACCAATGGGCGGCTTCCAGTCAAGTGGATGGCTCCAGAAGCCCTGTTTGATAGAGTATAC
ACTCATCAGAGTGATGTCTGGTCCTTCGGGGTGTTAATGTGGGAGATCTTCACTTTAGGG
GGCTCGCCCTACCCAGGGATTCCCGTGGAGGAACTTTTTAAGCTGCTGAAGGAAGGACAC
AGAATGGATAAGCCAGCCAACTGCACCAACGAACTGTACATGATGATGAGGGACTGTTGG
CATGCAGTGCCCTCCCAGAGACCAACGTTCAAGCAGTTGGTAGAAGACTTGGATCGAATT
CTCACTCTCACAACCAATGAGGAATACTTGGACCTCAGCCAACCTCTCGAACAGTATTCA
CCTAGTTACCCTGACACAAGAAGTTCTTGTTCTTCAGGAGATGATTCTGTTTTTTCTCCA
GACCCCATGCCTTACGAACCATGCCTTCCTCAGTATCCACACATAAACGGCAGTGTTAAA
ACATGA

SEQ ID No.8:

Amino acid sequence of homo sapiens Fibroblast Growth Factor Receptor 2 (FGFR2) >ENST00000358487 peptide: ENSP00000351276

MVSWGRFICLVVVTMATLSLARPSFSLVEDTTLEPEEPPTKYQISQPEVYVAAPGESLEV
RCLLKDAAVISWTKDGVHLGPNNRTVLIGEYLQIKGATPRDSGLYACTASRTVDSETWYF
MVNVTDAISSGDDEDDTDGAEDFVSENSNNKRAPYWTNTEKMEKRLHAVPAANTVKFRCP

AGGNPMPTMRWLKNGKEFKQEHRIGGYKVRNQHWSLIMESVVPSDKGNYTCVVENEYGSI
NHTYHLDVVERSPHRPILQAGLPANASTVVGGDVEFVCKVYSDAQPHIQWIKHVEKNGSK
YGPDGLPYLKVLKAAGVNTTDKEIEVLYIRNVTFEDAGEYTCLAGNSIGISFHSAWLTVL
PAPGREKEITASPDYLEIAIYCIGVFLIACMVVTVILCRMKNTTKKPDFSSQPAVHKLTK
RIPLRRQVTVSAESSSSMNSNTPLVRITTRLSSTADTPMLAGVSEYELPEDPKWEFPRDK
LTLGKPLGEGCFGQVVMAEAVGIDKDKPKEAVTVAVKMLKDDATEKDLSDLVSEMEMMKM
IGKHKNIINLLGACTQDGPLYVIVEYASKGNLREYLRARRPPGMEYSYDINRVPEEQMTF
KDLVSCTYQLARGMEYLASQKCIHRDLAARNVLVTENNVMKIADFGLARDINNIDYYKKT
TNGRLPVKWMAPEALFDRVYTHQSDVWSFGVLMWEIFTLGGSPYPGIPVEELFKLLKEGH
RMDKPANCTNELYMMMRDCWHAVPSQRPTFKQLVEDLDRILTLTTNEEYLDLSQPLEQYS
PSYPDTRSSCSSGDDSVFSPDPMPYEPCLPQYPHINGSVKT

SEQ ID No. 9:

Nucleotide sequence encoding homo sapiens Fibroblast Growth Factor Receptor 3 (FGFR3) >ENST00000260795

ATGGGCGCCCCTGCCTGCGCCCTCGCGCTCTGCGTGGCCGTGGCCATCGTGGCCGGCGCC
TCCTCGGAGTCCTTGGGGACGGAGCAGCGCGTCGTGGGGCGAGCGGCAGAAGTCCCGGGC
CCAGAGCCCGGCCAGCAGGAGCAGTTGGTCTTCGGCAGCGGGGATGCTGTGGAGCTGAGC
TGTCCCCCGCCCGGGGGTGGTCCCATGGGGCCCACTGTCTGGGTCAAGGATGGCACAGGG
CTGGTGCCCTCGGAGCGTGTCCTGGTGGGGCCCCAGCGGCTGCAGGTGCTGAATGCCTCC
CACGAGGACTCCGGGGCCTACAGCTGCCGGCAGCGGCTCACGCAGCGCGTACTGTGCCAC
TTCAGTGTGCGGGTGACAGACGCTCCATCCTCGGGAGATGACGAAGACGGGGAGGACGAG
GCTGAGGACACAGGTGTGGACACAGGGGCCCCTTACTGGACACGGCCCGAGCGGATGGAC
AAGAAGCTGCTGGCCGTGCCGGCCGCCAACACCGTCCGCTTCCGCTGCCCAGCCGCTGGC
AACCCCACTCCCTCCATCTCCTGGCTGAAGAACGGCAGGGAGTTCCGCGGCGAGCACCGC
ATTGGAGGCATCAAGCTGCGGCATCAGCAGTGGAGCCTGGTCATGGAAAGCGTGGTGCCC
TCGGACCGCGGCAACTACACCTGCGTCGTGGAGAACAAGTTTGGCAGCATCCGGCAGACG
TACACGCTGGACGTGCTGGAGCGCTCCCCGCACCGGCCCATCCTGCAGGCGGGGCTGCCG
GCCAACCAGACGGCGGTGCTGGGCAGCGACGTGGAGTTCCACTGCAAGGTGTACAGTGAC
GCACAGCCCCACATCCAGTGGCTCAAGCACGTGGAGGTGAATGGCAGCAAGGTGGGCCCG
GACGGCACACCCTACGTTACCGTGCTCAAGACGGCGGGCGCTAACACCACCGACAAGGAG
CTAGAGGTTCTCTCCTTGCACAACGTCACCTTTGAGGACGCCGGGGAGTACACCTGCCTG
GCGGGCAATTCTATTGGGTTTTCTCATCACTCTGCGTGGCTGGTGGTGCTGCCAGCCGAG
GAGGAGCTGGTGGAGGCTGACGAGGCGGGCAGTGTGTATGCAGGCATCCTCAGCTACGGG
GTGGGCTTCTTCCTGTTCATCCTGGTGGTGGCGGCTGTGACGCTCTGCCGCCTGCGCAGC
CCCCCCAAGAAAGGCCTGGGCTCCCCCACCGTGCACAAGATCTCCCGCTTCCCGCTCAAG
CGACAGGTGTCCCTGGAGTCCAACGCGTCCATGAGCTCCAACACACCACTGGTGCGCATC
GCAAGGCTGTCCTCAGGGGAGGGCCCCACGCTGGCCAATGTCTCCGAGCTCGAGCTGCCT
GCCGACCCCAAATGGGAGCTGTCTCGGGCCCGGCTGACCCTGGGCAAGCCCCTTGGGGAG
GGCTGCTTCGGCCAGGTGGTCATGGCGGAGGCCATCGGCATTGACAAGGACCGGGCCGCC
AAGCCTGTCACCGTAGCCGTGAAGATGCTGAAAGACGATGCCACTGACAAGGACCTGTCG
GACCTGGTGTCTGAGATGGAGATGATGAAGATGATCGGGAAACACAAAAACATCATCAAC
CTGCTGGGCGCCTGCACGCAGGGCGGGCCCCTGTACGTGCTGGTGGAGTACGCGGCCAAG
GGTAACCTGCGGGAGTTTCTGCGGGCGCGGCGGCCCCCGGGCCTGGACTACTCCTTCGAC
ACCTGCAAGCCGCCCGAGGAGCAGCTCACCTTCAAGGACCTGGTGTCCTGTGCCTACCAG
GTGGCCCGGGGCATGGAGTACTTGGCCTCCCAGAAGTGCATCCACAGGGACCTGGCTGCC
CGCAATGTGCTGGTGACCGAGGACAACGTGATGAAGATCGCAGACTTCGGGCTGGCCCGG
GACGTGCACAACCTCGACTACTACAAGAAGACGACCAACGGCCGGCTGCCCGTGAAGTGG
ATGGCGCCTGAGGCCTTGTTTGACCGAGTCTACACTCACCAGAGTGACGTCTGGTCCTTT
GGGGTCCTGCTCTGGGAGATCTTCACGCTGGGGGGCTCCCCGTACCCCGGCATCCCTGTG
GAGGAGCTCTTCAAGCTGCTGAAGGAGGGCCACCGCATGGACAAGCCCGCCAACTGCACA
CACGACCTGTACATGATCATGCGGGAGTGCTGGCATGCCGCGCCCTCCCAGAGGCCCACC
TTCAAGCAGCTGGTGGAGGACCTGGACCGTGTCCTTACCGTGACGTCCACCGACGAGTAC
CTGGACCTGTCGGCGCCTTTCGAGCAGTACTCCCCGGGTGGCCAGGACACCCCCAGCTCC
AGCTCCTCAGGGGACGACTCCGTGTTTGCCCACGACCTGCTGCCCCCGGCCCCACCCAGC
AGTGGGGGCTCGCGGACGTGA

SEQ ID No. 10:

Amino acid sequence of homo sapiens Fibroblast Growth Factor Receptor 3 (FGFR3) >ENST00000260795 peptide: ENSP00000260795

MGAPACALALCVAVAIVAGASSESLGTEQRVVGRAAEVPGPEPGQQEQLVFGSGDAVELS
CPPPGGGPMGPTVWVKDGTGLVPSERVLVGPQRLQVLNASHEDSGAYSCRQRLTQRVLCH
FSVRVTDAPSSGDDEDGEDEAEDTGVDTGAPYWTRPERMDKKLLAVPAANTVRFRCPAAG
NPTPSISWLKNGREFRGEHRIGGIKLRHQQWSLVMESVVPSDRGNYTCVVENKFGSIRQT
YTLDVLERSPHRPILQAGLPANQTAVLGSDVEFHCKVYSDAQPHIQWLKHVEVNGSKVGP
DGTPYVTVLKTAGANTTDKELEVLSLHNVTFEDAGEYTCLAGNSIGFSHHSAWLVVLPAE
EELVEADEAGSVYAGILSYGVGFFLFILVVAAVTLCRLRSPPKKGLGSPTVHKISRFPLK
RQVSLESNASMSSNTPLVRIARLSSGEGPTLANVSELELPADPKWELSRARLTLGKPLGE
GCFGQVVMAEAIGIDKDRAAKPVTVAVKMLKDDATDKDLSDLVSEMEMMKMIGKHKNIIN
LLGACTQGGPLYVLVEYAAKGNLREFLRARRPPGLDYSFDTCKPPEEQLTFKDLVSCAYQ
VARGMEYLASQKCIHRDLAARNVLVTEDNVMKIADFGLARDVHNLDYYKKTTNGRLPVKW
MAPEALFDRVYTHQSDVWSFGVLLWEIFTLGGSPYPGIPVEELFKLLKEGHRMDKPANCT
HDLYMIMRECWHAAPSQRPTFKQLVEDLDRVLTVTSTDEYLDLSAPFEQYSPGGQDTPSS
SSSGDDSVFAHDLLPPAPPSSGGSRT

SEQ ID No. 11:

Nucleotide sequence encoding homo sapiens Fibroblast Growth Factor Receptor 4 (FGFR4) >ENST00000292408

ATGCGGCTGCTGCTGGCCCTGTTGGGGGTCCTGCTGAGTGTGCCTGGGCCTCCAGTCTTG
TCCCTGGAGGCCTCTGAGGAAGTGGAGCTTGAGCCCTGCCTGGCTCCCAGCCTGGAGCAG
CAAGAGCAGGAGCTGACAGTAGCCCTTGGGCAGCCTGTGCGTCTGTGCTGTGGGCGGGCT
GAGCGTGGTGGCCACTGGTACAAGGAGGGCAGTCGCCTGGCACCTGCTGGCCGTGTACGG
GGCTGGAGGGGCCGCCTAGAGATTGCCAGCTTCCTACCTGAGGATGCTGGCCGCTACCTC
TGCCTGGCACGAGGCTCCATGATCGTCCTGCAGAATCTCACCTTGATTACAGGTGACTCC
TTGACCTCCAGCAACGATGATGAGGACCCCAAGTCCCATAGGGACCCCTCGAATAGGCAC
AGTTACCCCCAGCAAGCACCCTACTGGACACACCCCCAGCGCATGGAGAAGAAACTGCAT
GCAGTACCTGCGGGGAACACCGTCAAGTTCCGCTGTCCAGCTGCAGGCAACCCCACGCCC
ACCATCCGCTGGCTTAAGGATGGACAGGCCTTTCATGGGGAGAACCGCATTGGAGGCATT
CGGCTGCGCCATCAGCACTGGAGTCTCGTGATGGAGAGCGTGGTGCCCTCGGACCGCGGC
ACATACACCTGCCTGGTAGAGAACGCTGTGGGCAGCATCCGCTATAACTACCTGCTAGAT
GTGCTGGAGCGGTCCCCGCACCGGCCCATCCTGCAGGCCGGGCTCCCGGCCAACACCACA
GCCGTGGTGGGCAGCGACGTGGAGCTGCTGTGCAAGGTGTACAGCGATGCCCAGCCCCAC
ATCCAGTGGCTGAAGCACATCGTCATCAACGGCAGCAGCTTCGGAGCCGACGGTTTCCCC
TATGTGCAAGTCCTAAAGACTGCAGACATCAATAGCTCAGAGGTGGAGGTCCTGTACCTG
CGGAACGTGTCAGCCGAGGACGCAGGCGAGTACACCTGCCTCGCAGGCAATTCCATCGGC
CTCTCCTACCAGTCTGCCTGGCTCACGGTGCTGCCAGAGGAGGACCCCACATGGACCGCA
GCAGCGCCCGAGGCCAGGTATACGGACATCATCCTGTACGCGTCGGGCTCCCTGGCCTTG
GCTGTGCTCCTGCTGCTGGCCGGGCTGTATCGAGGGCAGGCGCTCCACGGCCGGCACCCC
CGCCCGCCCGCCACTGTGCAGAAGCTCTCCCGCTTCCCTCTGGCCCGACAGTTCTCCCTG
GAGTCAGGCTCTTCCGGCAAGTCAAGCTCATCCCTGGTACGAGGCGTGCGTCTCTCCTCC
AGCGGCCCCGCCTTGCTCGCCGGCCTCGTGAGTCTAGATCTACCTCTCGACCCACTATGG
GAGTTCCCCCGGGACAGGCTGGTGCTTGGGAAGCCCCTAGGCGAGGGCTGCTTTGGCCAG
GTAGTACGTGCAGAGGCCTTTGGCATGGACCCTGCCCGGCCTGACCAAGCCAGCACTGTG
GCCGTCAAGATGCTCAAAGACAACGCCTCTGACAAGGACCTGGCCGACCTGGTCTCGGAG
ATGGAGGTGATGAAGCTGATCGGCCGACACAAGAACATCATCAACCTGCTTGGTGTCTGC
ACCCAGGAAGGGCCCCTGTACGTGATCGTGGAGTGCGCCGCCAAGGGAAACCTGCGGGAG
TTCCTGCGGGCCCGGCGCCCCCCAGGCCCCGACCTCAGCCCCGACGGTCCTCGGAGCAGT
GAGGGGCCGCTCTCCTTCCCAGTCCTGGTCTCCTGCGCCTACCAGGTGGCCCGAGGCATG
CAGTATCTGGAGTCCCGGAAGTGTATCCACCGGGACCTGGCTGCCCGCAATGTGCTGGTG
ACTGAGGACAATGTGATGAAGATTGCTGACTTTGGGCTGGCCCGCGGCGTCCACCACATT
GACTACTATAAGAAAACCAGCAACGGCCGCCTGCCTGTGAAGTGGATGGCGCCCGAGGCC
TTGTTTGACCGGGTGTACACACACCAGAGTGACGTGTGGTCTTTTGGGATCCTGCTATGG
GAGATCTTCACCCTCGGGGGCTCCCCGTATCCTGGCATCCCGGTGGAGGAGCTGTTCTCG
CTGCTGCGGGAGGGACATCGGATGGACCGACCCCCACACTGCCCCCCAGAGCTGTACGGG

CTGATGCGTGAGTGCTGGCACGCAGCGCCCTCCCAGAGGCCTACCTTCAAGCAGCTGGTG
GAGGCGCTGGACAAGGTCCTGCTGGCCGTCTCTGAGGAGTACCTCGACCTCCGCCTGACC
TTCGGACCCTATTCCCCCTCTGGTGGGGACGCCAGCAGCACCTGCTCCTCCAGCGATTCT
GTCTTCAGCCACGACCCCCTGCCATTGGGATCCAGCTCCTTCCCCTTCGGGTCTGGGGTG
CAGACATGA

SEQ ID No. 12:

Amino acid sequence of homo sapiens Fibroblast Growth Factor Receptor 4 (FGFR4) >ENST00000292408 peptide: ENSP00000292408

MRLLLALLGVLLSVPGPPVLSLEASEEVELEPCLAPSLEQQEQELTVALGQPVRLCCGRA
ERGGHWYKEGSRLAPAGRVRGWRGRLEIASFLPEDAGRYLCLARGSMIVLQNLTLITGDS
LTSSNDDEDPKSHRDPSNRHSYPQQAPYWTHPQRMEKKLHAVPAGNTVKFRCPAAGNPTP
TIRWLKDGQAFHGENRIGGIRLRHQHWSLVMESVVPSDRGTYTCLVENAVGSIRYNYLLD
VLERSPHRPILQAGLPANTTAVVGSDVELLCKVYSDAQPHIQWLKHIVINGSSFGADGFP
YVQVLKTADINSSEVEVLYLRNVSAEDAGEYTCLAGNSIGLSYQSAWLTVLPEEDPTWTA
AAPEARYTDIILYASGSLALAVLLLLAGLYRGQALHGRHPRPPATVQKLSRFPLARQFSL
ESGSSGKSSSSLVRGVRLSSSGPALLAGLVSLDLPLDPLWEFPRDRLVLGKPLGEGCFGQ
VVRAEAFGMDPARPDQASTVAVKMLKDNASDKDLADLVSEMEVMKLIGRHKNIINLLGVC
TQEGPLYVIVECAAKGNLREFLRARRPPGPDLSPDGPRSSEGPLSFPVLVSCAYQVARGM
QYLESRKCIHRDLAARNVLVTEDNVMKIADFGLARGVHHIDYYKKTSNGRLPVKWMAPEA
LFDRVYTHQSDVWSFGILLWEIFTLGGSPYPGIPVEELFSLLREGHRMDRPPHCPPELYG
LMRECWHAAPSQRPTFKQLVEALDKVLLAVSEEYLDLRLTFGPYSPSGGDASSTCSSSDS
VFSHDPLPLGSSSFPFGSGVQT

SEQ ID No. 13:

Nucleotide sequence encoding wild type homo sapiens TP53(ENST00000269305)

ATGGAGGAGCCGCAGTCAGATCCTAGCGTCGAGCCCCCTCTGAGTCAGGAAACATT
TTCAGACCTATGGAAACTACTTCCTGAAAACAACGTTCTGTCCCCCTTGCCGTCCCA
AGCAATGGATGATTTGATGCTGTCCCCGGACGATATTGAACAATGGTTCACTGAAGA
CCCAGGTCCAGATGAAGCTCCCAGAATGCCAGAGGCTGCTCCCCCCGTGGCCCCTGC
ACCAGCAGCTCCTACACCGGCGGCCCCTGCACCAGCCCCCTCCTGGCCCCTGTCATC
TTCTGTCCCTTCCCAGAAAACCTACCAGGGCAGCTACGGTTTCCGTCTGGGCTTCTTG
CATTCTGGGACAGCCAAGTCTGTGACTTGCACGTACTCCCCTGCCCTCAACAAGATG
TTTTGCCAACTGGCCAAGACCTGCCCTGTGCAGCTGTGGGTTGATTCCACACCCCCG
CCCGGCACCCGCGTCCGCGCCATGGCCATCTACAAGCAGTCACAGCACATGACGGA
GGTTGTGAGGCGCTGCCCCCACCATGAGCGCTGCTCAGATAGCGATGGTCTGGCCCC
TCCTCAGCATCTTATCCGAGTGGAAGGAAATTTGCGTGTGGAGTATTTGGATGACAG
AAACACTTTTCGACATAGTGTGGTGGTGCCCTATGAGCCGCCTGAGGTTGGCTCTGA
CTGTACCACCATCCACTACAACTACATGTGTAACAGTTCCTGCATGGGCGGCATGAA
CCGGAGGCCCATCCTCACCATCATCACACTGGAAGACTCCAGTGGTAATCTACTGGG
ACGGAACAGCTTTGAGGTGCGTGTTTGTGCCTGTCCTGGGAGAGACCGGCGCACAG
AGGAAGAGAATCTCCGCAAGAAAGGGGAGCCTCACCACGAGCTGCCCCCAGGGAG
CACTAAGCGAGCACTGCCCAACAACACCAGCTCCTCTCCCCAGCCAAAGAAGAAAC

CACTGGATGGAGAATATTTCACCCTTCAGATCCGTGGGCGTGAGCGCTTCGAGATGT
TCCGAGAGCTGAATGAGGCCTTGGAACTCAAGGATGCCCAGGCTGGGAAGGAGCCA
GGGGGGAGCAGGGCTCACTCCAGCCACCTGAAGTCCAAAAAGGGTCAGTCTACCTC
CCGCCATAAAAAACTCATGTTCAAGACAGAAGGGCCTGACTCAGACTGA

SEQ ID No. 14:

Nucleotide sequence encoding mutated homo sapiens TP53(c.734 G>T loss of function mutation)

ATGGAGGAGCCGCAGTCAGATCCTAGCGTCGAGCCCCCTCTGAGTCAGGAAACATT
TTCAGACCTATGGAAACTACTTCCTGAAAACAACGTTCTGTCCCCCTTGCCGTCCCA
AGCAATGGATGATTTGATGCTGTCCCCGGACGATATTGAACAATGGTTCACTGAAGA
CCCAGGTCCAGATGAAGCTCCCAGAATGCCAGAGGCTGCTCCCCCCGTGGCCCCTGC
ACCAGCAGCTCCTACACCGGCGGCCCCTGCACCAGCCCCCTCCTGGCCCCTGTCATC
TTCTGTCCCTTCCCAGAAAACCTACCAGGGCAGCTACGGTTTCCGTCTGGGCTTCTTG
CATTCTGGGACAGCCAAGTCTGTGACTTGCACGTACTCCCCTGCCCTCAACAAGATG
TTTTGCCAACTGGCCAAGACCTGCCCTGTGCAGCTGTGGGTTGATTCCACACCCCCG
CCCGGCACCCGCGTCCGCGCCATGGCCATCTACAAGCAGTCACAGCACATGACGGA
GGTTGTGAGGCGCTGCCCCCACCATGAGCGCTGCTCAGATAGCGATGGTCTGGCCCC
TCCTCAGCATCTTATCCGAGTGGAAGGAAATTTGCGTGTGGAGTATTTGGATGACAG
AAACACTTTTCGACATAGTGTGGTGGTGCCCTATGAGCCGCCTGAGGTTGGCTCTGA
CTGTACCACCATCCACTACAACTACATGTGTAACAGTTCCTGCATGGGCG**T**CATGAA
CCGGAGGCCCATCCTCACCATCATCACACTGGAAGACTCCAGTGGTAATCTACTGGG
ACGGAACAGCTTTGAGGTGCGTGTTTGTGCCTGTCCTGGGAGAGACCGGCGCACAG
AGGAAGAGAATCTCCGCAAGAAAGGGGAGCCTCACCACGAGCTGCCCCCAGGGAG
CACTAAGCGAGCACTGCCCAACAACACCAGCTCCTCTCCCCAGCCAAAGAAGAAAC
CACTGGATGGAGAATATTTCACCCTTCAGATCCGTGGGCGTGAGCGCTTCGAGATGT
TCCGAGAGCTGAATGAGGCCTTGGAACTCAAGGATGCCCAGGCTGGGAAGGAGCCA
GGGGGGAGCAGGGCTCACTCCAGCCACCTGAAGTCCAAAAAGGGTCAGTCTACCTC
CCGCCATAAAAAACTCATGTTCAAGACAGAAGGGCCTGACTCAGACTGA

SEQ ID No. 15:

Nucleotide sequence of FGFR1 specific primer (forward)
5'- ATGTGGAGCTGGAAGTGC - 3'

SEQ ID No. 16:

Nucleotide sequence of FGFR1 specific primer (reverse)
5'- TCAGCGGCGTTTGAGTC - 3'

SEQ ID No. 17:

Nucleotide sequence of FGFR1 specific primers with attB overhangs (forward)
5'-AAAAAGCAGGCTTCACC ATGTGGAGCTGGAAGTGC- 3'

SEQ ID No. 18:

Nucleotide sequence of FGFR1 specific primers with attB overhangs (reverse)
5'- AGAAAGCTGGGTC TCAGCGGCGTTTGAGTC- 3'

SEQ ID No. 19:

Nucleotide sequence of attB Primers (forward)
5'-GGGGACAAGTTTGTACAAAAAAGCAGGCT- 3'

SEQ ID No. 20:

Nucleotide sequence of attB Primers (reverse)
5'-GGGGACCACTTTGTACAAGAAAGCTGGGT- 3'

All references cited herein are fully incorporated by reference. Having now fully described the invention, it will be understood by a person skilled in the art that the invention may be practiced within a wide and equivalent range of conditions, parameters and the liek, without affecting the spirit or scope of the invention or any embodiment thereof.

## Claims

1. A method for predicting the responsiveness of a mammalian tumor cell or cancer cell to an inhibitor of a fibroblast growth factor receptor (FGFR), said method comprising evaluating the status of FGFR and the status of c-myc in said cell, wherein said status of FGFR and of c-myc is indicative of the responsiveness of said cell to the inhibitor.

2. A method for predicting the responsiveness of an individual to an inhibitor of a fibroblast growth factor receptor (FGFR), said method comprising evaluating the status of FGFR and the status of c-myc in a sample of an individual suspected to suffer from cancer, suffering from cancer or being prone to suffer from cancer, wherein said status of FGFR and of c-myc is indicative of a responsive individual to the inhibitor.

3. The method of claim 1 or 2, wherein said inhibitor of a fibroblast growth factor receptor (FGFR) is selected from the group consisting of BGJ398, PD173074, AP24534, AZD-4547, and E-3810 and pharmaceutically acceptable salts, solvates, and/or hydrates of these inhibitors.

4. The method of any one of claims 1 to 3, wherein said status of FGFR is the gene amplification status of FGFR.

5. The method of any one of claims 1 to 3, wherein said status of FGFR is the expression level of FGFR.

6. The method of any one of claims 1 to 5, wherein said status of c-myc is the expression level of c-myc.

7. The method of claim 6, wherein an increase in said gene amplification status of FGFR in comparison to the control and an increase in said expression level of c-myc in comparison to the control is indicative of the responsiveness of said cell to the inhibitor or indicative of a responsive individual to the inhibitor.

8. The method of claim 6, wherein an increase in said expression level of FGFR in comparison to the control and an increase in said expression level of c-myc in comparison to the control is indicative of the responsiveness of said cell to the inhibitor or indicative of a responsive individual to the inhibitor.

9. The method of any one of claims 5, 6 and 8, wherein said expression level of FGFR is the protein expression level of FGFR.

10. The method of any one of claims 5 to 9, wherein said expression level of c-myc is the protein expression level of c-myc; whereby said expression level is intermediate like a score of 2 in immunohistochemistry (IHC); or
whereby said expression level is high like a score of 3 in immunohistochemistry (IHC).

11. The method of any one of claims 1 to 10, wherein the cancer/tumor cell is a solid cancer/solid tumor cell, like a lung

cancer/tumor cell, such as a small cell lung cancer cell or non-small cell lung cancer cell, like a squamous cell lung cancer cell, a breast cancer cell or bladder cancer; or wherein said sample comprises a solid cancer/solid tumor cell, like a lung cancer/tumor cell, such as a small cell lung cancer cell or non-small cell lung cancer cell, like a squamous cell lung cancer cell, or like a breast cancer cell or bladder cancer cell..

**12.** The method of any of one of claims 2 to 10, wherein said cancer is a solid cancer.

**13.** The method of claim 12, wherein said cancer is a lung cancer, such as small cell lung cancer or non-small cell lung cancer, like squamous cell lung cancer, breast cancer or bladder cancer.

**14.** The method of any one of claims 1 to 13, wherein said FGFR is FGFR1, such as human FGFR1.

**15.** An FGFR inhibitor as defined in any of claims 1 to 3 for use in the treatment of cancer in an individual identified by the method of any one of claims 2 to 14.

Figure 1.

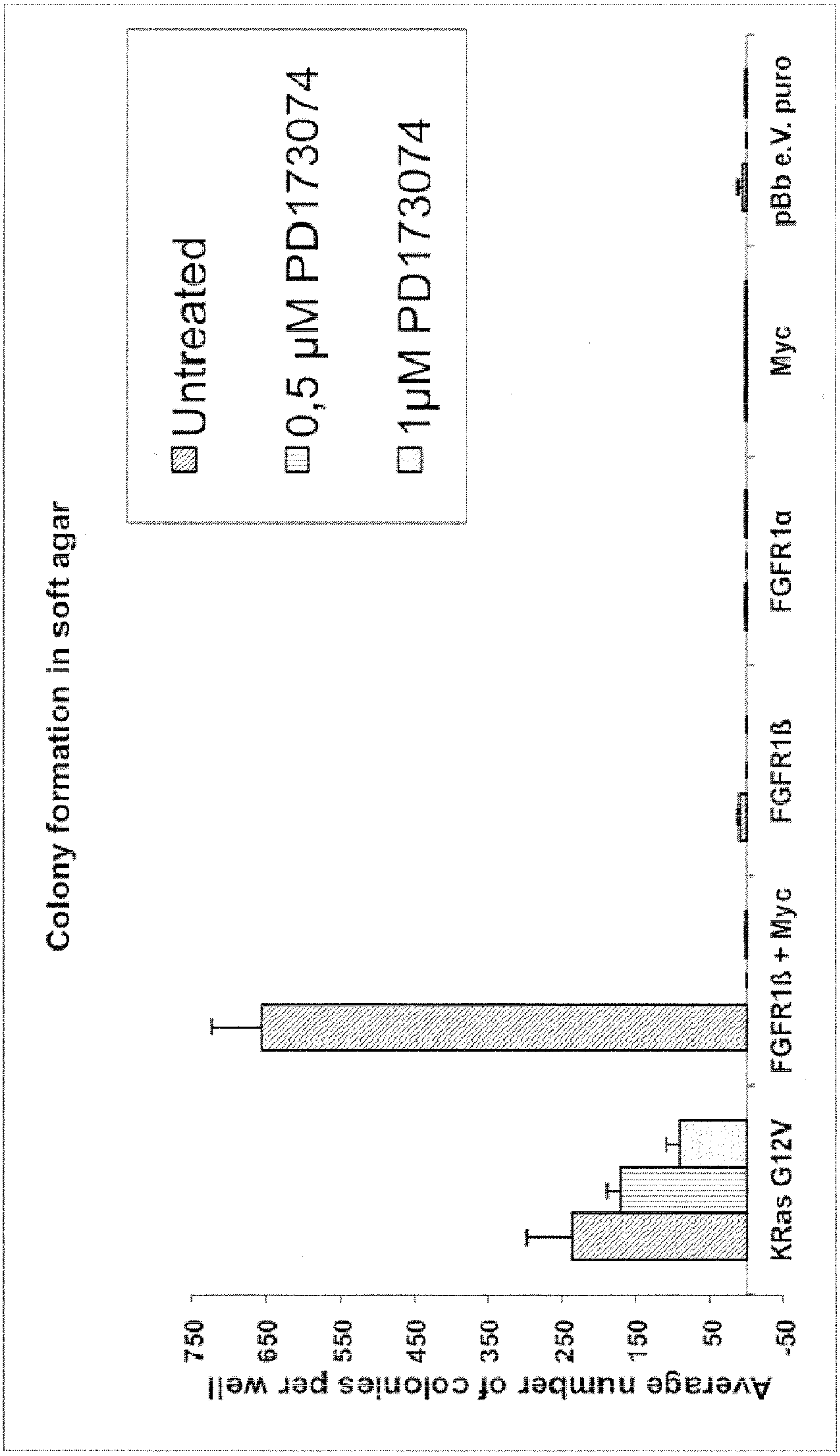
Colony formation in soft agar
Untreated
0,5 µM PD173074
1µM PD173074
Average number of colonies per well
750
650
550
450
350
250
150
50
-50
KRas G12V
FGFR1ß + Myc
FGFR1ß
FGFR1α
Myc
pBb e.V. puro

Figure 2.

## FGFR1_DMSO

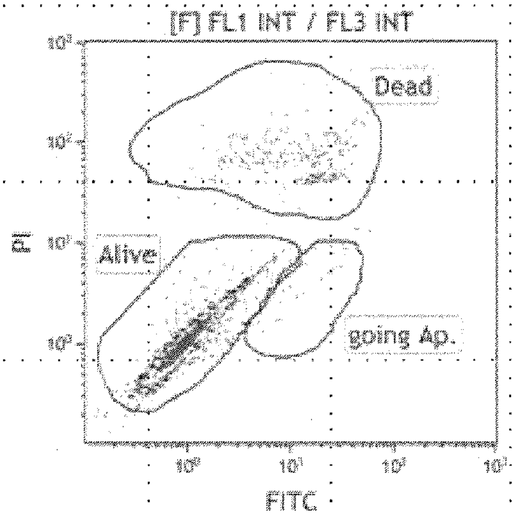


| Gate | %Total | %Gated |
|---|---|---|
| All | 73.57 | 100.00 |
| Alive | 71.19 | 96.76 |
| Dead | 1.92 | 2.61 |
| going Ap. | 0.24 | 0.33 |

## FGFR1+Myc_DMSO

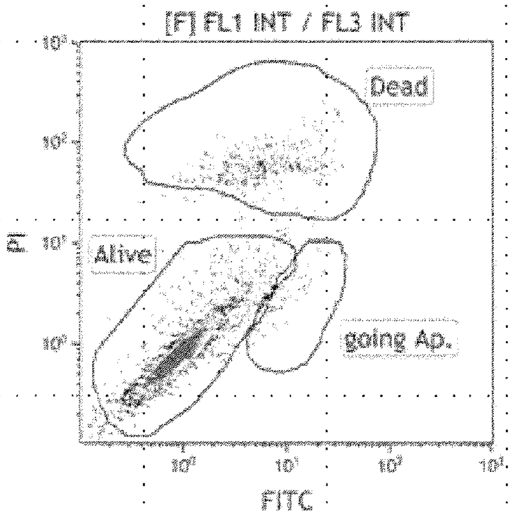


| Gate | %Total | %Gated |
|---|---|---|
| All | 75.25 | 100.00 |
| Alive | 70.52 | 93.71 |
| Dead | 3.18 | 4.23 |
| going Ap. | 0.62 | 0.83 |

**Figure 2 (cont.).**

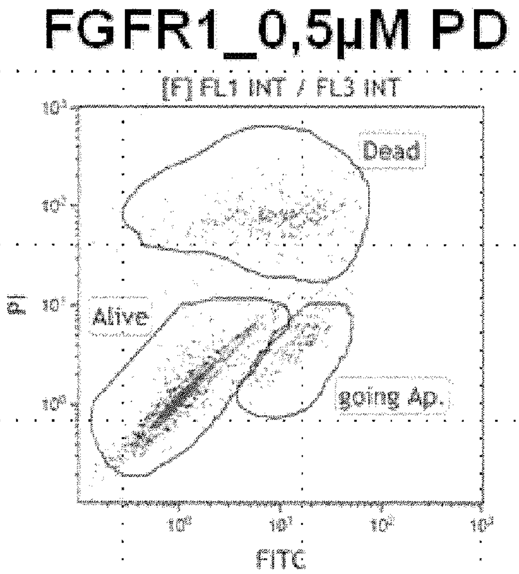


| Gate | %Total | %Gated |
|---|---|---|
| All | 80.78 | 100.00 |
| Alive | 76.10 | 94.21 |
| Dead | 2.92 | 3.61 |
| going Ap. | 1.16 | 1.43 |

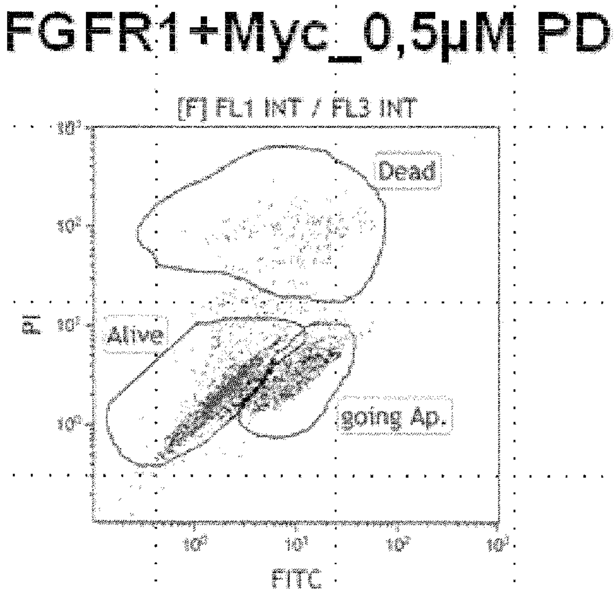


| Gate | %Total | %Gated |
|---|---|---|
| All | 73.24 | 100.00 |
| Alive | 55.75 | 76.12 |
| Dead | 1.38 | 1.88 |
| going Ap. | 15.64 | 21.36 |

Figure 2 (cont.).

FGFR1_1µM PD

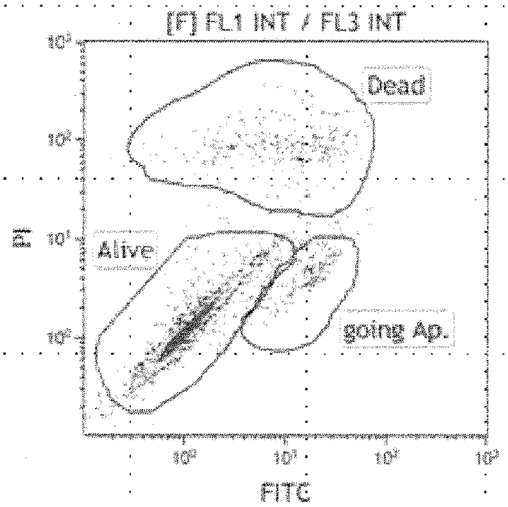


| Gate | %Total | %Gated |
|---|---|---|
| All | 82.80 | 100.00 |
| Alive | 78.36 | 94.63 |
| Dead | 2.47 | 2.98 |
| going Ap. | 1.37 | 1.66 |

FGFR1+Myc_1µM PD

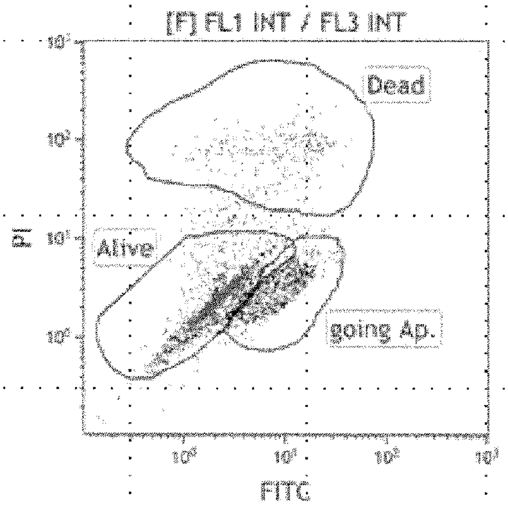


| Gate | %Total | %Gated |
|---|---|---|
| All | 72.32 | 100.00 |
| Alive | 55.20 | 76.33 |
| Dead | 1.78 | 2.47 |
| going Ap. | 14.97 | 20.70 |

Figure 3.

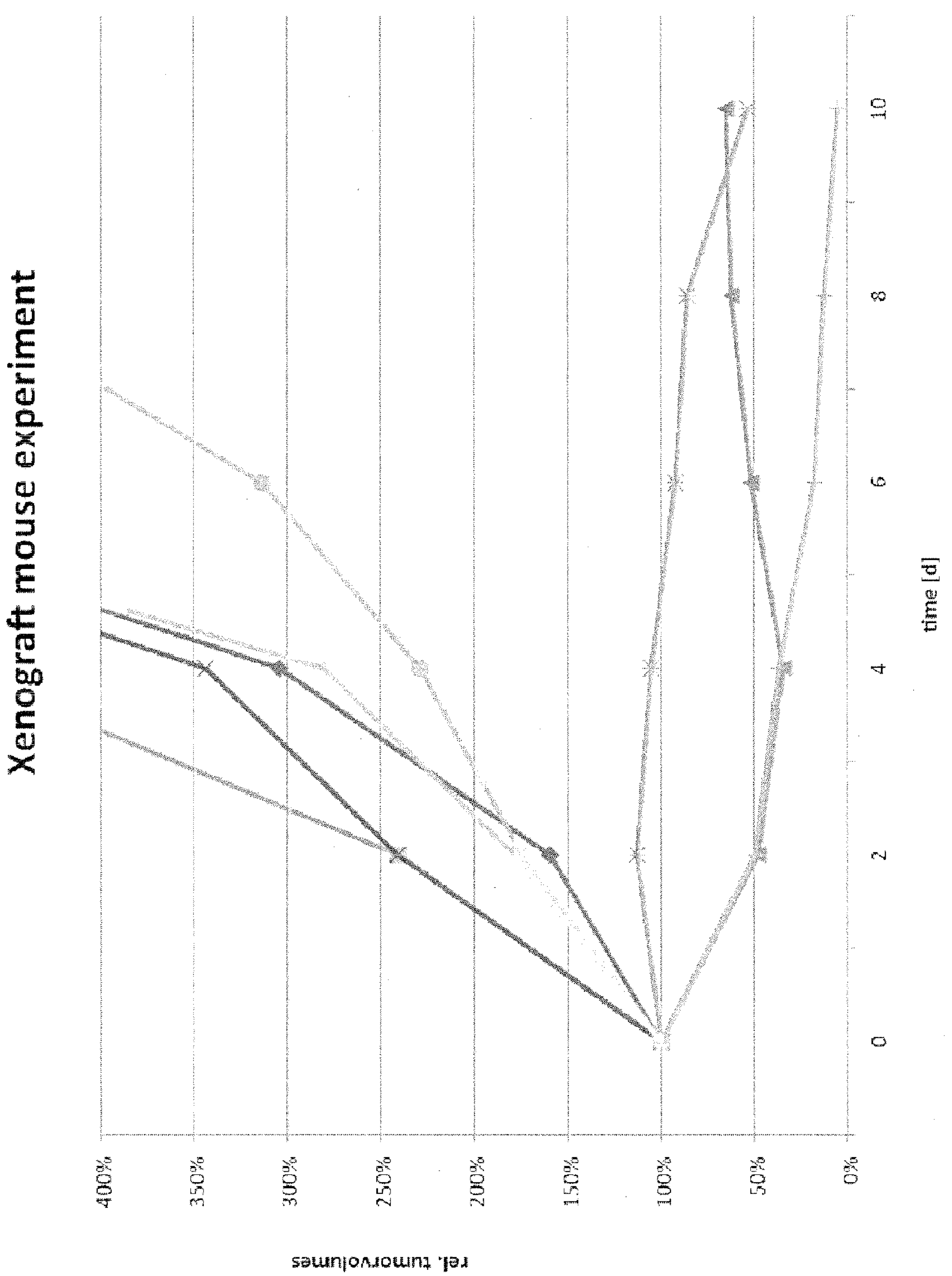
Xenograft mouse experiment
rel. tumorvolumes
400%
350%
300%
250%
200%
150%
100%
50%
0%
0
2
4
6
8
10
time [d]

Figure 3 (cont).

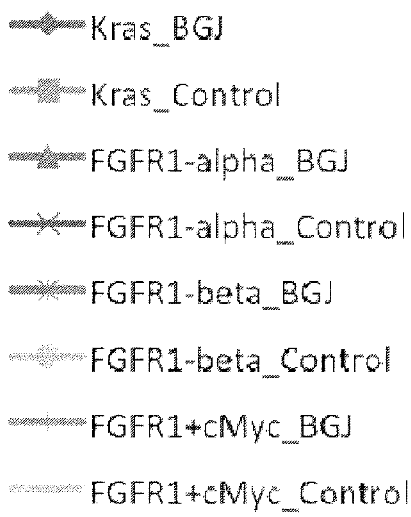

Figure 4.

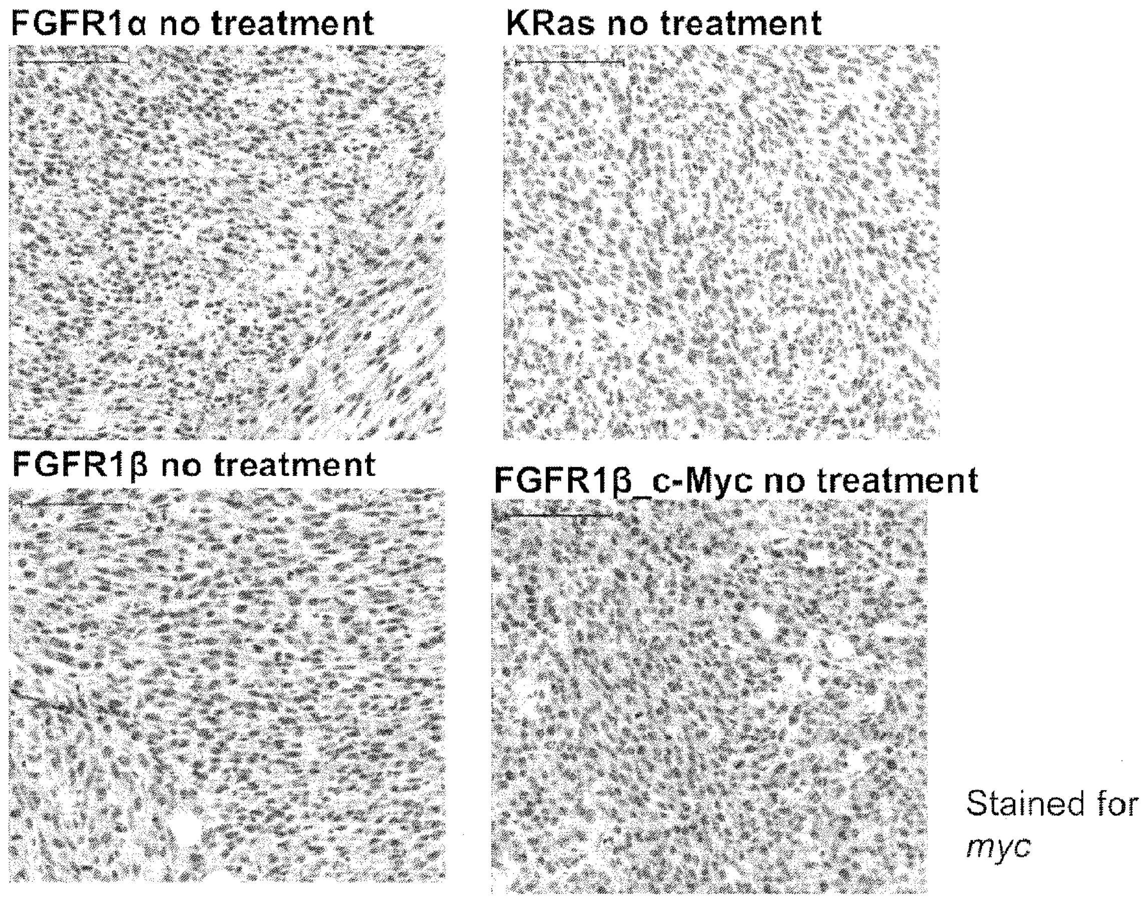
FGFR1α no treatment
KRas no treatment
FGFR1β no treatment
FGFR1β_c-Myc no treatment
Stained for *myc*

Figure 5.

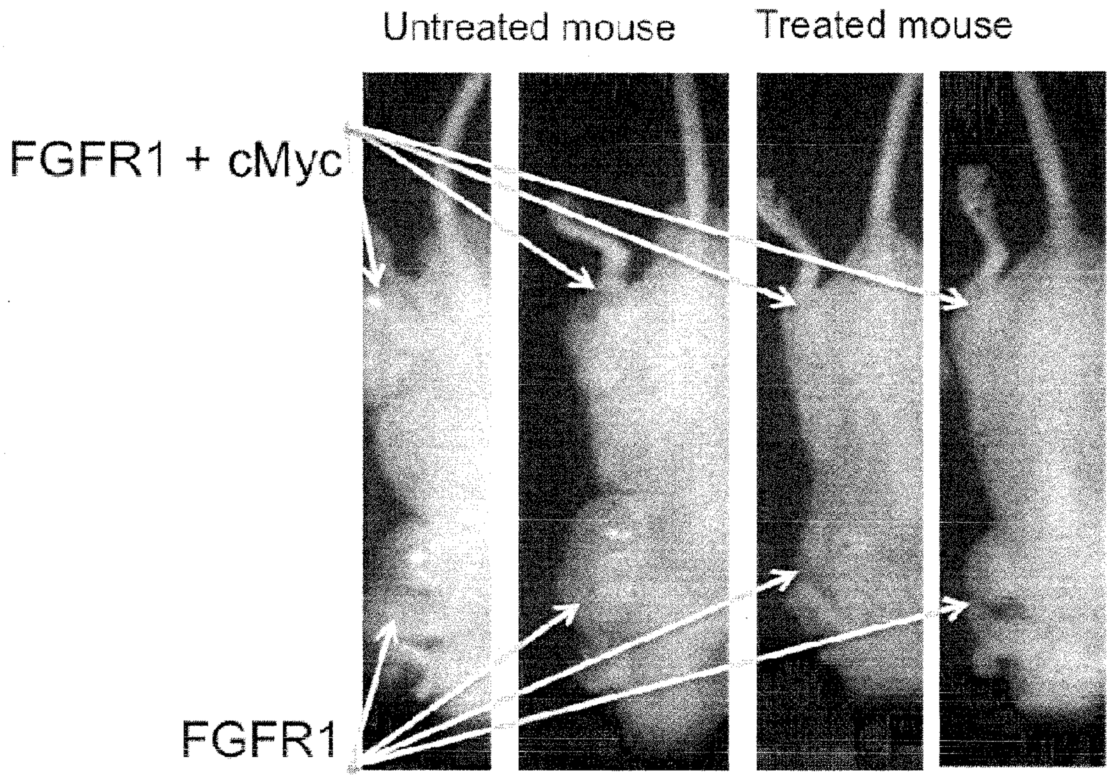

Figure 6.

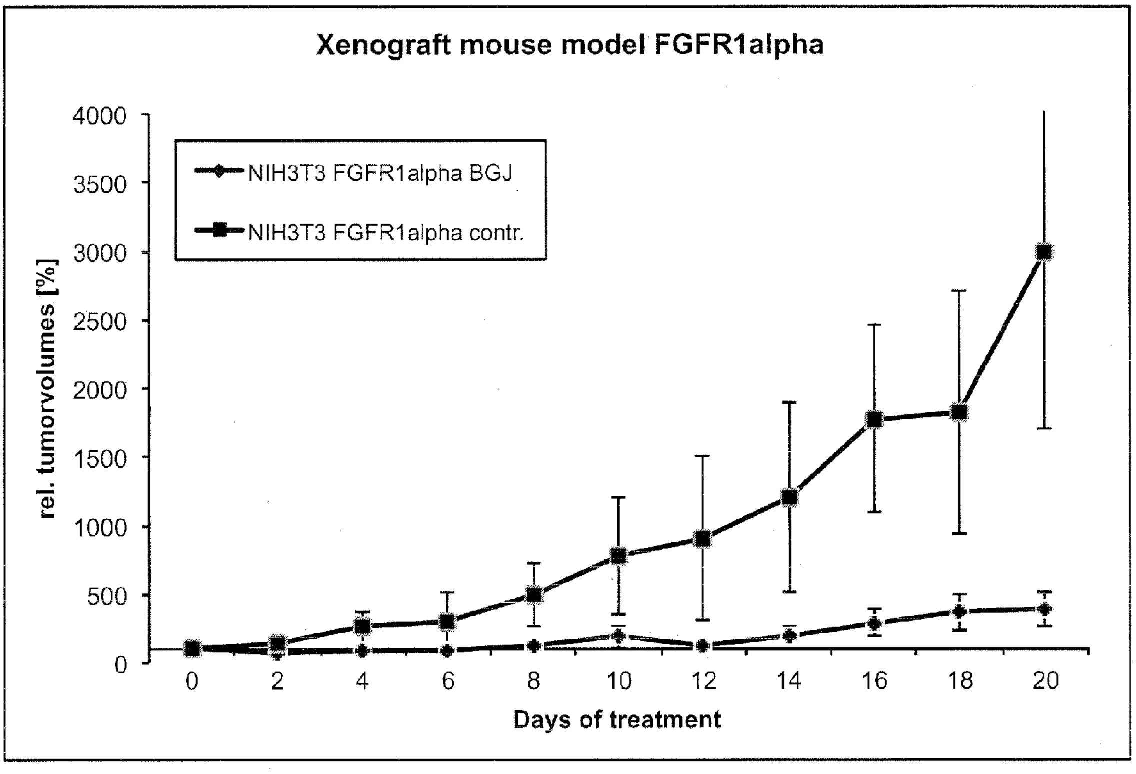
Xenograft mouse model FGFR1alpha
NIH3T3 FGFR1alpha BGJ
NIH3T3 FGFR1alpha contr.
rel. tumorvolumes [%]
4000
3500
3000
2500
2000
1500
1000
500
0
0
2
4
6
8
10
12
14
16
18
20
Days of treatment

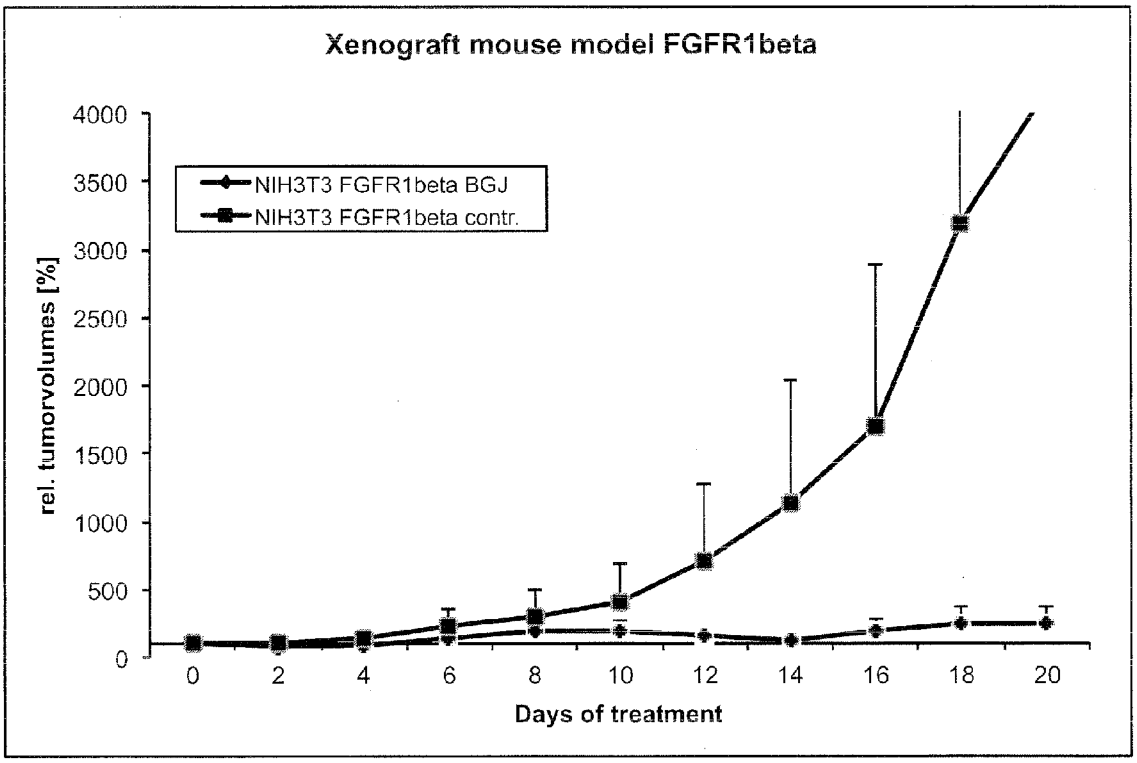
Xenograft mouse model FGFR1beta
NIH3T3 FGFR1beta BGJ
NIH3T3 FGFR1beta contr.
rel. tumorvolumes [%]
4000
3500
3000
2500
2000
1500
1000
500
0
0
2
4
6
8
10
12
14
16
18
20
Days of treatment

Figure 6 (cont.).

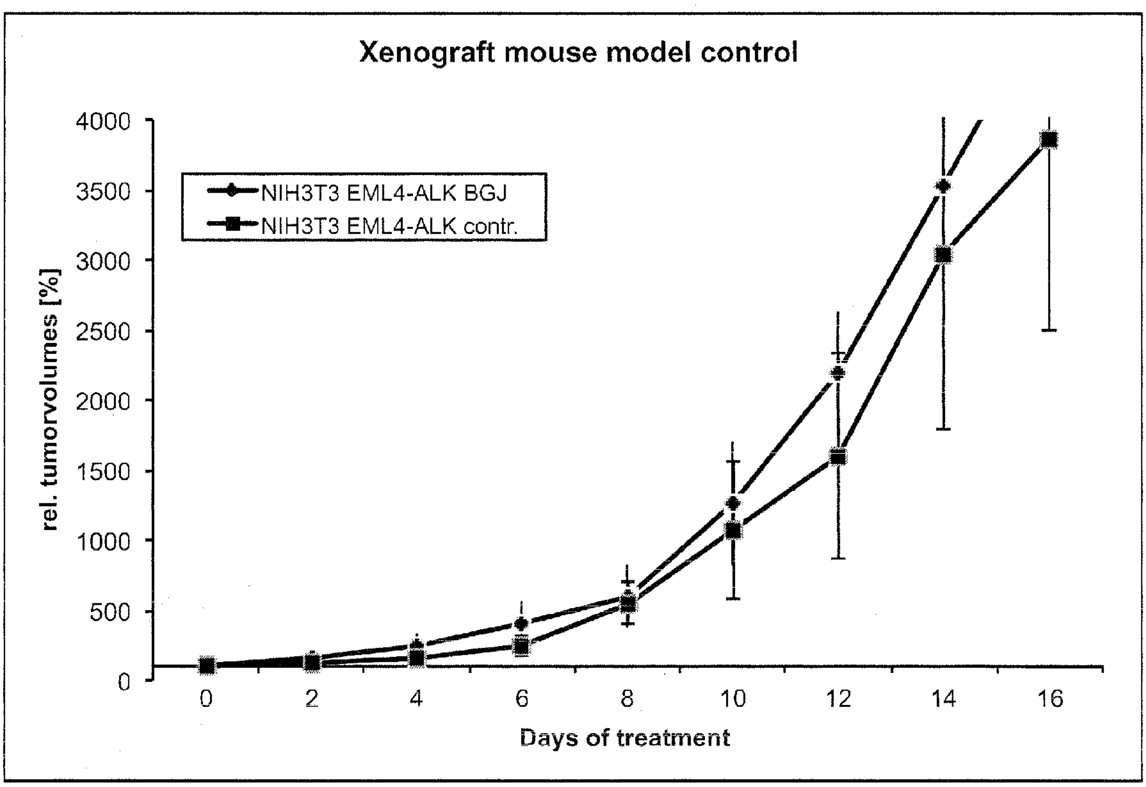

Figure 7.

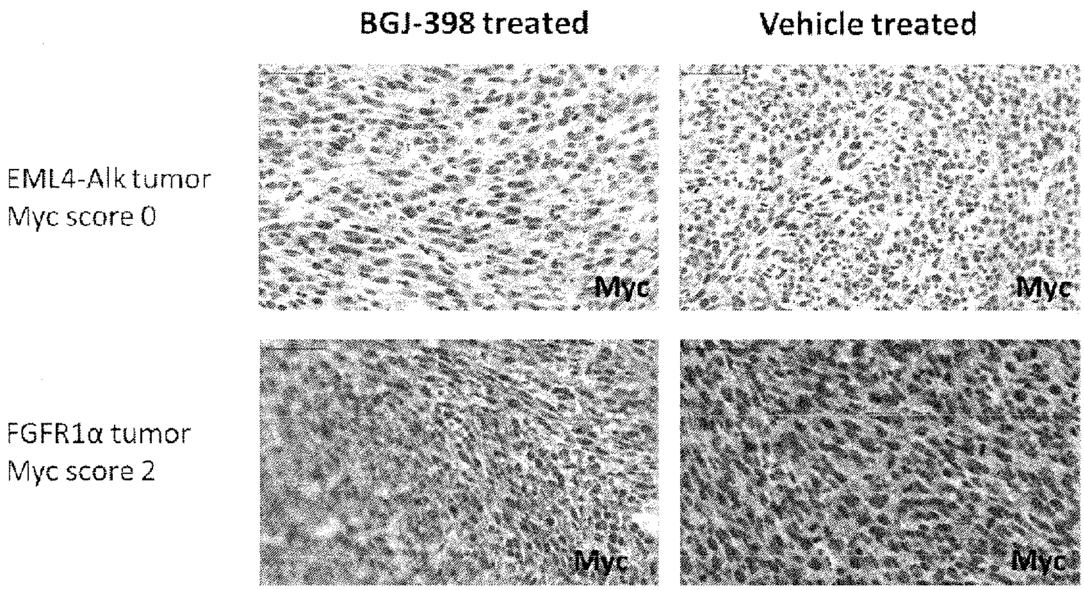
BGJ-398 treated
Vehicle treated
EML4-Alk tumor
Myc score 0
FGFR1α tumor
Myc score 2
Myc
Myc
Myc
Myc

**Figure 8.**

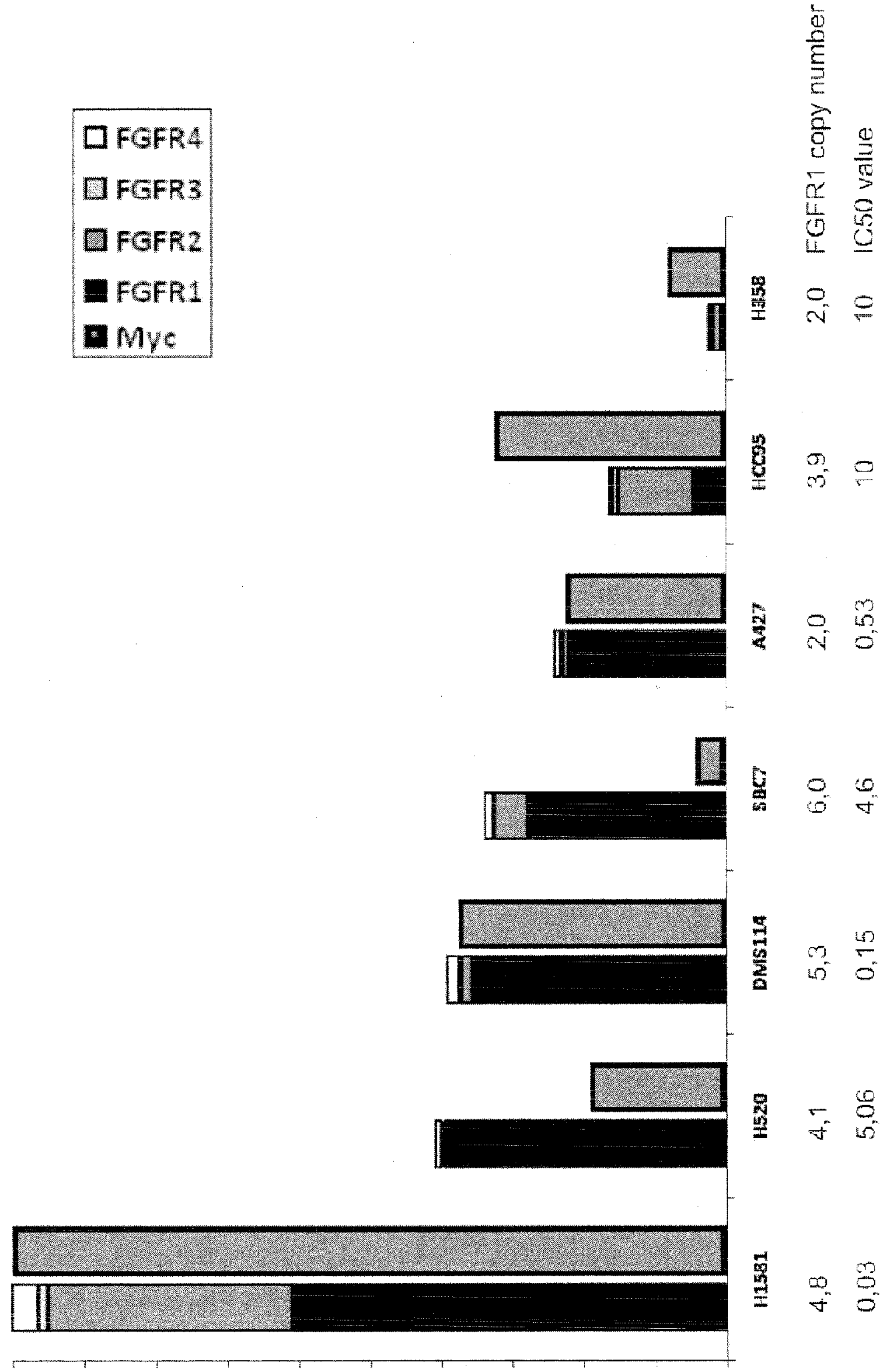
FGFR4
FGFR3
FGFR2
FGFR1
Myc
H1581
H520
DMS114
SBC7
A427
HCC95
H358
FGFR1 copy number
4,8
4,1
5,3
6,0
2,0
3,9
2,0
IC50 value
0,03
5,06
0,15
4,6
0,53
10
10

Figure 9.

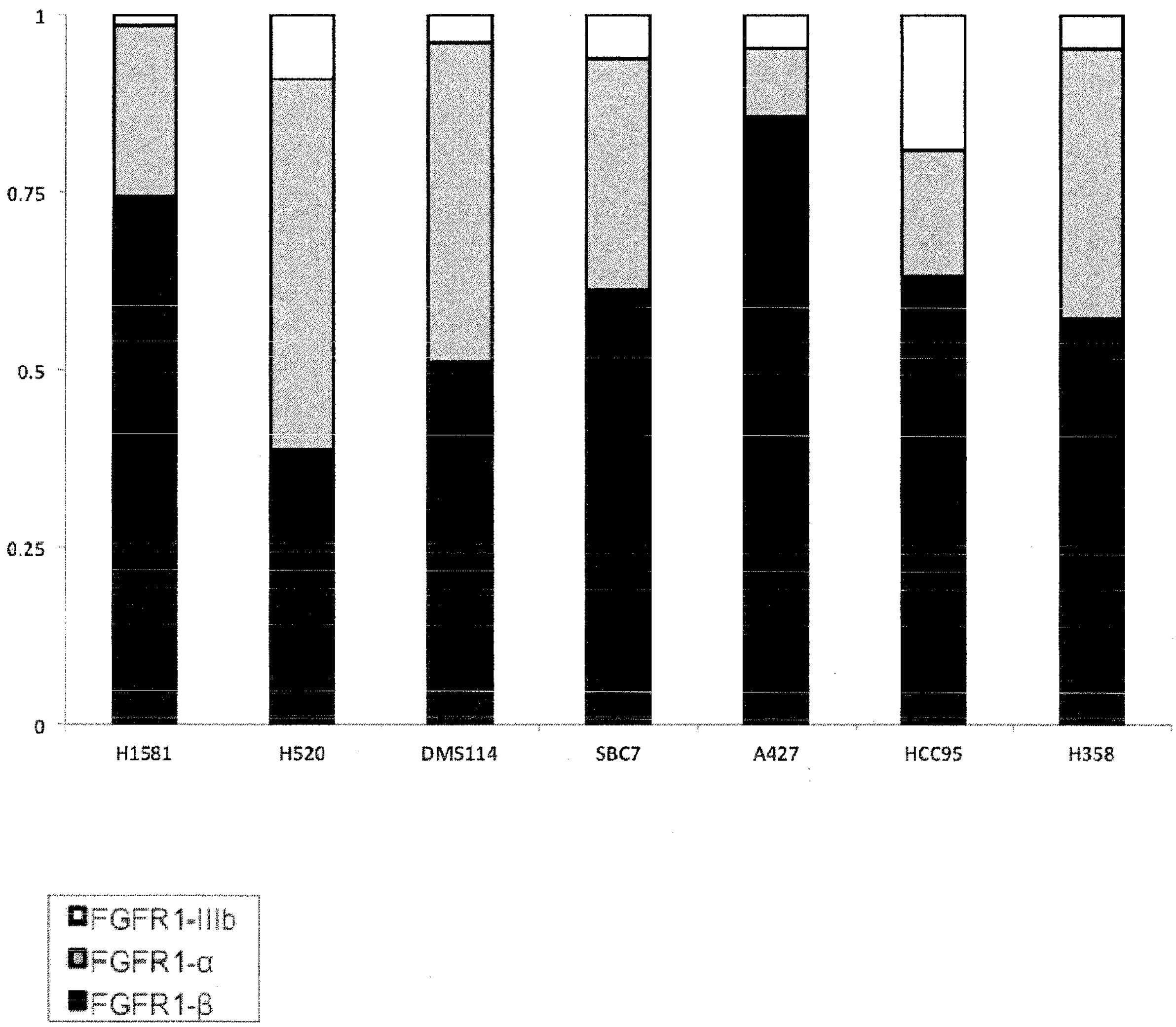
1
0.75
0.5
0.25
0
H1581
H520
DMS114
SBC7
A427
HCC95
H358
FGFR1-IIIb
FGFR1-α
FGFR1-β

Figure 10.

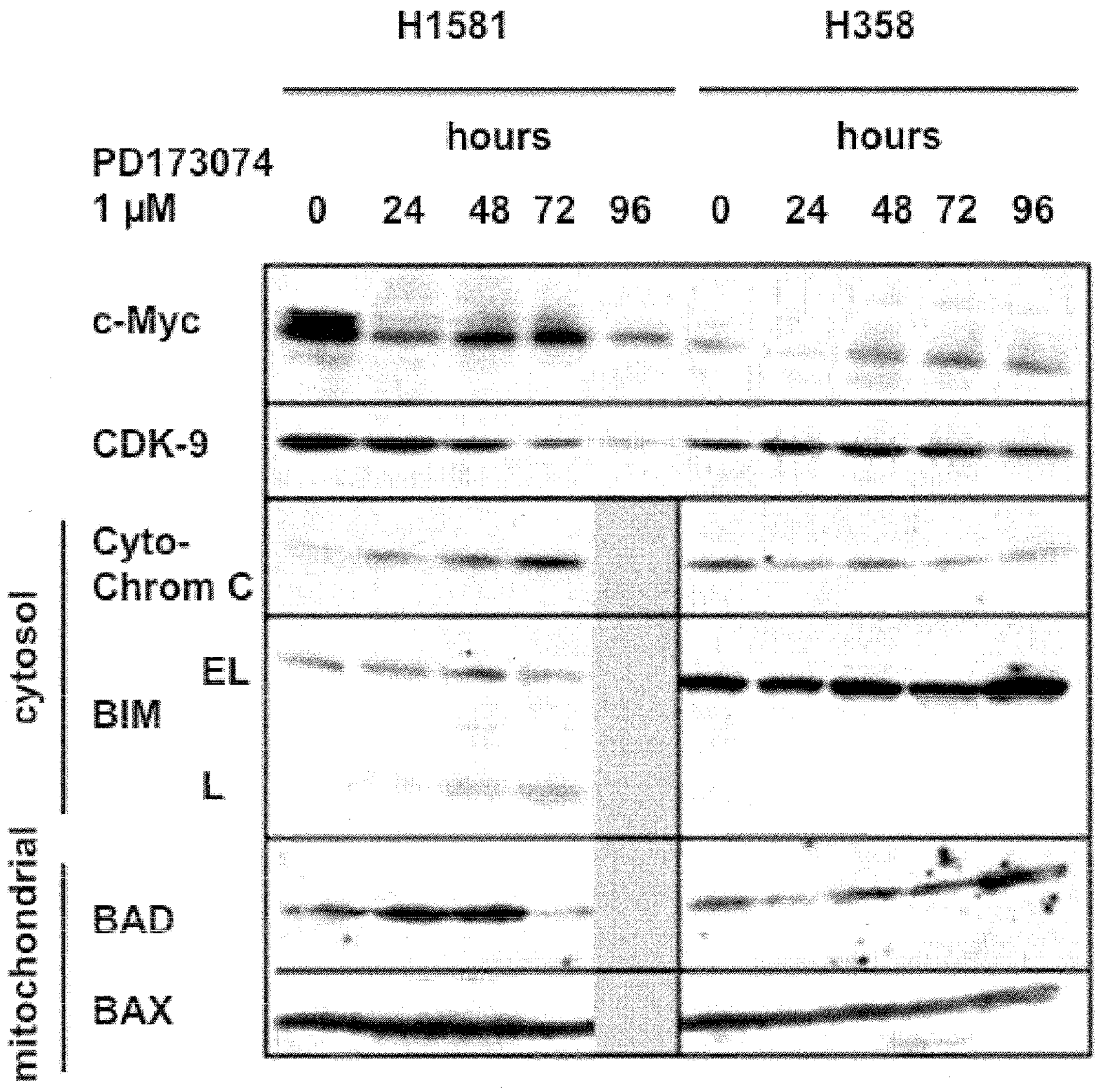
H1581
H358
hours
hours
PD173074
1 µM
0 24 48 72 96 0 24 48 72 96
c-Myc
CDK-9
Cyto-
Chrom C
BIM
EL
L
BAD
BAX
cytosol
mitochondrial

Figure 11.

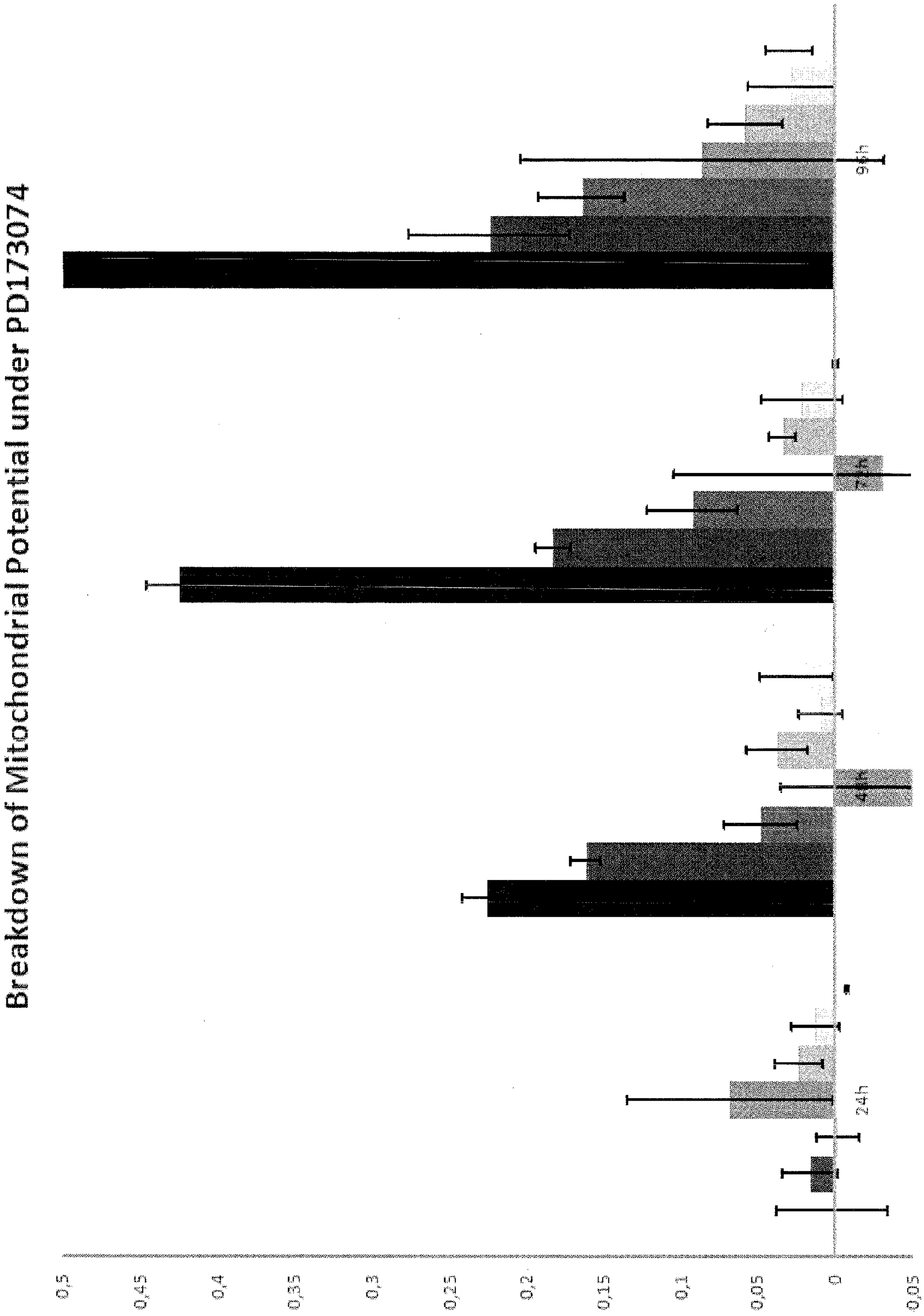
Breakdown of Mitochondrial Potential under PD173074
0,5
0,45
0,4
0,35
0,3
0,25
0,2
0,15
0,1
0,05
0
24h

Figure 11 (cont).

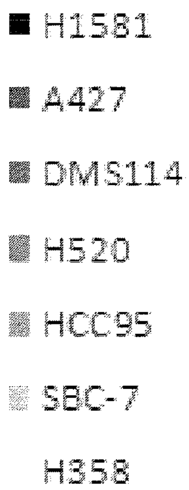

Figure 12.

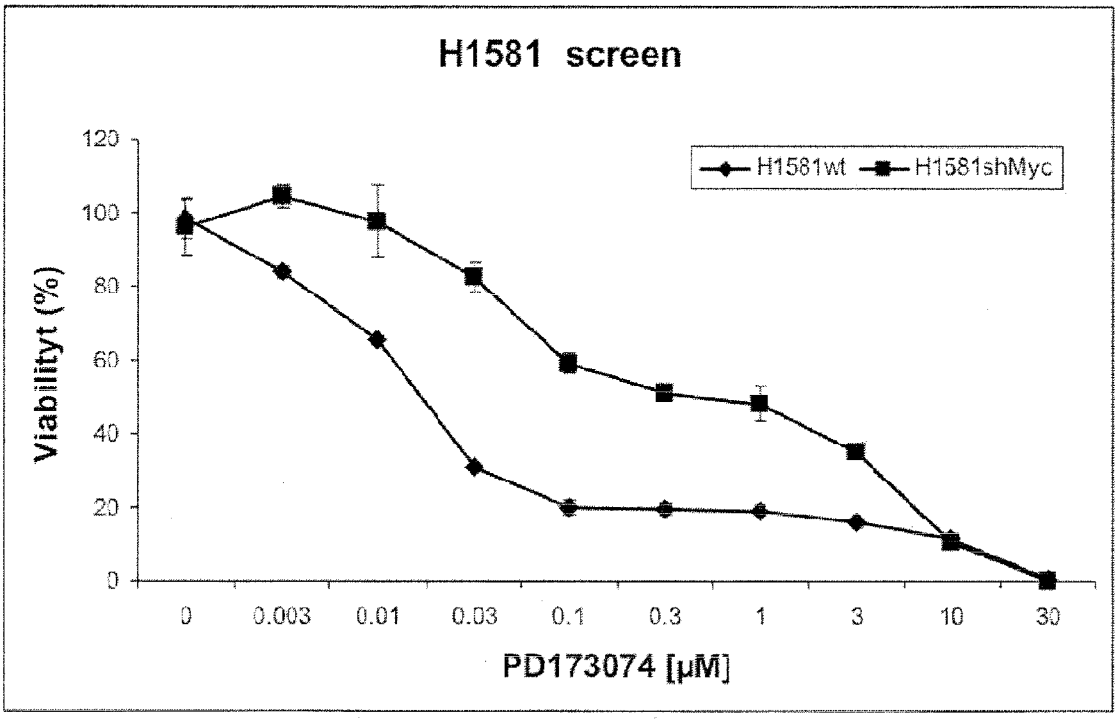
H1581 screen
120
100
80
60
40
20
0
Viabilityt (%)
H1581wt
H1581shMyc
0
0.003
0.01
0.03
0.1
0.3
1
3
10
30
PD173074 [μM]

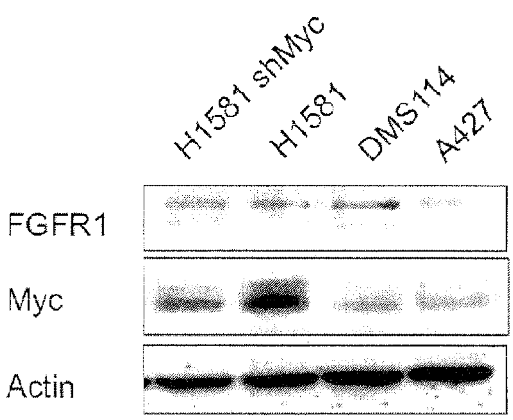
H1581 shMyc
H1581
DMS114
A427
FGFR1
Myc
Actin

Europäisches Patentamt
European Patent Office
Office européen des brevets

# EUROPEAN SEARCH REPORT

Application Number
EP 12 18 0179

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/040083 A2 (US HEALTH [US]; GREEN JEFFREY E [US]; KIM HARK K [KR] U S A AS REPRESE) 8 April 2010 (2010-04-08) * pg, l 9-13; pg 2, l 9-27; pg 3, l 24-pg 4, l 2; pg 4 l 21-23; pg 9, l 28-pg10, l 4. * ----- | 1,2,4-9, 11-13 | INV. C12Q1/68 G01N33/574 |
| X | OTA SARA ET AL: "The role of senescence and prosurvival signaling in controlling the oncogenic activity of FGFR2 mutants associated with cancer and birth defects", HUMAN MOLECULAR GENETICS, vol. 18, no. 14, July 2009 (2009-07), pages 2609-2621, XP002689269, | 1,2,4-9, 11,12 | |
| Y | * pg 2069; pg 2610, left col, last par-right col, first par; pg 2617-2619; Fig 5. * ----- | 3,10, 13-15 | |
| Y | BROOKS A NIGEL ET AL: "Molecular Pathways: Fibroblast Growth Factor Signaling: A New Therapeutic Opportunity in Cancer", CLINICAL CANCER RESEARCH, vol. 18, no. 7, April 2012 (2012-04), pages 1855-1862, XP002689270, * pg 1855; pg 1859, left column. * ----- | 1-15 | TECHNICAL FIELDS SEARCHED (IPC): C12Q G01N |
| | -/-- | | |

~~The present search report has been drawn up for all claims~~

| Place of search | Date of completion of the search | Examiner |
|---|---|---|
| Munich | 6 June 2013 | Motrescu-Hateley, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

4

EPO FORM 1503 03.82 (P04C01)

Europäisches Patentamt
European Patent Office
Office européen des brevets

# EUROPEAN SEARCH REPORT

Application Number
EP 12 18 0179

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WEISS J ET AL: "Frequent and focal FGFR1 amplification associates with therapeutically tractable FGFR1 dependency in squamous cell lung cancer", SCIENCE TRANSLATION MEDICINE, AMERICAN ASSOCIATION FOR THE ADVANCEMENT OF SCIENCE, US, vol. 2, no. 62, 1 January 2010 (2010-01-01), page 62ra93, XP009165597, ISSN: 1946-6234, DOI: 10.1126/SCITRANSLMED.3001451 [retrieved on 2010-12-15] * pg 1, pg 2, right col; pg 3-5. * ----- | 1-15 | |
| T | SOS MARTIN L ET AL: "A framework for identification of actionable cancer genome dependencies in small cell lung cancer", PROCEEDINGS OF THE NATIONAL ACADEMY OF SCIENCES OF THE UNITED STATES OF AMERICA, vol. 109, no. 42, October 2012 (2012-10), pages 17034-17039, XP002689271, ISSN: 0027-8424 * the whole document * ----- | | TECHNICAL FIELDS SEARCHED (IPC) |
| A | GUAGNANO VITO ET AL: "Discovery of 3-(2,6-Dichloro-3,5-dimethoxy-phenyl)-1-{6-[4-(4-ethyl-piperazin-1-yl)-phenylamino]-pyrimidin-4-yl}-1-methyl-urea (NVP-BGJ398), A Potent and Selective Inhibitor of the Fibroblast Growth Factor Receptor Family of Receptor Tyrosine Kinase", JOURNAL OF MEDICINAL CHEMISTRY, vol. 54, no. 20, October 2011 (2011-10), pages 7066-7083, XP002689272, ISSN: 0022-2623 * the whole document * ----- -/-- | 1-15 | |

~~The present search report has been drawn up for all claims~~

| Place of search | Date of completion of the search | Examiner |
|---|---|---|
| Munich | 6 June 2013 | Motrescu-Hateley, E |

4

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 18 0179

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2010/052225 A1 (HOFFMANN LA ROCHE [CH]; KIERMAIER ASTRID [DE]; KOEHLER ASTRID [US]; PI) 14 May 2010 (2010-05-14)<br>* pg 1, first par;pg 10, last par-pg 12, second par;cl 1-3, 26-27. *<br>----- | 1-15 | |
| Y | J. M. GOZGIT ET AL: "Ponatinib (AP24534), a Multitargeted Pan-FGFR Inhibitor with Activity in Multiple FGFR-Amplified or Mutated Cancer Models",<br>MOLECULAR CANCER THERAPEUTICS,<br>vol. 11, no. 3,<br>11 January 2012 (2012-01-11), pages 690-699, XP055065380,<br>ISSN: 1535-7163, DOI: 10.1158/1535-7163.MCT-11-0450<br>* pg 690-693, 695. *<br>----- | 3,14,15 | |
| A | TIANJUN ZHOU ET AL: "Structural Mechanism of the Pan-BCR-ABL Inhibitor Ponatinib (AP24534): Lessons for Overcoming Kinase Inhibitor Resistance",<br>CHEMICAL BIOLOGY & DRUG DESIGN,<br>vol. 77, no. 1,<br>30 November 2010 (2010-11-30), pages 1-11, XP055065379,<br>ISSN: 1747-0277, DOI: 10.1111/j.1747-0285.2010.01054.x<br>* the whole document *<br>----- | 1-15 | TECHNICAL FIELDS SEARCHED (IPC) |

-/--

~~The present search report has been drawn up for all claims~~

4

| Place of search | Date of completion of the search | Examiner |
|---|---|---|
| Munich | 6 June 2013 | Motrescu-Hateley, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
&: member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 18 0179

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | P. R. GAVINE ET AL: "AZD4547: An Orally Bioavailable, Potent, and Selective Inhibitor of the Fibroblast Growth Factor Receptor Tyrosine Kinase Family", CANCER RESEARCH, vol. 72, no. 8, 27 February 2012 (2012-02-27), pages 2045-2056, XP055065340, ISSN: 0008-5472, DOI: 10.1158/0008-5472.CAN-11-3034 * pg 2405-2050. * ----- | 3,14,15 | |
| | | | TECHNICAL FIELDS SEARCHED (IPC) |

~~The present search report has been drawn up for all claims~~

4

| Place of search | Date of completion of the search | Examiner |
|---|---|---|
| Munich | 6 June 2013 | Motrescu-Hateley, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 12 18 0179

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

1-15(partially)

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number

EP 12 18 0179

The Search Division considers that the present European patent application does not comply with the requirements of unity of invention and relates to several inventions or groups of inventions, namely:

1. claims: 1-15(partially)

A method for predicting the responsiveness of a mammalian tumor cell or an individual having a tumor to an inhibitor of a fibroblast growth factor receptor (FGFR), said method comprising evaluating the status of FGFR and the status of c-myc in said cell, wherein said status of FGFR and of c-myc is indicative of the responsiveness of said cell to the inhibitor. The said inhibitor of a fibroblast growth factor receptor (FGFR) is BGJ398. An inhibitor to be used in the treatment of cancer in individuals identified by above mentioned method.

---

2. claims: 1-15(partially)

A method for predicting the responsiveness of a mammalian tumor cell or an individual having a tumor to an inhibitor of a fibroblast growth factor receptor (FGFR), said method comprising evaluating the status of FGFR and the status of c-myc in said cell, wherein said status of FGFR and of c-myc is indicative of the responsiveness of said cell to the inhibitor. The said inhibitor of a fibroblast growth factor receptor (FGFR) is PD173074. An inhibitor to be used in the treatment of cancer in individuals identified by above mentioned method.

---

3. claims: 1-15(partially)

A method for predicting the responsiveness of a mammalian tumor cell or an individual having a tumor to an inhibitor of a fibroblast growth factor receptor (FGFR), said method comprising evaluating the status of FGFR and the status of c-myc in said cell, wherein said status of FGFR and of c-myc is indicative of the responsiveness of said cell to the inhibitor. The said inhibitor of a fibroblast growth factor receptor (FGFR) is AP24534. An inhibitor to be used in the treatment of cancer in individuals identified by above mentioned method.

---

4. claims: 1-15(partially)

A method for predicting the responsiveness of a mammalian tumor cell or an individual having a tumor to an inhibitor of a fibroblast growth factor receptor (FGFR), said method comprising evaluating the status of FGFR and the status of c-myc in said cell, wherein said status of FGFR and of c-myc is indicative of the responsiveness of said cell to the inhibitor. The said inhibitor of a fibroblast growth factor

Europäisches Patentamt
European Patent Office
Office européen des brevets

**LACK OF UNITY OF INVENTION**
**SHEET B**

Application Number
EP 12 18 0179

The Search Division considers that the present European patent application does not comply with the requirements of unity of invention and relates to several inventions or groups of inventions, namely:

receptor (FGFR) is AZD4547. An inhibitor to be used in the treatment of cancer in individuals identified by above mentioned method.

---

5. claims: 1-15(partially)

A method for predicting the responsiveness of a mammalian tumor cell or an individual having a tumor to an inhibitor of a fibroblast growth factor receptor (FGFR), said method comprising evaluating the status of FGFR and the status of c-myc in said cell, wherein said status of FGFR and of c-myc is indicative of the responsiveness of said cell to the inhibitor. The said inhibitor of a fibroblast growth factor receptor (FGFR) is E-3810. An inhibitor to be used in the treatment of cancer in individuals identified by above mentioned method.

---

## ANNEX TO THE EUROPEAN SEARCH REPORT ON EUROPEAN PATENT APPLICATION NO. EP 12 18 0179

This annex lists the patent family members relating to the patent documents cited in the above-mentioned European search report.
The members are as contained in the European Patent Office EDP file on
The European Patent Office is in no way liable for these particulars which are merely given for the purpose of information.

06-06-2013

| Patent document cited in search report | Publication date | Patent family member(s) | Publication date |
|---|---|---|---|
| WO 2010040083 A2 | 08-04-2010 | NONE | |
| WO 2010052225 A1 | 14-05-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3773919 A
- EP 58481 A
- EP 133988 A
- DE 3218121
- EP 52322 A
- EP 36676 A
- EP 88046 A
- EP 143949 A
- EP 142641 A
- JP 58118008 A
- US 4485045 A
- US 4544545 A
- EP 102324 A
- US 5525711 A
- US 4711955 A
- US 5792608 A
- EP 302175 A


### Non-patent literature cited in the description


- **ZHU.** *Cancer Cell,* vol. 21, 362-373
- **DUTT.** *PloS one,* 2011, vol. 6, 20351
- **COURJAL, F. et al.** *Cancer Res.,* 1997, vol. 57, 4360-4367
- **SIMON, R. et al.** *Cancer Res.,* 2001, vol. 61, 4514-4519
- **GREULICH ; POLLOCK.** *Trends Mol Med,* 2001, vol. 17, 283-292
- **WEISS.** *Sci Transl Med,* 2010, vol. 2, 62ra93
- **WOLFER et al.** *PNAS,* 2009, vol. 107, 3698-3703
- **BELLO et al.** *Cancer Res.,* 2011
- **BACHOVCHIN.** *Nature biotechnology,* 2009, vol. 27, 387-394
- **TURNER ; GROSE.** *Nature,* 2010, vol. 10, 116-129
- **GREULICH ; POLLOCK.** *Trends in Molecular Medicine,* 2011
- **SIDMAN, U. et al.** *Biopolymers,* 1983, vol. 22, 547-556
- **R. LANGER et al.** *J. Biomed. Mater. Res.,* vol. 15, 167-277
- **R. LANGER.** *Chem. Tech.,* 1982, vol. 12, 98-105
- **EPSTEIN et al.** *Proc. Natl. Acad. Sci. (USA,* 1985, vol. 82, 3688-3692
- **HWANG et al.** *Proc. Natal. Acad. Sci. (USA,* 1980, vol. 77, 4030-4034
- Current Protocols in Neuroscience. John Wiley&Sons, 2001
- *Methods in Enzymology,* 1987, vol. 153, 385-516
- **BITTER et al.** *Methods in Enzymology,* 1987, vol. 153, 516-544
- **SAWERS et al.** *Applied Microbiology and Biotechnology,* 1996, vol. 46, 1-9
- **BILLMAN-JACOBE.** *Current Opinion in Biotechnology,* 1996, vol. 7, 500-4
- **HOCKNEY.** *Trends in Biotechnology,* 1994, vol. 12, 456-463
- **GRIFFITHS et al.** *Methods in Molecular Biology,* 1997, vol. 75, 427-440
- **SAMBROOK ; RUSSELL.** Molecular Cloning: A Laboratory Manual. CSH Press, 2001
- Methods in Yeast Genetics, A Laboratory Course Manual. Cold Spring Harbor Laboratory Press, 1990
- **COLESTRAUSS.** *Science,* 1996, 1386-1389
- **SAMBROOK, RUSSELL.** Molecular Cloning, A Laboratory Manual. Cold Spring Harbor Laboratory, 2001
- **AUSUBEL.** Current Protocols in Molecular Biology. Green Publishing Associates and Wiley Interscience, 1989
- Nucleic acid hybridization, a practical approach. IRL Press Oxford, 1985
- **THOMPSON.** *Nucl. Acids Res.,* 1994, vol. 2, 4673-4680
- **BRUTLAG.** *Comp. App. Biosci.,* 1990, vol. 6, 237-245
- **ALTSCHUL.** *Nucl. Acids Res.,* 1997, vol. 25, 3389-3402
- **ALTSCHUL.** *J. Mol. Evol.,* 1993, vol. 36, 290-300
- **ALTSCHUL.** *J. Mol. Biol.,* 1990, vol. 215, 403-410